# EUROPEAN PATENT SPECIFICATION

(11) **EP 1 765 310 B1**
(45) Date of publication and mention of the grant of the patent: **28.10.2015**
(21) Application number: 05826138.9
(22) Date of filing: 25.05.2005
(51) Int. Cl.: A61F 13/00, A61K 9/70, A61K 9/00, A61K 47/36, A61K 47/44

(54) **A MIXTURE FOR TRANSDERMAL DELIVERY OF LOW AND HIGH MOLECULAR WEIGHT COMPOUNDS**
MISCHUNG ZUR TRANSDERMALEN ABGABE VON VERBINDUNGEN MIT NIEDRIGEM UND HOHEM MOLEKULARGEWICHT
MELANGE POUR ADMINISTRATION TRANSDERMIQUE DE COMPOSES A POIDS MOLECULAIRE FAIBLE ET ELEVE

(30) Priority: 28.05.2004 US 856567; 28.05.2004 WO PCT/US2004/017169
(43) Date of publication of application: 28.03.2007
(60) Divisional application: 15169877.6
(73) Proprietor: ORYXE, Irvine, California 92618 (US)
(72) Inventor: JORDAN, Frederick, Santa Ana, California 92705 (US); DOLBEAR, Geoff E., Diamond Bar, California 91765 (US)
(74) Representative: Plougmann & Vingtoft A/S
(86) International application number: PCT/US2005/019017
(87) International publication number: WO 2006/041538

(56) References cited:
- US-A- 5 128 324
- US-A- 5 318 960
- US-A1- 2003 064 093
- US-B1- 6 656 499
- M. BERGH: "Allergenic Oxidation Products", ACTA DERMATO-VENEREOLOGICA, vol. 79, no. 0, 3 December 1999 (1999-12-03), pages 5-26, XP55021650, ISSN: 0001-5555, DOI: 10.1080/000155599750208040

## Description

### FIELD OF THE INVENTION

Embodiments of the present invention relate to the discovery of several formulations of a transdermal delivery composition that delivers low and high molecular weight compounds, particularly drugs and cosmetic agents to a subject. Aspects of the invention include said transdermal delivery compositions, transdermal delivery devices for providing said compositions to subjects in need thereof and methods of making and using the foregoing.

### BACKGROUND OF THE INVENTION

The skin provides a protective barrier against foreign materials and infection. In mammals this is accomplished by forming a highly insoluble protein and lipid structure on the surface of the corneocytes termed the cornified envelope (CE). (Downing et al., Dermatology in General Medicine, Fitzpatrick, et al., eds., pp. 210-221 (1993), Ponec, M., The Keratinocyte Handbook, Leigh, et al., eds., pp. 351-363 (1994)). The CE is composed of polar lipids, such as ceramides, sterols, and fatty acids, and a complicated network of cross-linked proteins; however, the cytoplasm of stratum corneum cells remains polar and aqueous. The CE is extremely thin (10 microns) but provides a substantial barrier. Because of the accessibility and large area of the skin, it has long been considered a promising route for the administration of drugs, whether dermal, regional, or systemic effects are desired. Further relevant prior art includes US2003064093.

A topical route of drug administration is sometimes desirable because the risks and inconvenience of parenteral treatment can be avoided; the variable absorption and metabolism associated with oral treatment can be circumvented; drug administration can be continuous, thereby permitting the use of pharmacologically active agents with short biological half-lives; the gastrointestinal irritation associated with many compounds can be avoided; and cutaneous manifestations of diseases can be treated more effectively than by systemic approaches.

Most transdermal delivery compositions achieve epidermal penetration by using a skin penetration enhancing vehicle. Such compounds or mixtures of compounds are known in the art as "penetration enhancers" or "skin enhancers". While many of the skin enhancers in the literature enhance transdermal absorption, several possess certain drawbacks in that (i) some are regarded as toxic; (ii) some irritate the skin; (iii) some have a thinning effect on the skin after prolonged use; (iv) some change the intactness of the skin structure resulting in a change in the diffusability of the drug; and (v) all are incapable of delivering high molecular weight pharmaceuticals and cosmetic agents. Despite these efforts, there remains a need for transdermal delivery compositions that deliver a wide-range of pharmaceuticals and cosmetic agents.

### BRIEF SUMMARY OF THE INVENTION

Disclosed herein are formulations of transdermal delivery compositions used to deliver pharmaceuticals, therapeutic compounds, diagnostics, and cosmetic agents of various molecular weights. In several embodiments, the transdermal delivery composition comprises a unique formulation of penetration enhancer (an ethoxylated lipid, modified lipid, fatty acid, fatty alcohol, or fatty amine therein having 10-19 ethoxylations per molecule) or transdermal delivery enhancer (an ethoxylated compound with a multi-functional backbone) that delivers a wide range of pharmaceuticals and cosmetic agents having molecular weights of less than 100 daltons to greater than 500,000 daltons. For example, embodiments of the transdermal delivery composition include formulations that deliver a therapeutically effective amount of a pharmaceutical, including NSAIDs, capsaicin or Boswellin-containing pain-relief solutions, other drugs or chemicals, dyes, low and high molecular weight peptides (*e.g*., collagens or fragments thereof), hormones, nucleic acids, antibiotics, vaccine preparations, and immunogenic preparations. Methods of making the transdermal delivery compositions described herein and systems for their delivery are embodiments. Further embodiments include methods of using said compositions (e.g., the treatment and prevention of undesired human conditions or diseases or cosmetic applications).

Aspects of the invention concern transdermal delivery compositions that comprise lipospheres. In some embodiments, the liposphere comprises an ethoxylated composition having a carbon chain length of at least 10, wherein the ethoxylated composition (e.g., a fatty acid, fatty alcohol, or fatty amine), comprises, consists of, or consists essentially of at least 10, 11, 12, 13, 14, 15, 16, 17, 18, or 19 ethoxylations per molecule. Aspects of the invention also include propoxylated compositions or compositions that comprise a combination of propoxylated and ethoxylated compositions. In some formulations, the ethoxylated or propoxylated composition is a fatty moiety, such as a fatty acid (e.g., an unsaturated fatty acid or a polyunsaturated fatty acid). In other formulations, the fatty moiety is a fatty alcohol. In other embodiments, the liposphere comprises an ethoxylated or propoxylated oil or lipid having carbon chain lengths of at least 10, wherein the ethoxylated or propoxylated oil or lipid (e.g., a nut oil, a tri-alcohol, a tri-fatty amine, a glycolipid, a sphingolipid, a glycosphingolipid, or any other modified lipid moiety), comprises, consists of, or consists essentially of at least 10, 11, 12, 13, 14, 15, 16, 17, 18, or 19 ethoxylations per molecule.

In preferred embodiments, the number of ethoxylations or propoxylations per molecule is the same as the number of carbons in the fatty moiety or lipid moiety. Desirably, the fatty moiety has a carbon chain length of at least 10, 12, 14, 16, 18, 20, 22, or 24. The liposphere comprises a homogeneous mixture of an ethoxylated or propoxylated fatty moiety in some embodiments, while in other embodiments, the liposphere comprises a heterogeneous mixture of an ethoxylated or propoxylated fatty moiety.

Other aspects of the invention concern transdermal delivery compositions comprised of an ethoxylated lipid moiety, such as an oil, glycolipid, sphingolipid, or glycosphingolipid. The ethoxylated oil that can be used in the formulations described herein can be a vegetable, nut, animal, or synthetic oil having at least 10, 11, 12, 13, 14, 15, 16, 17, 18, 19, or more ethoxylations per molecule. Preferred oils include macadamia nut oil or meadowfoam (*limnanthes alba*). It should be understood that when an oil is ethoxylated, one or more of the components of the oil are ethoxylated (e.g., fatty acids, fatty alcohols, and/or fatty amines) and it is generally recognized in the field that an average number of ethoxylations for the oil and components is obtained and therefore provided. That is, the measured composition is the algebraic sum of the compositions of the species in the mix.

Still other aspects of the invention relate to transdermal delivery compositions comprising a delivery enhancer. As used herein, the term "transdermal delivery enhancer" refers to a molecule that comprises a multi-functional backbone having at least two reactive (R) groups. The R groups on the multifunctional backbone comprise a reactive hydrogen, such as -OH, COOH, amines, sulfydryl groups, and aldehydes. Thus, multifunctional backbones include trialcholols, triacids, amino acids, dipeptides, tripeptides, sugars, and other compounds such as glucosamine. At least one R group is substituted with a fatty moiety, and least one reactive group is substituted with a polyethoxy or polyethoxy/polypropoxy group, wherein the polyethoxy or the polyethoxy/polypropoxy group comprises between 10 and 19 ethoxy or propoxy/ethoxy substituents, respectively.

In several formulations, the ethoxylated fatty moiety is about 0.1% to greater than 99% by weight of the transdermal delivery composition described herein.

In some embodiments of the invention, the transdermal delivery composition further comprises an alcohol and/or water and/or an aqueous adjuvant. In some embodiments, the aqueous adjuvant is a plant extract from the family of *Liliaceae,* such as *Aloe Vera.* Other embodiments of the invention include the transdermal delivery composition described above, wherein about 0/1% to 15% by weight or volume is alcohol or 0.1% to 15% is water or both, or wherein about 0.1 % to 85% by weight or volume is water or *Aloe Vera* or another aqueous adjuvant.

Alcohol, water, and other aqueous adjuvants are not present in some formulations of the transdermal delivery composition described herein. It has been discovered that some delivered agents (e.g., steroids) are soluble and stable in ethoxylated oil in the absence of alcohol or water and some delivered agents are soluble and stable in ethoxylated oil/alcohol emulsions, ethoxylated oil/water emulsions, ethoxylated oil/alcohol/water emulsions, and ethoxylated oil/alcohol/water/*Aloe Vera* emulsions. In particular, it was found that a particular *Aloe Vera,* alcohol, or water mixture was not essential to obtain a transdermal delivery composition provided that an appropriately ethoxylated oil was mixed with the delivered agent. That is, the alcohol, water, and *Aloe Vera* can be removed from the formulation by using a light oil *(e.g.,* macadamia nut oil) that has been ethoxylated to approximately 10-19 ethoxylations/molecule, desirably 11-19 ethoxylations/molecule, more desirably 12-18 ethoxylations/molecule, still more desirably 13-17 ethoxylations/molecule, preferably 14 -16 ethoxylations/molecule and most preferably 15 or16 ethoxylations/molecule. For example, some ethoxylated oils (*e.g.,* macadamia nut oil comprising, consisting of or consisting essentially of 10, 11, 12, 13, 14, 15, 16, 17, 18, or 19 ethoxylations/molecule) can deliver low and high molecular weight peptides (e.g., collagen and fragments of collagen) or amino acids in the absence of alcohol and *Aloe Vera.* Some embodiments, however, have a ratio of ethoxylated lipid:alcohol:aqueous adjuvant selected from the group consisting of 1:1:4, 1:1:14, 3:4:3, and 1:10:25.

In still other embodiments, the transdermal delivery compositions described herein can also include fragrances, creams, bases and other ingredients that stabilize the formulation, facilitate delivery, or protect the delivered agent from degradation (e.g., agents that inhibit DNAse, RNAse, or proteases).

The transdermal delivery compositions described herein are useful for the delivery of a wide variety of delivered agents. In certain embodiments, the transdermal delivery composition comprises delivered agents that are hormones. In some embodiments, the delivered agent is a peptide hormone. Non-limiting examples of peptide hormones include oxytocin, vasopressin, melanocyte-stimulating hormone, corticortropin, lipotropin, thyrotropin, growth hormone, prolactin, luteinizing hormone, human chorionic gonadotropin, follicle stimulating hormone, corticotropin-releasing factor, gonadotropin-releasing factor, prolactin-releasing factor, prolactin-inhibiting factor, growth-hormone releasing factor, somatostatin, thyrotropin-releasing factor, calcitonin gene-related peptide, parathyroid hormone, glucagon-like peptide 1, glucose-dependent insulinotropic polypeptide, gastrin, secretin, cholecystokinin, motilin, vasoactive intestinal peptide, substance P, pancreatic polypeptide, peptide tyrosine tyrosine, neuropeptide tyrosine, amphiregulin, insulin, glucagon, placental lactogen, relaxin, angiotensin II, calctriol, atrial natriuretic peptide, melatonin, and insulin.

In other embodiments, the delivered agent is a non-peptide hormone. Non-limiting examples of hormones that are not peptide hormones useful in embodiments include thyroxine, triiodothyronine, calcitonin, estradiol, estrone, progesterone, testosterone, cortisol, corticosterone, aldosterone, epinephrine, norepinepherine, androstiene, or calcitriol.

Other peptides such as collagen, or fragments thereof, are delivered agents in certain embodiments.

In additional embodiments, the delivered agent is a pharmacologically active small compound. For example, in certain embodiments, the delivered agent is an anesthetic such as articaine, procaine, tetracaine, chloroprocaine and benzocaine, novocain, mepivicaine, bupivicaine, benzocaine, or lidocaine. Analgesics are delivered agents in other embodiments. Thus, in certain embodiments the delivered agent is tramadol hydrochloride, fentanyl, metamizole, morphine sulphate, ketorolac tromethamine, hydrocodone, oxycodone, morporine, loxoprofen, Capsaicin, or Boswellin.

Other pharmacologically active compounds that are suitable delivered agents include non-steroidal anti-inflammatory drugs ("NSAIDs"). Thus, in embodiments of the invention the delivered agent is ibuprofen (2-(isobutylphenyl)-propionic acid); methotrexate (N-[4-(2, 4 diamino 6 - pteridinyl - methyl] methylamino] benzoyl)-L-glutamic acid); aspirin (acetylsalicylic acid); salicylic acid; diphenhydramine (2-(diphenylmethoxy)-NN-dimethylethylamine hydrochloride); naproxen (2-naphthaleneacetic acid, 6-methoxy-9-methyl-, sodium salt, (-)); phenylbutazone (4-butyl-1,2-diphenyl-3,5-pyrazolidinedione); sulindac-(2)-5-fluoro-2-methyl-1-[[p-(methylsulfinyl)phenyl]methylene-]-1H-indene-3-acetic acid; diflunisal (2',4', -difluoro-4-hydroxy-3-biphenylcarboxylic acid; piroxicam (4-hydroxy-2-methyl-N-2-pyridinyl-2H-1, 2-benzothiazine-2-carboxamide 1, 1-dioxide, an oxicam; indomethacin (1-(4-chlorobenzoyl)-5-methoxy-2-methyl-H-indole-3-acetic acid); meclofenamate sodium (N-(2, 6-dichloro-m-tolyl) anthranilic acid, sodium salt, monohydrate); ketoprofen (2- (3-benzoylphenyl)-propionic acid; tolmetin sodium (sodium 1-methyl-5-(4-methylbenzoyl-1H-pyrrole-2-acetate dihydrate); diclofenac sodium (2-[(2,6-dichlorophenyl)amino] benzeneatic acid, monosodium salt); hydroxychloroquine sulphate (2-{[4-[(7-chloro-4-quinolyl) amino] pentyl] ethylamino}ethanol sulfate (1:1); penicillamine (3-mercapto-D-valine); flurbiprofen ([1,1-biphenyl]-4-acetic acid, 2-fluoro-alphamethyl-, (+-.)); cetodolac (1-8- diethyl-13,4,9, tetra hydropyrano-[3-4-13] indole-1-acetic acid; mefenamic acid (N-(2,3-xylyl)anthranilic acid; and diphenhydramine hydrochloride (2-diphenyl methoxy-N, N-di-methylethamine hydrochloride).

In other embodiments, the delivered agent is a steroidal anti-inflammatory compound, such as hydrocortisone, prednisolone, triamcinolone, or piroxicam.

In yet other embodiments, the delivered agent is an anti-infective agent. By way of example, in some embodiments, the delivered agent is an antimicrobial or antifungal agent such as amoxicillin, clavulanate potassium, itraconazole, flucanazole, erythromycin ehtysuccinate, acetyl sulfisoxazole, penicillin V, erythromycin, azithromycin, tetracycline, ciproflaxin, gentamycin sulfathiazole. In still other embodiments, the delivered agent is an anti-viral compound, such as for example acyclovir, lamivudine, indinavir sulfate, stavudine, saquinavir, ritonavir or hepsysls.

In still other embodiments, the delivered agent is a nucleic acid. In some embodiments, the nucleic acid is an oligonulcoeitde consisting of cysteine and guanidine, e.g., a CpG molecule. In further embodiments, the nucleic acid is a polynucleotide. In some embodiments, the polynucleotide comprises a nucleic acid sequence that is capable of eliciting an immune response from an animal. For example, in some embodiments, the nucleic acid comprises nucleic acid sequences from HIV, influenza A virus, hepatitis C virus, hepatitis A virus, hepatitis B virus, hantavirus, SARS, or sequences encoding members of the Inhibitor of Apoptosis family of proteins.

Embodiments of the transdermal delivery compositions disclosed herein also include transdermal delivery systems that comprise adjuvants and immunogenic compositions. Thus, some formulations of transdermal delivery compositions comprise an immunogenic peptide or nucleic acid encoding said peptide, a vaccine, such as a DNA vaccine, polypeptide vaccine, or other vaccine, and an adjuvant, such as aluminium hydroxide, calcium phosphate, cytokines (such as, e.g., interleukin-12 (IL-12)), co-stimulatory molecules (such as, e.g., B7-1 (CD80) or B7-2 (CD86)), and haptens, such as dinitrophenyl (DNP), and the like.

In yet other embodiments, the delivered agent is an immune response modifier. For example, in some embodiments, the delivered immune response modifier is an imidazoquinoline amine including, but not limited to, substituted imidazoquinoline amines. For example in some embodiments the delivered agent is an amide substituted imidazoquinoline amine, a sulfonamide substituted imidazoquinoline amine, a urea substituted imidazoquinoline amine, an aryl ether substituted imidazoquinoline amine, a heterocyclic ether substituted imidazoquinoline amine, an amido ether substituted imidazoquinoline amine, a sulfonamido ether substituted imidazoquinoline amine, a urea substituted imidazoquinoline ether, a thioether substituted imidazoquinoline amine, or a 6-, 7-, 8-, or 9-aryl or heteroaryl substituted imidazoquinoline amine. In other embodiments, the delivered agent is a tetrahydroimidazoquinoline amine such as an amide substituted tetrahydroimidazoquinoline amine, a sulfonamide substituted tetrahydroimidazoquinoline amine, a urea substituted tetrahydroimidazoquinoline amine, an aryl ether substituted tetrahydroimidazoquinoline amine, a heterocyclic ether substituted tetrahydroimidazoquinoline amine, an amido ether substituted tetrahydroimidazoquinoline amine, a sulfonamido ether substituted tetrahydroimidazoquinoline amine, a urea substituted tetrahydroimidazoquinoline ether, or a thioether substituted tetrahydroimidazoquinoline amine. In other embodiments, the deleivered agent is an imidazopyridine amine such as an amide substituted imidazopyridine amine, a sulfonamide substituted imidazopyridine amine, a urea substituted imidazopyridine amine, an aryl ether substituted imidazopyridine amine, a heterocyclic ether substituted imidazopyridine amine, an amido ether substituted imidazopyridine amine, a sulfonamido ether substituted imidazopyridine amine, a urea substituted imidazopyridine ether, or a thioether substituted imidazopyridine amines. In yet other embodiments, the delivered agent is a 1,2-bridged imidazoquinoline amine; 6,7-fused cycloalkylimidazopyridine amine, a idazonaphthyridine amine, a tetrahydroimidazonaphthyridine amines, an oxazoloquinoline amine, a thiazoloquinoline amine; an oxazolopyridine amine, a thiazolopyridine amine, a oxazolonaphthyridine amine, a thiazolonaphthyridine amine, a 1H-imidazo dimers fused to a pyridine amine, a quinoline amine, a tetrahydroquinoline amine, a naphthyridine amine, or a tetrahydronaphthyridine amine.

In further embodiments, the immune response modifier is a purine derivative, an imidazoquinoline amide derivative, a 1H-imidazopyridine derivative, a benzimidazole derivatives, a derivative of a 4-aminopyrimidine fused to a five membered nitrogen containing heterocyclic ring (including adenine derivatives), a 3-.beta.-D-ribofuranosylthiazolo[4,5-d]pyri- midine derivative, or a 1H-imidazopyridine derivatives.

Examples of particular immune response modifier compounds useful as delivered agents include 2-propyl[1,3]thiazolo[4,5-c]quinolin-4-amine, 4-amino-.alpha.,.alpha.-dimethyl-1H-imidazo[4,5-c]quinoline-1-ethanol, and 4-amino-2-(ethoxymethyl)-.alpha.,.alpha.-dimethyl-6,7- ,8,9-tetrahydro-1H-imidazo[4,5-c]quinoline-1-ethanol. Other examples of Immune response modifier compounds include N-[4-(4-amino-2-butyl-1H-imidazo[4,5-c][1,5]naphthyridin-1-yl)butyl]-N'-c- yclohexylurea, 2-methyl-1-(2-methylpropyl)-1H-imidazo[4,5-c][1,5]naphthyri- din-4-amine, 1-(2-methylpropyl)-1H-imidazo[4,5-c][1,5]naphthyridin-4-amine-, N-{2-[4-amino-2-(ethoxymethyl)-1H-imidazo[4,5-c]quinolin-1-yl]-1,1-dimet- hylethyl}methanesulfonamide, N-[4-(4-amino-2-ethyl-1H-imidazo[4,5-c]quinol- in-1-yl)butyl]methanesulfonamide, 2-methyl-1-[5-(methylsulfonyl)pentyl]-1H- -imidazo[4,5-c]quinolin-4-amine, N-[4-(4-amino-2-propyl-1H-imidazo[4,5-c]q- uinolin-1-yl)butyl]methanesulfonamide, 2-butyl-1-[3-(methylsulfonyl)propyl- ]-1H-imidazo[4,5-c]quinoline-4-amine, 2-butyl-1-{2-[(1-methylethyl)sulfony- 1]ethyl}-1H-imidazo[4,5-c]quinolin-4-amine, N-{2-[4-amino-2-(ethoxymethyl)- -1H-imidazo[4,5-c]quinolin-1-yl]-1,1-dimethylethyl}-N'-cyclohexylurea, N- {2-[4-amino-2-(ethoxymethyl)-1 H-imidazo [4,5-c]quinolin-1-yl]-1,1-dimethylethyl}cyclohexanecarboxamide, N-{2-[4-amino-2-(ethoxymethyl)-1H-imidazo[- 4,5-c]quinolin-1-yl]ethyl}-N'-isopropylurea. Resiquimod, and 4-amino-2-ethoxymethyl-.alpha.,.alpha.-dimethyl-1H-imidazo[4,5-c]quinolin- e-1-ethanol.

In certain embodiments, the delivered agent is an analgesic. Non-limiting examples of analgesiscs include tramadol hydrochloride, fentanyl, metamizole, morphine sulphate, ketorolac tromethamine, morphine, and loxoprofen sodium. In other embodiments, the delivered agent is a migraine therapeutic, such as ergotamine, melatonin, sumatriptan, zolmitriptan, or rizatriptan.

In yet other embodiments, the delivered agent is an imaging component, such as iohexol, technetium, TC99M, sestamibi, iomeprol, gadodiamide, oiversol, and iopromide. Diagnostic contrast components such as alsactide, americium, betazole, histamine, mannitol, metyraphone, petagastrin, phentolamine, radioactive B12, gadodiamide, gadopentetic acid, gadoteridol, perflubron are delivered agents in certain embodiments.

Another aspect of the invention concerns methods of making lipopsheres useful for transdermal delivery of a delivered agent. In one embodiment, a liposphere is made by identifying a delivered agent for incorporation into a liposphere and mixing said delivered agent with an ethoxylated or propoxylated fatty moiety, ethoxylated or propoxylated lipid moiety, or ethoxylated or propoxylated multifunctional backbone, wherein said ethoxylated fatty moiety, lipid moiety, or multifunctional backbone has between 10 and 19 ethoxylations per molecule. In preferred embodiments, the fatty moiety, or at least one of the fatty components of the lipid moiety or multifunctional backbone has a carbon chain length of between about 10 and 24 carbon residues.

The formulations described herein are placed into a vessel that is joined to an applicator such that the active ingredients can be easily provided to a subject. Applicators include, but are not limited to, roll-ons, bottles, jars, tubes, sprayer, atomizers, brushes, swabs, gel dispensing devices, and other dispensing devices.

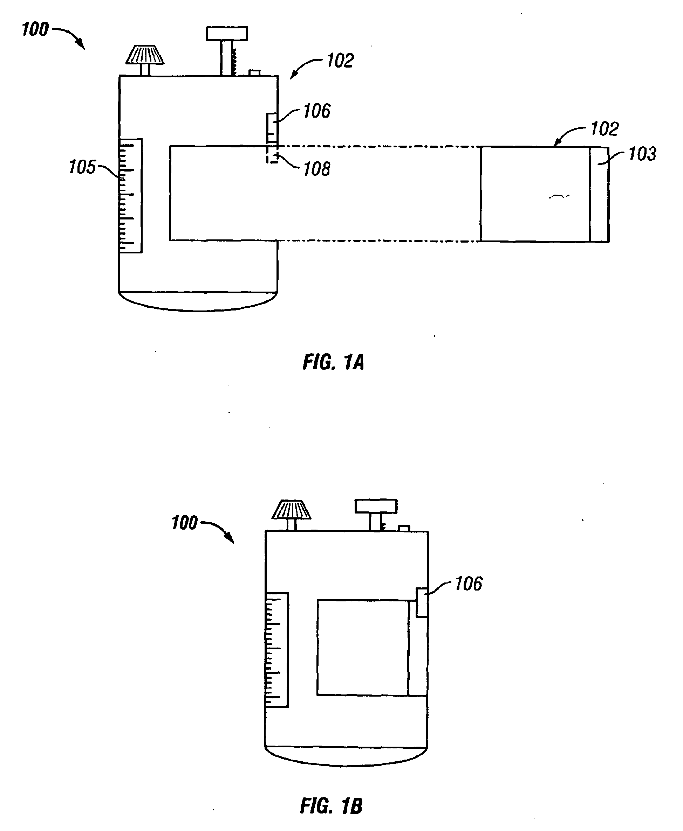

Aspects of the present invention also concern compositions comprising a transdermal delivery and a transdermal delivery device, which provides a measured amount of said transdermal delivery system. Accordingly, desired dosages of delivered agents can be delivered to a subject in need. An exemplary transdermal delivery device is depicted in Figures 1A-4B.

Yet other aspects of the present invention relate to methods of delivering an amount of a transdermal delivery composition comprising providing a transdermal delivery composition within a transdermal delivery device, wherein the device is designed to administer a measured amount of the transdermal delivery composition and providing a transdermal delivery composition to be administered to a subject.

Several methods of using the transdermal delivery compositions are also embodiments. For example, one approach involves a method of reducing pain or inflammation by using a transdermal delivery composition that comprises an anti-inflammatory molecule (*e.g*., an NSAID or MSM) on a subject in need of a reduction of pain or inflammation. Monitoring the reduction in inflammation may also be desired as part of a rehabilitation program.

NSAIDs and other chemotherapeutic agents have also been shown to improve the health, welfare, or survival of subjects that have cancer or Alzheimer's disease. The tendency of these compounds to cause adverse side effects such as gastrointestinal irritation liver and kidney problems renders them particularly desirable transdermal delivery agents. Accordingly, some embodiments concern methods of using transdermal delivery compositions that comprise delivered agents (*e.g*., any one or combination of the NSAIDs disclosed above or other chemotherapeutic agents such as fluorouracil) to treat or prevent cancer or hyperproliferative cell disorders (*e.g.,* basal cell carcinoma or actinic keratosis.) For example, a method to improve the health, welfare, or survival of a subject that has cancer or Alzheimer's disease or a method of treating or preventing cancer or Alzheimer's disease in said subject can be conducted by using a transdermal delivery composition that comprises a COX enzyme inhibitor and providing said transdermal delivery composition to said subject.

Some formulations of transdermal delivery compositions can be used to reduce oxidative stress to cells, tissues and the body of a subject. For example, a method to improve the health, welfare, or survival of a subject that is in need of a reduction in oxidative stress to a cell, tissue, or the body as a whole involves providing to said subject a transdermal delivery composition that comprises an antioxidant such as ascorbic acid, tocopherol or tocotrienol or an anti-stress compound such as Bacocalmine (Bacopa Monniera Extract obtained from Sederma Laboratories). Methods of treating or preventing diseases or conditions associated with oxidative stress or vitamin deficiency and methods of reducing an oxidative stress or a vitamin deficiency in a subject in need thereof are also embodiments.

Other formulations of transdermal delivery composition can be used to reduce psoriasis or eczema or a related condition or can be used to promote wound healing in a subject in need thereof. By one approach, a transdermal delivery composition that comprises peptides that promote wound healing (*e.g*., peptides comprising the sequence LKEKK (**SEQ. ID. NO:1),** are provided to a subject in need of a treatment or reduction in psoriasis or eczema or a condition associated with psoriasis or eczema (*e.g*., allergies) or treatment of a wound.

An exemplary formulation for the treatment of Psoriasis, or Eczema is as follows:

| | |
|---|---|
| Hepsyl | 2 Grams |
| Distilled Water with Sodium Bi Carbonate (to alter ph to 8.4 +/- 0.2) | 18 mls |
| Ethoxylated Macadamia Nut Oil | 20 mls |
| Ethyl Alcohol Anhydrous | 20 mls |

Admix in the listed order and pour off into 60 ml roll on bottles and apply directly to affected areas morning and evening.

Other formulations of transdermal delivery composition can be used to relax the muscles of a subject. By one approach, a transdermal delivery composition that comprises a compound that relaxes the muscles (*e.g*., chlorzoxazone or ibuprofen) is provided to a subject in need of a muscle relaxant. Accordingly methods of treating or preventing muscle soreness are embodiments.

Other formulations of transdermal delivery composition can be used to raise the levels of a hormone in a subject in need thereof. By one approach, a transdermal delivery composition that comprises a hormone *(e.g.,* any one of or combination of the hormones disclosed above or derivatives or functional analogues thereof) is provided to a subject in need thereof. Accordingly methods of treating or preventing a hormone deficiency or methods of increasing the level of a hormone in a subject using one of the transdermal delivery compositions described herein are embodiments.

Other formulations of transdermal delivery composition can be used to raise the levels of a hormone, for example, growth factor in a subject in need thereof. By one approach, a transdermal delivery composition that comprises a growth factor (*e.g*., a growth factor contained in Bioserum, which is obtainable through Atrium Biotechnologies of Quebec City, Canada) is provided to a subject in need thereof. In other embodiments, a transdermal delivery composition comprising a peptide that comprises the sequence LKEKK (SEQ ID NO:1) is provided to a subject in need of an increase in a growth factor. Accordingly methods of treating or preventing a growth factor deficiency or methods of increasing the level of a growth factor in a subject using one of the transdermal delivery compositions described herein are embodiments. By another approach, a transdermal delivery composition that comprises oxytocin, vasopressin, insulin, melanocyte-stimulating hormone, corticortropin, lipotropin, thyrotropin, growth hormone, prolactin, luteinizing hormone, human chorionic gonadotropin, follicle stimulating hormone, corticotropin-releasing factor, gonadotropin-releasing factor, prolactin-releasing factor, prolactin-inhibiting factor, growth-hormone releasing factor, somatostatin, thyrotropin-releasing factor, calcitonin, calcitonin gene-related peptide, parathyroid hormone, glucagon-like peptide 1, glucose-dependent insulinotropic polypeptide, gastrin, secretin, cholecystokinin, motilin, vasoactive intestinal peptide, substance P, pancreatic polypeptide, peptide tyrosine tyrosine, neuropeptide tyrosine, amphiregulin, insulin, glucagon, placental lactogen, relaxin, angiotensin II, atrial natriuretic peptide, melatonin, thyroxine, triiodothyronine, estradiol, estrone, progesterone, testosterone, cortisol, corticosterone, aldosterone, epinephrine, norepinepherine, or calctriol, is provided to a subject in need of the same.

Other formulations of the transdermal delivery composition described herein are used to brighten the skin, reduce age spots or skin discolorations, reduce stretch marks, reduce spider veins, or add dyes, inks, (*e.g*., tattoo ink), perfumes, or fragrances to the skin of a subject. In some embodiments, for example, transdermal delivery compositions that comprise a compound that brightens the skin or reduces age spots or skin discolorations (*e.g*., Melaslow, a citrus-based melanin (tyrosinase) inhibitor obtainable from Revivre Laboratories of Singapore or Etioline, a skin brightener made from an extract from the *Mitracarpe* leaf obtainable from Krobell, USA), or a compound that reduces stretch marks (Kayuuputih Eucalyptus Oil, obtainable from Striad Laboratories) or add dyes, inks, (*e.g*., tattoo ink), perfumes, or fragrances are provided to the skin of a subject.

It has also been discovered that ethoxylated oil by itself, preferably macadamia nut oil having 10-19 ethoxylations/molecule (*i.e.,* 10, 11, 12, 13, 14, 15, 16, 17, 18, or 19 ethoxylations/molecule), has therapeutic and cosmetic properties. For example, application of an ethoxylated oil (*e.g*., macadamia nut oil having 16 ethoxylations/molecule) was found to reduce stretch marks and spider veins on a subject in need thereof. Application of an ethoxylated oil (*e.g*., macadamia nut oil having 16 ethoxylations/molecule) to a burn (*e.g*., a sun burn or a skin burn obtained from overheated metal) was found to significantly expedite recovery from the burn, oftentimes without blistering. Accordingly, some embodiments concern a transdermal delivery composition comprising an ethoxylated oil (*e.g*., macadamia nut oil that was ethoxylated 10-19 ethoxylations per molecule, 11-19 per molecule, 12-18 ethoxylations per molecule, 13-17 ethoxylations per molecule, 14-16 ethoxylations per molecule, or 15 ethoxylations per molecule) and these compositions are used to reduce the appearance of stretch marks and spider veins or facilitate the recovery from bums of the skin.

In addition to the delivery of low and medium molecular weight delivered agents, several compositions that have high molecular weight delivered agents (*e.g*., collagens) and methods of use of such compositions are embodiments of the invention. Preferred formulations of the transdermal delivery composition comprise a collagen (natural or synthetic) or fragment thereof at least 2, 4, 6, 8, 10, 12, 14, 16, 18, 20, 24, 30, 40, 50, 100, 250, 500, 1000, 1500, 2000, 2500, 3000, 5000, or more amino acids in length and these compositions are used to reduce wrinkles and fine lines on a subject.

For example, some embodiments concern a transdermal delivery composition comprising an ethoxylated fatty moiety, an ethoxylated lipid (*e.g*., macadamia nut oil that was ethoxylated 10-19 ethoxylations per molecule, 11-19 per molecule, 12-18 ethoxylations per molecule, 13-17 ethoxylations per molecule, 14-16 ethoxylations per molecule, or 15 ethoxylations per molecule), or an ethoxylated transdermal delivery enhancer and a therapeutically effective amount of a collagen or fragment thereof (*e.g*., marine collagen). In some aspects of the invention, a transdermal delivery composition comprising an ethoxylated oil and collagen also contains water and/or an alcohol and/or an aqueous adjuvant such as *Aloe Vera.*

In different embodiments of this transdermal delivery composition, the collagen has a molecular weight less than, or equal to 6,000 daltons or greater than 6,000 daltons. Thus, in some embodiments, the collagen can have an approximate molecular weight as low as 2,000 daltons or lower. In other embodiments, the molecular weight is from about 300,000 daltons to about 500,000 daltons. Further, these transdermal delivery compositions can have a therapeutically effective amount of collagen or fragment thereof by weight or volume that is 0.1% to 85.0%. The collagen can be any natural or synthetic collagen, for example, Hydrocoll EN-55, bovine collagen, human collagen, a collagen derivative, marine collagen, Solu-Coll, or Plantsol, recombinant or otherwise man made collagens or derivatives or modified versions thereof (*e.g*., protease resistant collagens). As above, an apparatus having a vessel joined to an applicator that houses the transdermal delivery composition containing collagen is also an embodiment and preferred applicators or dispensers include a roll-on or a sprayer.

Accordingly, some of the embodied methods concern the reduction of wrinkles and or the improvement of skin tone by using a transdermal delivery composition comprising an ethoxylated oil and a collagen and/or a fragment thereof. Some formulations to be used to reduce wrinkles and improve skin tone include an ethoxylated fatty moiety, an ethoxylated lipid moiety (*e.g*., macadamia nut oil that was ethoxylated 10-19 ethoxylations per molecule, 11-19 per molecule, 12-18 ethoxylations per molecule, 13-17 ethoxylations per molecule, 14-16 ethoxylations per molecule, or 15 ethoxylations per molecule), or an ethoxylated transdermal delivery enhancer, and a therapeutically effective amount of a collagen or fragment thereof (*e.g*., marine collagen) that is at least 2, 4, 6, 8, 10, 12, 14, 16, 18, 20, 24, 30, or 40 amino acids in length. Some formulations that can be used to practice the method above include a transdermal delivery composition comprising an ethoxylated oil and collagen or fragment thereof, as described above, and, optionally, water and/or an alcohol and/or an aqueous adjuvant such as *Aloe Vera.* For example, by one approach, a method of reducing wrinkles or improving skin tone is practiced by identifying a subject in need thereof and providing said subject a transdermal delivery composition, as described herein and, optionally, monitoring the subject for restoration or improvement of skin tone and the reduction of wrinkles.

### BRIEF DESCRIPTION OF THE DRAWINGS

Embodiments of a transdermal delivery device are depicted in Figures 1A through 4B. In aspects of the invention, a transdermal delivery system comprises a transdermal delivery composition and a transdermal delivery device.
Figure 1A schematically depicts in an exploded state a dispenser for delivery of a transdermal drug delivery system fluid comprising a removable cartridge.
Figure 1B schematically depicts the dispenser of Figure 1A in an assembled state.
Figure 2 schematically depicts a cross section of the dispenser of Figure 1B.
Figure 3A schematically depicts a cross section of the upper portion of a partially filled dosing chamber having an upper wall configured allow air to escape, but prevent fluid from escaping.
Figure 3B schematically depicts the upper portion of the dosing chamber of Figure 3A, where the dosing chamber is full and fluid is prevented from escaping.
Figure 4A schematically depicts a cross-section of the dispenser of Figure 2, taken along the line 4, wherein the slidable member is in a first position permitting filling of the dosing chamber.
Figure 4B schematically depicts the cross-section of Figure 4A, wherein the slidable member is in a second position permitting delivery of the dosed fluid.

### DETAILED DESCRIPTION OF THE INVENTION

Several transdermal delivery compositions and devices for providing said compositions to a subject are described herein. Embodiments of the invention can be used to transdermally deliver low or high (or both low and high) molecular weight pharmaceuticals, prophylactics, diagnostics, and cosmetic agents to a subject. The transdermal delivery compositions disclosed herein are useful for the delivery of various types of compounds including but not limited to nucleic acids, peptides, modified peptides, small molecules, immunogenic preparations, and the like. Some embodiments include transdermal delivery compositions that can administer compounds having molecular weights greater than 6,000 daltons. One embodiment, for example, includes a transdermal delivery composition that can administer a therapeutically effective amount of a non-steroidal anti-inflammatory drug (NSAID). Still more embodiments concern transdermal delivery compositions that can administer hormones, anesthetics, collagen preparations *e.g*., soluble collagens, hydrolyzed collagens, and fragments of collagen), cardiovascular pharmaceutical compounds, anti-infective compounds (*e.g*. antibiotics and antiviral compounds), diabetes-related treatments, immunogenic compositions, vaccines, immune response modifiers, enzyme inhibitors, analgesics (*e.g*., a formulation comprising capsaicin or Boswellin or both), migraine therapies, sedatives, imaging and contrast compounds. These examples are provided to demonstrate that embodiments of the invention can be used to transdermally deliver both low and high molecular weight compounds and it should be understood that many other molecules can be effectively delivered to the body, using the embodiments described herein, in amounts that are therapeutically, prophylactically, or cosmetically beneficial.

Some transdermal delivery compositions described herein comprise a liposphere that is configured to deliver a wide variety of delivered agents. As used herein, the term "liposphere" refers to a spherical or ovoid-shaped structure comprising an ethoxylated or propoxylated fatty moiety, which contains or is associated with (*e.g*., joined to) a delivered agent. That is, the term "lipospheres" includes, but is not limited to, liposomes that comprise an ethoxylated or propoxylated oil, fatty acid, fatty amine, or fatty alcohol. Accordingly, the term "fatty moiety" can refer to a fatty acid, a fatty alcohol, fatty amine, or other fatty acid derivative. The ethoxylated fatty moiety or lipid moiety has both hydrophobic and hydrophilic properties, in that the hydrocarbon chain of the fatty moiety or lipid moiety is hydrophobic, and the polyethoxy groups confer hydrophilicity on the molecule. The preparation of propoxylated fatty moieties and lipid moieties is well known. (*See, e.g.,* Raths et al., supra). Due to their similarity in structure, propoxylated fatty moieties and lipids will share many of the same characteristics as ethoxylated fatty moieties and lipids. Accordingly, fatty moieties and lipid moieties that are propoxylated or ethoxylated and propoxylated are contemplated penetration enhancers and transdermal delivery enhancers.

In the embodiments disclosed herein, the number of ethoxylations is adjusted to between 10 and 19 ethoxylations per molecule to achieve optimal transdermal delivery of the delivered agent. Ethoxylated fatty acids, fatty alcohols, and fatty amines, are commercially available (*e.g*., Ethox Chemicals, LLC, Greenville, SC; A&E Connock, Ltd., Hampshire, England; Floratech, Glibert, AZ). Alternatively ethoxylated fatty moieties are synthesized using methods known to those skilled in the art (*See,* U.S. Patent No. 6,300,508 to Raths et al.; U.S. Patent No. 5,936,107 to Raths et al.) by reacting fatty moieties with ethylene oxide.

By way of example, ethoxylated oils can be prepared using a two-step process that starts with trans-esterification with added glycerol followed by ethoxylation of the product of this reaction. Trans-esterification is performed by any method available to those skilled in the art, such as heating an ester, such as the glycerol esters present in natural vegetable oils, in the presence of another alcohol or polyol, such as glycerol, in the presence of a catalyst. Catalysts useful in the transesterification reaction include gaseous catalysts, such hydrochloric acid bubbled through the reaction mixture. Alternatively, solid catalysts such as zinc oxide or the acetates of copper, cobalt or zinc can also be used. The transesterificaiton reaction produces one or two fatty acids attached to a molecule of glycerol. The ratio of mono- and di-esters can be controlled by the amount of glycerol used in the reaction (i.e. higher ratios of glycerol:oil will yield more reactive -OH and fewer fatty acid moieties per molecule, and a lower ratio of glycerol:oil would give more fatty acids, as is apparent to those skilled in the art. The hydroxyl groups are subsequently reacted with ethylene oxide in the presence of an appropriate catalyst, (e.g., aluminum) using methods known to those skilled in the art.

Purified fatty moieties commercially available from a variety of sources (*e.g*., SIGMA-Aldrich, St. Louis, MO) are suitable for use in the transdermal delivery compositions described above.

Alternative embodiments of transdermal delivery compositions described herein comprise a penetration enhancer that includes an ethoxylated lipid moiety. It was discovered that ethoxylated lipids (*e.g*., ethoxylated oils) can be used as transdermal penetration enhancers in that they effectively transport low and high molecular weight compounds through the skin. It was also discovered that ethoxylated oils, by themselves, have therapeutic and cosmetic applications (*e.g*., the reduction of the appearance of spider veins and stretch marks or promoting expedited recovery from burns to the skin). Ethoxylated lipids can be created in many ways, however, a preferred approach involves the reaction of ethylene oxide with a vegetable, nut (*e.g*., macadamia nut), animal, or synthetic oil. In embodiments where the transdermal delivery composition comprises an ethoxylated oil, it is contemplated that in some embodiments, ethoxylated fatty moieties are used to fortify or supplement ethoxylated oils in some embodiments. By way of example, ethoxylated macadamia nut oil can be fortified with ethoxylated palmitic or oleic acid.

Several transdermal delivery enhancers disclosed herein are compounds having a multifunctional backbone. The multifunctional backbone can be one of many chemical structures that have at least two reactive hydrogen residues, such that the multifunctional backbone is the basis of a transdermal delivery enhancer with least one fatty moiety and at least one polyethoxy group. The reactive hydrogen residues (R) are present in -OH, COOH, SH, and NH₂, groups.

The polyethoxy group has the structure:

-O-(CH₂-CH₂-O-)ₙH

Embodiments wherein n is between 10 and 19 per molecule of transdermal delivery enhancer to possess superior transdermal delivery properties.

In preferred embodiments, the fatty moiety component of the transdermal delivery enhancer has a carbon chain of at least 10 carbon residues. The chain length of the fatty moiety can be for example 10, 12, 14, 16, 18, 20, 22, or 24 residues. Further, the fatty moiety may be saturated, unsaturated, or polyunsaturated.

Desirably, the multifunctional backbone has at least three reactive groups. The reactive groups can be homogeneous. For example, in some embodiments, the multifunctional backbone is a tri-alcohol comprising three -OH groups, such as 1, 2, 3-butanetriol, 1, 2, 4 butantetriol, pyrogallol (1, 2, 3-benezentriol), hydroxyquinol (1, 2, 4-benzenetriol), trimethyololpropane, 1, 2, 6-hexanetriol and the like. Other examples of multifunctional backbones suitable as the foundation of a transdermal delivery enhancer include tri-acids, comprising three carboxylate groups, such as hemi-mellitic acid, trimellitic acid, trimesic acid, nitrilotriacetic acid, and the like. Those skilled in the art will appreciate that other tricarboxylic acids are suitable as multifunctional backbones.

Alternative multifunctional backbones have heterogeneous reactive groups, e.g., a combination of at least two different reactive groups (e.g., a COOH group and an NH₂ group). For example, amino acids such as glutamic acid, aspartic acid, cysteine, glutamine, serine, threonine, tryrosine, and lysine have three reactive groups and are suitable as multifunctional backbones. Similarly, di- and tri-peptides will have three or more reactive groups and are thus suitable as multifunctional backbones.

Triethanolamine, diethanolamine, dimethylolurea, and glucosamine are other exemplary multifunctional backbones with heterogeneous reactive groups.

Simple carbohydrates are small straight-chain aldehydes and ketones with several hydroxyl groups, usually one on each carbon except the functional group. Due to the presence of the multiple -OH groups on carbohydrates such as tetroses, pentoses, hexoses, and so forth, these compounds are another source of multifunctional backbones useful as components of transdermal delivery enhancers. Exemplary carbohydrates that are useful components of transdermal delivery enhancers include glucose, mannose, fructose, ribose, xylose, threose, erythrose, and the like.

Sugar alcohols such as sorbitol, mannitol, xylitol, erythritol, petaerythritol, and inositol are useful components of transdermal delivery enhancers.

Not wanting to be tied to any particular mechanism or mode of action and offered only to expand the knowledge in the field, it is contemplated that the ethoxylated fatty moiety, ethoxylated lipid moiety, or ethoxylated multifunctional backbone encapsulates the delivered agent in a sphere-like composition, forming a "liposphere" that exhibits greatly enhanced transdermal delivery properties.

Each of the disclosed transdermal delivery compositions can contain additional compounds such as alcohols, nonionic solubilizers or emulsifiers. In some compositions, these compounds are added to improve the solubility of the delivered agent or effectiveness or fluidity of the liposphere, penetration enhancer, or transdermal delivery enhancer. Suitable hydrophilic components include, but are not limited to, ethylene glycol, propylene glycol, dimethyl sulfoxide (DMSO), dimethyl polysiloxane (DMPX), oleic acid, caprylic acid, isopropyl alcohol, 1-octanol, ethanol (denatured or anhydrous), and other pharmaceutical grade or absolute alcohols.

Other embodiments of the transdermal delivery compositions comprise an aqueous adjuvant. Aqueous adjuvants include, but are not limited to, water (distilled, deionized, filtered, or otherwise prepared), *Aloe Vera* juice, and other plant extracts such as chlorophyll or *Spirulina.* Thus, several embodiments of the invention have a hydrophobic/hydrophilic component comprising an ethoxylated fatty moiety (*e.g*., palmitoleic acid, oleic acid, or palmitic acid) or an ethoxylated oil (*e.g*., macadamia nut oil, coconut oil, eucalyptus oil, synthetic oils, castor oil, glycerol, corn oil, jojoba oil, or emu oil) and may contain a hydrophilic component comprising an alcohol, a nonionic solubilizer, or an emulsifier (*e.g.,* isopropyl alcohol) and/or, optionally, an aqueous adjuvant, such as water and/or *Aloe Vera* extract.

Other materials can also be components of a transdermal delivery composition of the invention including fragrance, creams, ointments, colorings, and other compounds so long as the added component does not deleteriously affect transdermal delivery of the delivered agent. It has been found that the *Aloe Vera,* which allows for transdermal delivery of high molecular weight delivered agents, including collagen having an average molecular weight greater than 6,000 daltons, can be removed from transdermal delivery compositions comprising a light oil (*e.g*., macadamia nut oil) that has been ethoxylated to the range of 10 - 19 ethoxylations/molecule. Formulations lacking *Aloe Vera* provide the unexpected benefit of efficient transdermal delivery, uniform application and quick penetration making these formulations superior to formulations that contain *Aloe Vera.*

Similarly, formulations of transdermal delivery compositions that lack alcohol provide the unexpected benefit of efficient transdermal delivery, uniform application, and quick penetration without the drying or irritation brought about by the alcohol. Additionally, formulations lacking water or other aqueous adjuvants provide efficient transdermal delivery while maintaining the highest possible concentration of delivered agent and, also, provide for quick penetration without the skin-drying effects seen with some formulations that contain alcohol.

A molecule or a mixture of molecules (*e.g*., a pharmaceutical, chemical, or cosmetic agent) that are delivered to the body using an embodiment of a transdermal delivery composition are termed "delivered agents". A delivered agent that can be administered to the body using an embodiment of the invention can include, for example, a protein or peptide, a sugar, a nucleic acid, a chemical, a lipid, or derivatives of the same. Desirable delivered agents include, but are not limited to, glycoproteins, enzymes, genes, nucleic acids, peptides, drugs, and ceramides. Preferred delivered agents include NSAIDS, collagens or fragments thereof, capsaicin, and Boswellin. In some embodiments, a transdermal delivery composition comprises a combination of any two of the aforementioned delivered agents. Other delivered agents include, for example, hormones, anti-inflammatory drugs, anesthetics, analgesics, sedatives, migraine therapies, cardiovascular pharmaceuticals, anti-infective agents, diabetes-related therapies, vaccines, imaging agents, contrast agents, glucosamine, chondroitin sulfate, MSM, perfumes, melasin, nicotine, nicotine analogs, peptides, amino acids, nucleic acids, and peptidomimetics.

In addition to the aforementioned compositions, methods of making and using the embodiments of the invention are provided. In one aspect, a transdermal delivery composition is prepared by mixing an ethoxylated fatty moiety with a delivered agent.

In another aspect, a transdermal delivery composition is prepared by mixing a hydrophilic component with a hydrophobic component and an aqueous adjuvant. Depending on the solubility of the delivered agent, the delivered agent can be solubilized in either the ethoxylated oil, a hydrophobic, hydrophilic, or aqueous adjuvant or water prior to mixing.

In addition to physical mixing techniques, (*e.g*., magnetic stirring or rocker stirring), embodiments of the methods contemplate heat can be applied to help coalesce the mixture. Desirably, the temperature is not raised above 40°C.

Several formulations of transdermal delivery compositions are within the scope of aspects of the invention. In embodiments wherein the transdermal delivery composition includes an aqueous adjuvant, in further embodiments, the formulation comprises a ratio of hydrophilic component:hydrophobic component:aqueous adjuvant of 3:4:3. The amount of delivered agent that is incorporated into the penetration enhancer depends on the compound, desired dosage, and application. The amount of delivered agent in a particular formulation can be expressed in terms of percentage by weight, percentage by volume, or concentration. Several specific formulations of delivery systems are provided in the Examples described herein.

Methods of treatment and prevention of pain, inflammation, and human disease are also provided. In some embodiments, a transdermal delivery composition comprising an NSAID, capsaicin, Boswellin or any combination thereof is provided to a patient in need of treatment, such as for relief of pain and/or inflammation. A patient can be contacted with the transdermal delivery composition and treatment continued for a time sufficient to reduce pain or inflammation or inhibit the progress of disease.

Additionally, a method of reducing wrinkles, removing age spots, and increasing skin tightness and flexibility is provided. By this approach, a transdermal delivery composition comprising a collagen or fragment thereof or melaslow or other skin brightening agent is provided to a patient in need, the patient is contacted with the transdermal delivery composition, and treatment is continued for a time sufficient to restore a desired skin tone (*e.g*., reduce wrinkles, age spots, or restore skin brightness, tightness and flexibility). In the disclosure below, there is provided a description of several of the delivered agents that can be incorporated into the transdermal delivery compositions described herein.

### Delivered agents

Many different delivered agents can be incorporated into the various transdermal delivery compositions described herein. While the transdermal delivery of molecules having a molecular weight in the vicinity of 6000 daltons has been reported, it has not been possible, until the present invention, to administer molecules of greater size transdermally. *(See* U.S. Pat. No. 5,614,212 to D'Angelo et al.).

The described embodiments can be organized according to their ability to deliver a low or high molecular weight delivered agent. Low molecular weight molecules (*e.g*., a molecule having a molecular weight less than 6,000 daltons) can be effectively delivered using an embodiment of the invention and high molecular weight molecules (*e.g*., a molecule having a molecular weight greater than 6,000 daltons) can be effectively delivered using an embodiment of the invention. Desirably, a transdermal delivery composition described herein provides a therapeutically, prophylactically, diagnostically, or cosmetically beneficial amount of a delivered agent having a molecular weight of 50 daltons to less than 6,000 daltons. Preferably, however, a transdermal delivery composition described herein provides a therapeutically, prophylactically, diagnostically, or cosmetically beneficial amount of a delivered agent having a molecular weight of 50 daltons to 2,000,000 daltons or less. That is, a transdermal delivery composition described herein, preferably, provides a delivered agent having a molecular weight of less than or equal to or greater than 50, 100, 200, 500, 1,000, 1,500, 2,000, 2,500, 3,000, 3,500, 4,000, 4,500, 5,000, 5,500, 6,000, 7,000, 8,000, 9,000, 10,000, 11,000, 12,000, 13,000, 14,000, 15,000, 16,000, 17,000, 18,000, 19,000, 20,000, 21,000, 22,000, 23,000, 24,000, 25,000, 26,000, 27,000, 28,000, 29,000, 30,000, 31,000, 32,000, 33,000, 34,000, 35,000, 36,000, 37,000, 38,000, 39,000, 40,000, 41,000, 42,000, 43,000, 44,000, 45,000, 46,000, 47,000, 48,000, 49,000, 50,000, 51,000, 52,000, 53,000, 54,000, 55,000, 56,000, 57,000, 58,000, 59,000, 60,000, 61,000, 62,000, 63,000, 64,000, 65,000, 66,000, 67,000, 68,000, 69,000, 70,000, 75,000, 80,000, 85,000, 90,000, 95,000, 100,000, 125,000, 150,000, 175,000, 200,000, 225,000, 250,000, 275,000, 300,000, 350,000, 400,000, 450,000, 500,000, 600,000, 700,000, 800,000, 900,000, 1,000,000, 1,500,000, 1,750,000, and 2,000,000 daltons.

In one aspect, a low molecular weight compound (*e.g*., a pain relieving substance or mixture of pain relieving substances) is transdermally delivered to cells of the body using an embodiment described herein. The delivered agent can be, for example, any one or more of a number of compounds, including non-steroidal anti-inflammatory drugs (NSAIDs) that are frequently administered systemically. These include ibuprofen (2-(isobutylphenyl)-propionic acid); methotrexate (N-[4-(2, 4 diamino 6 - pteridinyl - methyl] methylamino] benzoyl)-L-glutamic acid); aspirin (acetylsalicylic acid); salicylic acid; diphenhydramine (2-(diphenylmethoxy)-NN-dimethylethylamine hydrochloride); naproxen (2-naphthaleneacetic acid, 6-methoxy-9-methyl-, sodium salt, (-)); phenylbutazone (4-butyl-1,2-diphenyl-3,5-pyrazolidinedione); sulindac-(2)-5-fluoro-2-methyl-1-[[p-(methylsulfinyl)phenyl]methylene-]-1H-indene-3-acetic acid; diflunisal (2',4', -difluoro-4-hydroxy-3-biphenylcarboxylic acid; piroxicam (4-hydroxy-2-methyl-N-2-pyridinyl-2H-1, 2-benzothiazine-2-carboxamide 1, 1-dioxide, an oxicam; indomethacin (1-(4-chlorobenzoyl)-5-methoxy-2-methyl-H-indole-3-acetic acid); meclofenamate sodium (N-(2, 6-dichloro-m-tolyl) anthranilic acid, sodium salt, monohydrate); ketoprofen (2- (3-benzoylphenyl)-propionic acid; tolmetin sodium (sodium 1-methyl-5-(4-methylbenzoyl-1H-pyrrole-2-acetate dihydrate); diclofenac sodium (2-[(2,6-dichlorophenyl)amino] benzeneatic acid, monosodium salt); hydroxychloroquine sulphate (2-{[4-[(7-chloro-4-quinolyl) amino] pentyl] ethylamino}ethanol sulfate (1:1); penicillamine (3-mercapto-D-valine); flurbiprofen ([1,1-biphenyl]-4-acetic acid, 2-fluoro-alphamethyl-, (+-.)); cetodolac (1-8- diethyl-13,4,9, tetra hydropyrano-[3-4-13] indole-1-acetic acid; mefenamic acid (N-(2,3-xylyl)anthranilic acid; and diphenhydramine hydrochloride (2-diphenyl methoxy-N, N-di-methylethamine hydrochloride).

The transdermal delivery compositions described herein, which contain NSAIDs, desirably comprise an amount of the compound that is therapeutically beneficial for the treatment or prevention of disease or inflammation. Several studies have determined an appropriate dose of an NSAID for a given treatment or condition. (*See e.g.,* Woodin, RN, August: 26-33 (1993) and Amadio et al., Postgrduate Medicine, 93(4):73-97 (1993)). The maximum recommended daily dose for several NSAIDs is listed in **TABLE 1**.

A sufficient amount of NSAID can be incorporated into a transdermal delivery composition described herein such that a therapeutically effective amount of NSAID is effectively delivered to a subject. For example, about 0.5ml of the transdermal delivery composition described herein is applied in a single application. A therapeutically effective amount of ibuprofen is about 800mg/dose. Accordingly, a 30 ml bottle containing a transdermal delivery system formulation and ibuprofen can contain 24 grams of ibuprofen such that 800mg of ibuprofen is provided in each 1.0 ml. Because the transdermal delivery compositions described herein can provide a delivered agent in a site-specific manner, a lower total dose of therapeutic agent, as compared to the amounts provided systemically, will provide therapeutic benefit. Additionally, greater therapeutic benefit can be gained by using a transdermal delivery composition described herein because a greater concentration of therapeutic agent (*e.g*., an NSAID) can be provided to the particular site of inflammation. That is, in contrast to systemic administration, which applies the same concentration of therapeutic to all regions of the body, a transdermal delivery composition can site-specifically provide the therapeutic agent and, thereby, provide a much greater regional concentration of the agent than if the same amount of therapeutic were administered systemically.

**TABLE 1**

| Agent | Maximum Recommended Daily Dose |
|---|---|
| Indomethacin | 100 mg |
| Ibuprofen | 3200 mg |
| Naproxen | 1250 mg |
| Fenoprofen | 3200 mg |
| Tolmetin | 2000 mg |
| Sulindac | 400 mg |
| Meclofenamate | 400 mg |
| Ketoprofen | 300 mg |
| Proxicam | 10 mg |
| Flurbiprofen | 300 mg |
| Diclofenac | 200 mg |

Additional embodiments include a transdermal delivery composition that provides a pain relieving mixture comprising capsaicin (*e.g*., oleoresin capsicum) or Boswellin or both. Capsaicin (8-methyl-N-vanillyl-6-nonenamide), the pungent component of paprika and peppers, is a potent analgesic. (*See* U.S. Patent Nos. 5,318,960 to Toppo, 5,885,597 to Botknecht et al., and 5,665,378 to Davis et al., herein expressly incorporated by reference in their entireties). Capsaicin produces a level of analgesia comparable to morphine, yet it is not antagonized by classical narcotic antagonists such as naloxone. Further, it effectively prevents the development of cutaneous hyperalgesia, but appears to have minimal effects on normal pain responses at moderate doses. At high doses capsaicin also exerts analgesic activity in classical models of deep pain, elevating the pain threshold above the normal value. Capsaicin can be readily obtained by the ethanol extraction of the fruit of *Capsicum frutescens* or *Capsicum annum.* Capsaicin and analogs of capsaicin are available commercially from a variety of suppliers, and can also be prepared synthetically by published methods. Aspects of the invention encompass the use of synthetic and natural capsaicin, capsaicin derivatives, and capsaicin analogs.

A form of capsaicin used in several desirable embodiments is oleoresin capsicum. Oleoresin capsicum contains primarily capsaicin, dihydrocapsaicin, nordihydrocapsaicin, homocapsaicin, and homodihydrocapsaicin. The term "capsaicin" collectively refers to all forms of capsaicin, capsicum, and derivatives or modifications thereof. The pungency of these five compounds, expressed in Scoville units, is provided in **TABLE 2.**

**TABLE 2**

| Compound | Pungency x 100,000 SU |
|---|---|
| Capsaicin | 160 |
| Dihydrocapsaicin | 160 |
| Nordihydrocapsaicin | 91 |
| Homocapsaicin | 86 |
| Homodihydrocapsaicin | 86 |

The transdermal delivery compositions that are formulated to contain capsaicin desirably comprise by weight or volume 0.01% to 1.0% capsaicin or 1.0% to 10% oleoresin capsicum. Preferred amounts of this delivered agent include by weight or volume 0.02% to 0.75% capsaicin or 2.0% to 7.0% oleoresin capsicum. For example, the transdermal delivery compositions that contain capsaicin can comprise by weight or volume less than or equal to 0.01%, 0.015%, 0.02%, 0.025%, 0.03%, 0.035%, 0.04%, 0.045%, 0.05%, 0.055%, 0.06%, 0.065%, 0.07%, 0.075%, 0.08%, 0.085%, 0.09%, 0.095%, 0.1%, 0.15%, 0.175%, 0.2%, 0.225%, 0.25%, 0.275%, 0.3%, 0.325%, 0.35%, 0.375%, 0.4%, 0.425%, 0.45%, 0.475%, 0.5%, 0.55%, 0.6%, 0.65%, 0.7%, 0.75%, 0.8%, 0.85%, 0.9%, 0.95%, and 1.0% capsaicin. The transdermal delivery compositions of that contain capsaicin can also comprise an amount of capsaicin by weight or volume that is greater than 1.0%, such as 1.2%, 1.5%, 1.8%, 2.0%, 2.2%, 2.5%, 2.8%, 3.0%, 3.5%, 4.0%, 4.5%, and 5.0%. Similarly, the transdermal delivery compositions that contain oleoresin capsicum can comprise an amount of oleoresin capsicum less than 1.0%, 1.5%, 2.0%, 2.5%, 3.0%, 3.5%, 4.0%, 4.5%, 5.0%, 5.5%, 6.0%, 6.5%, 7.0%, 7.5%, 8.0%, 8.5%, 9.0%, 9.5%, 10.0%, 11.0%, 12.0%, and 13.0%.

Boswellin, also known as Frankincense, is an herbal extract of a tree of the *Boswellia* family. Boswellin can be obtained, for example, from *Boswellia thurifera, Boswellia carteri, Boswellia sacra,* and *Boswellia serrata.* There are many ways to extract Boswellin and Boswellin gum resin and boswellic acids are obtainable from several commercial suppliers (a 65% solution of Boswellic acid is obtainable from Nature's Plus). Some suppliers also provide creams and pills having Boswellin with and without capsaicin and other ingredients. Embodiments of the invention comprise Boswellin and the term "Boswellin" collectively refers to Frankincense, an extract from one or more members of the *Boswellia* family, Boswellic acid, synthetic Boswellin, or modified or derivatized Boswellin.

The transdermal delivery compositions that contain Boswellin desirably comprise 0.1% to 10% Boswellin by weight or volume. Preferred amounts of this delivered agent include 1.0% to 5.0% Boswellin by weight. For example, the transdermal delivery compositions that contain Boswellin can comprise by weight or volume less than or equal to 0.1%, 0.15%, 0.2%, 0.25%, 0.3%, 0.35%, 0.4%, 0.45%, 0.5%, 0.55%, 0.6%, 0.65%, 0.7%, 0.75%, 0.8%, 0.85%, 0.9%, 0.95%, 1.0%, 1.1%, 1.15%, 1.2%, 1.25%, 1.3%, 1.35%, 1.4%, 1.45%, 1.5%, 1.55%, 1.6%, 1.65%, 1.7%, 1.75%, 1.8%, 1.85%, 1.9%, 1.95%, and 2.0%, 2.1%, 2.15%, 2.2%, 2.25%, 2.3%, 2.35%, 2.4%, 2.45%, 2.5%, 2.55%, 2.6%, 2.65%, 2.7%, 2.75%, 2.8%, 2.85%, 2.9%, 2.95%, 3.0%, 3.1%, 3.15%, 3.2%, 3.25%, 3.3%, 3.35%, 3.4%, 3.45%, 3.5%, 3.55%, 3.6%, 3.65%, 3.7%, 3.75%, 3.8%, 3.85%, 3.9%, 3.95%, 4.0%,. 4.1%, 4.15%, 4.2%, 4.25%, 4.3%, 4.35%, 4.4%, 4.45%, 4.4%, 4.45%, 4.5%, 4.55%, 4.6%, 4.65%, 4.7%, 4.75%, 4.8%, 4.85%, 4.9%, 4.95%, and 5.0% Boswellin. The transdermal delivery compositions that contain Boswellin can also comprise amounts of Boswellin by weight that are greater than 5.0%, such as 5.5%, 5.7%, 6.0%, 6.5%%, 6.7%, 7.0%, 7.5%, 7.7%, 8.0%, 8.5%, 8.7%, 9.0%, 9.5%, 9.7%, and 10.0% or greater. Additionally, Boswellin from different sources can be combined to compose the Boswellin component of an embodiment. For example, in one embodiment an extract from *Boswellia thurifera* is combined with an extract from *Boswellia serrata.*

Additional embodiments of the invention comprise a transdermal delivery composition that can administer a pain relieving solution comprising two or more members selected from the group consisting of NSAIDs, capsacin, and Boswellin. The transdermal delivery compositions that include two or more members selected from the group consisting of NSAIDs, capsacin, and Boswellin desirably comprise an amount of delivered agent that can be included in a delivered agent having an NSAID, capsaicin, or Boswellin by itself. For example, if the delivered agent comprises an NSAID, the amount of NSAID that can be used can be an amount recommended in the literature (See *e.g.,* Woodin, RN, August: 26-33 (1993) and Amadio, et al., Postgrduate Medicine, 93(4):73-97 (1993)), or an amount listed in **TABLE 1**. Similarly, if capsaicin is a component of the delivered agents then the transdermal delivery composition can comprise by weight or volume less than or equal to 0.01%, 0.015%, 0.02%, 0.025%, 0.03%, 0.035%, 0.04%, 0.045%, 0.05%, 0.055%, 0.06%, 0.065%, 0.07%, 0.075%, 0.08%, 0.085%, 0.09%, 0.095%, 0.1%, 0.15%, 0.175%, 0.2%, 0.225%, 0.25%, 0.275%, 0.3%, 0.325%, 0.35%, 0.375%, 0.4%, 0.425%, 0.45%, 0.475%, 0.5%, 0.55%, 0.6%, 0.65%, 0.7%, 0.75%, 0.8%, 0.85%, 0.9%, 0.95%, and 1.0% capsaicin or less than 1.0%, 1.5%, 2.0%, 2.5%, 3.0%, 3.5%, 4.0%, 4.5%, 5.0%, 5.5%, 6.0%, 6.5%, 7.0%, 7.5%, 8.0%, 8.5%, 9.0%, 9.5%, 10.0%, 11.0%, 12.0%, 13.0%, oleoresin capsicum. Further, if Boswellin is a component of the delivered agents, then the delivery system can comprise by weight or volume less than or equal to 0.1%, 0.15%, 0.2%, 0.25%, 0.3%, 0.35%, 0.4%, 0.45%, 0.5%, 0.55%, 0.6%, 0.65%, 0.7%, 0.75%, 0.8%, 0.85%, 0.9%, 0.95%, 1.0%, 1.1%, 1.15%, 1.2%, 1.25%, 1.3%, 1.35%, 1.4%, 1.45%, 1.5%, 1.55%, 1.6%, 1.65%, 1.7%, 1.75%, 1.8%, 1.85%, 1.9%, 1.95%, 2.0%, 2.1%, 2.15%, 2.2%, 2.25%, 2.3%, 2.35%, 2.4%, 2.45%, 2.5%, 2.55%, 2.6%, 2.65%, 2.7%, 2.75%, 2.8%, 2.85%, 2.9%, 2.95%, 3.0%, 3.1%, 3.15%, 3.2%, 3.25%, 3.3%, 3.35%, 3.4%, 3.45%, 3.5%, 3.55%, 3.6%, 3.65%, 3.7%, 3.75%, 3.8%, 3.85%, 3.9%, 3.95%, 4.0%,. 4.1%, 4.15%, 4.2%, 4.25%, 4.3%, 4.35%, 4.4%, 4.45%, 4.4%, 4.45%, 4.5%, 4.55%, 4.6%, 4.65%, 4.7%, 4.75%, 4.8%, 4.85%, 4.9%, 4.95%, 5.0%, 5.5%, 5.7%, 6.0%, 6.5%%, 6.7%, 7.0%, 7.5%, 7.7%, 8.0%, 8.5%, 8.7%, 9.0%, 9.5%, 9.7%, and 10.0% Boswellin.

Other analgesics are useful delivered agents in the transdermal delivery compositions described herein. For example, tramadol hydrochloride, fentanyl, metamizole, morphine sulphate, ketorolac tromethamine, hydrocodone, oxycodone, morphine and loxoprofen sodium are delivered agents in certain embodiments.

Steroidal anti-inflammatory compounds are also useful delivered agents in the transdermal delivery compositions described herein. For example, hydrocortisone, prednisolone, triamcinolone, and priroxicam are delivered agents in certain embodiments.

Local anesthetics are low molecular weight compounds that are useful as delivered agents in the transdermal delivery compositions described herein. The transdermal delivery compositions disclosed herein are particularly useful in the context of local anesthetics, where a local, concentrated dose of a delivered agent is desirable. Embodiments of the transdermal delivery compositions include local anesthetics, such as articaine, procaine, tetracaine, chloroprocaine and benzocaine, novocain, mepivicaine, bupivicaine, benzocaine, and lidocaine, and the like. The maximum single dose for local anesthetic solutions is somewhere between 70 mg to 500 mg, depending upon the age and health of the patient.

Compounds that have anti-infective activity are also useful in the present invention, particularly in the context of dermal bacterial, fungal, or viral infections. Antibiotics are compounds that either kill bacterial or fungal cells, or prevent them from multiplying. Several antibiotics are known to those skilled in the art and are delivered agents in certain embodiments of the transdermal delivery compositions, including but not limited to amoxicillin, clavulanate potassium, itraconazole, acyclovir, fluconazole, terbinafine hydrochloride, erythromycin ethylsuccinate, acetyl sulfisoxazole, penicillin V, cephalexin, erythromycin, azithromycin, tetracycline, ciproflaxin, gentamycin, sulfathiazole, nitrofurantoin, norfloxacin, flumequine, and ibafloxacin, metronidazole, and nystatin. Likewise, several compounds that have antiviral activity useful as delivered agents include but are not limited to acyclovir, lamivudine, indinavir sulfate, and stavudine. Those skilled in the art will appreciate that analogs and derivatives of the anti-infective compounds now known (*e.g*. valacyclovir) and discovered in the future are contemplated in the present invention.

In addition to low molecular weight delivered agents, many medium molecular weight delivered agents (*eg*., humates) can be delivered to cells in the body by using an embodiment of the transdermal delivery composition. Synthetic humates ("hepsyls") are medium molecular weight compounds (1,000 to 100,000 daltons), which are known to be strong antiviral and antimicrobial medicaments. *(See* International Application Publication No. WO 9834629 to Laub). Hepsyls are generally characterized as polymeric phenolic materials comprised of conjugated aromatic systems to which are attached hydroxyl, carboxyl, and other covalently bound functional groups. A transdermal delivery composition that can provide hepsyls to cells of the body has several pharmaceutical uses, including but not limited to, treatment of topical bacterial and viral infections.

Accordingly, in another aspect of the invention, a transdermal delivery system that can provide a medium molecular weight compound (*e.g*., a form of hepsyl) to cells of the body is provided. As described above, many different medium molecular weight compounds can be provided using an embodiment of a transdermal delivery composition described herein and the use of a medium molecular weight hepsyl as a delivered agent is intended to demonstrate that embodiments of the invention can deliver many medium molecular weight compounds to cells of the body.

In some embodiments, amino acids, peptides, nucleotides, nucleosides, and nucleic acids are transdermally delivered to cells in the body using an embodiment of the transdermal delivery composition described herein. That is, any amino acid or peptide having at least, less than, more than, or equal to 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 75, 100, 125, 150, 200, 300, 400, 500, 600, 700, 800, 900, 1000, 1500, 2000, 2500, 3000, 3500, 4000, 4500, 5000, 7000, or 10, 000 amino acids can be incorporated into a transdermal delivery composition described herein and said delivered agent can be delivered to cells in the body shortly after application of the composition. These embodiments can be used, for example, to stimulate an immune response, promote wound healing, induce collagen synthesis, or to supplement collagen. These embodiments are also useful for the delivery of peptide hormones. Non-limiting examples of peptide hormones that are delivered agents in certain embodiments include oxytocin (SEQ ID NO:2), vasopressin (SEQ ID NO:3), melanocyte-stimulating hormone (SEQ ID NO:4 (alpha) SEQ ID NO:5 (beta) SEQ ID NO:6 (gamma)), corticortropin (SEQ ID NO:7), lipotropin (SEQ ID NO:8 (beta) SEQ ID NO:9 (gamma)), thyrotropin (SEQ ID NO: 10), growth hormone (SEQ ID NO: 1), prolactin (SEQ ID NO: 11), luteinizing hormone (SEQ ID NO: 12), human chorionic gonadotropin (available from SIGMA-Aldrich, St. Louis, MO, Cat. No. C1063), follicle stimulating hormone, corticotropin-releasing factor (SEQ ID NO:13) gonadotropin-releasing factor (SEQ ID NO:43), prolactin-releasing factor (SEQ ID NO: 14), prolactin-inhibiting factor (SEQ ID NO:15), growth-hormone releasing factor (SEQ ID NO: 16), somatostatin (SEQ ID NO: 17), thyrotropin-releasing factor (SEQ ID NO:18), calcitonin (SEQ ID NO:19), calcitonin gene-related peptide (SEQ ID NO:20), parathyroid hormone (SEQ ID NO:21), glucagon-like peptide 1 (SEQ ID NO:22), glucose-dependent insulinotropic polypeptide (SEQ ID NO:23), gastrin (SEQ ID NO:24), secretin (SEQ ID NO:25), cholecystokinin (SEQ ID NO:26), motilin (SEQ ID NO:27), vasoactive intestinal peptide (SEQ ID NO:28), substance P (SEQ ID NO:30), pancreatic polypeptide (SEQ ID NO:31), peptide tyrosine tyrosine (SEQ ID NO:32), neuropeptide tyrosine (SEQ ID NO:33), amphiregulin (SEQ ID NO:34), insulin (available from SIGMA Aldrich, St. Louis, MO, Cat. No. I643), glucagon (SEQ ID NO:35), placental lactogen (SEQ ID NO:37), relaxin (SEQ ID NO:38), inhibin A (SEQ ID NO:39), Inhibin B (SEQ ID NO:40), Endorphins (e.g., SEQ ID NO:41), angiotensin II (SEQ ID NO:42), atrial natriuretic peptide (SEQ ID NO:),

Several other hormones are not peptide hormones, but are nevertheless suitable delivered agents in embodiments of the invention. Accordingly, embodiments of the invention include cortisol (available from SIGMA Aldrich, St. Louis, MO, Cat. No. H3160), corticosterone (available from SIGMA Aldrich, St. Louis, MO, Cat. No. C27840), aldosterone (available from SIGMA Aldrich, St. Louis, MO, Cat. No. 05521), epinephrine (available from SIGMA Aldrich, St. Louis, MO, Cat. No. 02252), noepinephrine (available from SIGMA Aldrich, St. Louis, MO, Cat. No. 74460), calcitriol (available from SIGMA Aldrich, St. Louis, MO, Cat. No. 17936), progesterone (available from SIGMA Aldrich, St. Louis, MO, Cat. No. P8783), testosterone (available from SIGMA Aldrich, St. Louis, MO, Cat. No. T1500), androstene (available from SIGMA Aldrich, St. Louis, MO) and melatonin (available from SIGMA Aldrich, St. Louis, MO, Cat. No. 63610).

Any nucleotide or nucleoside, modified nucleotide or nucleoside, or nucleic acid having at least 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 75, 100, 125, 150, 200, 300, 400, 500, 600, 700, 800, 900, 1000, 1500, 2000, 2500, 3000, 3500, 4000, 4500, 5000, 7000, or 10, 000 or more nucleotides can be incorporated into a transdermal delivery composition described herein and said delivered agent can be delivered to cells in the body shortly after application of the composition. These embodiments can also be used, for example, to stimulate an immune response, promote wound healing, or induce collagen synthesis.

Several nucleic acid immunogens and/or vaccines and therapies are known in the art and are useful as delivered agents in embodiments of the transdermal delivery compositions disclosed herein. Several nucleic acid immunogens that induce an immune response (both humoral and cellular) upon administration to a host have been described. DNA vaccines for several viruses, as well as for tumors, are known. Those skilled in the art will appreciate that nucleic acid immunogens contain essential regulatory elements such that upon administration to a host, the immunogen is able to direct host cellular machinery to produce translation products encoded by the respective delivered nucleic acids. Furthermore, those skilled in the art will appreciate that the specific sequences disclosed herein are non-limiting, and that while Applicants reference specific nucleic acids, allelic variants, fragments of nucleic acids, as well as orthologs and paralogs, now known or later discovered such as those made publicly available on databases such as Genbank™ are contemplated in the present invention.

Several immunogens for Human Immunodeficiency Virus (HIV), have been described. International Publication No. WO 01/46393 teaches that compositions comprising the nucleic acid encoding the HIV Nef gene, fragments thereof, or variants that are optimized for efficacy as vaccines in humans, are capable of inducing a cellular immune response in a host. The HIV Nef protein has been shown to promote viral replication. DNA sequences comprising the Nef sequence, including the sequences of SEQ ID NOs:52, 53, and 54 are known to be capable of inducing a cellular immune response in individuals. International Publication No. WO 04/050856 discloses that DNA vaccines comprising the nucleic acid sequences and variants of HIV gp120 (SEQ ID NOs:153, 154, 155, 156) and a codon-optimized nucleic acid encoding HIV-1 Gag (SEQ ID NO:152) are capable of inducing antibody and humoral immune responses. Nucleic acids encoding HIV-1 Gag and variants thereof have also been shown to induce an immune response when administered to a host (Qui et al., 2000, J. Virology. 74(13):5997-6005). Any of the above sequences from HIV are useful delivered agents for the transdermal delivery compositions disclosed herein.

Influenza A is the causative agent of the flu in humans. Flu epidemics cause morbidity and mortality worldwide, and each year in the USA alone more than 200,000 patients are admitted to hospitals because of influenza and there are approximately 36,000 influenza-related deaths. Immunogens directed against Influenza A generally comprise attenuated strains of the virus. WO 04/060720 teaches that a DNA vaccine comprising nucleic acids of sequence SEQ ID NO:51 are capable of inducing a cellular immune response against Influenza virus A.

Much work has also been done on nucleic acid-based immunogens and vaccines for the hepatitis viruses, such as hepatitis C, hepatitis B and hepatitis A. ("HCV", "HBV", and "HAV") The amino acid sequence encoded by the complete coding sequence of the prototype HCV-1 genome (HCVgp1) is provided (SEQ ID NO:128). Houghton et al. (U.S.S.N. 2002/0002272) disclose nucleic acids that encode several portions of HCVgp1 that are capable of inducing a humoral immune response. For example, nucleic acids encoding the HCV E2 envelope protein or portions thereof (SEQ ID NOs:129, 130, 131, 132), or nucleic acids encoding both HCVE1/E2 envelope proteins (SEQ ID NOs:133, 134) were capable of eliciting an immune response. Schiver et al. (International Pub. No. WO 01/43693) disclose other nucleic acid sequences from HCV that elicit protective immune responses, including the sequences of SEQ ID NO's:52, 53, 54.

Embodiments of the present invention also contemplate sequences from HBV, such as nucleic acids that encode HBV core antigen (SEQ ID NO: 135); HBVsAg (Genbank™ Accession No. AR141190), and the like. Additionally, nucleic acid sequences from the HAV genome (Genbank™ Accession No. NC_001489) are contemplated as delivered agents.

Various other nucleic acid-based immunogens and vaccines against viral pathogens have been described in the art, such as vaccines comprising nucleic acids from Hantavirus. Hantavirus is the causative agent of Hantavirus Pulmonary Syndrome (HPS), a form of adult respiratory disease syndrome that is potentially fatal in humans. WO 04/058808 discloses sequences (SEQ ID NOs:126, 127) that are useful delivered agents. Chen (International Pub. No. WO 04/110483) discloses several amino acid sequences, (SEQ ID NOs:147, 148, 149 150), the encoding nucleic acid sequences of which are useful as delivered agents for vaccines SARS.

Vaccines and immunogens comprising nucleic acids that encode a member of the Inhibitor of Apoptosis (IAP) family of proteins are also useful in the context of cancer treatment. For Example, Xiang *et al.* (International Publication No. WO 04/099389) teach DNA vaccines comprising sequences encoding members of the Inhibitor of Apoptosis (IAP) family of proteins, such as nucleic acids encoding the sequences of SEQ ID NO's:136, 137, 138, and 139. These sequences are also useful as delivered agents in one or more of the transdermal delivery systems described herein for the purposes of anti-tumor therapy.

Globulins constitute a diverse group of proteins that share the common characteristic of being soluble in water or dilute salt solutions. Due to their ability to specifically bind to target antigens, antibodies are an extremely valuable example of globulins. Offered as non-limiting examples of therapeutically valuable antibody therapies that are contemplated as delivered agents are: e.g. Rituxin™ for lymphoma; human rabies immune globulin (HRIG) for rabies; bacterial polysaccharide immune globulin (BPIG) for passive immunization of infants against bacterial infections; pertussis immune globulin, and Herceptin™ for breast cancer;. Those skilled in the art will appreciate that a wide variety of antibody therapies - polyclonal, monoclonal, including chemically or physically modified antibodies - can be used as delivered agents in the transdermal delivery compositions described herein.

Other globulins are involved in the transport of a variety of substances, including lipids, hormones, and inorganic ions. For example, sex-hormone binding globulin binds to and transports testosterone, and to a lesser degree estrogens, and thyronine-binding globulin binds to thyoxine. Globulins other than immunoglobulins are also contemplated as delivered agents in the transdermal delivery system described herein.

Immune response modifiers ("IRMs") are compounds that act on the immune system by inducing and/or suppressing cytokine biosynthesis. IRMs possess potent immunostimulating activity including, but not limited to, antiviral and antitumor activity, and can also down-regulate other aspects of the immune response, for example shifting the immune response away from a TH₂ immune response, which is useful for treating a wide range of TH₂ mediated diseases. IRMs can also be used to modulate humoral immunity by stimulating antibody production by B cells. Some IRMs are small organic compounds having a molecular weight under about 1000 daltons, preferably under about 500 daltons.

Examples of classes of small molecule IRM compounds include, but are not limited to, compounds having a 2-aminopyridine fused to a five-membered nitrogen-containing heterocyclic ring. Such compounds include, for example, imidazoquinoline amines including, but not limited to, substituted imidazoquinoline amines such as, for example, amide substituted imidazoquinoline amines, sulfonamide substituted imidazoquinoline amines, urea substituted imidazoquinoline amines, aryl ether substituted imidazoquinoline amines, heterocyclic ether substituted imidazoquinoline amines, amido ether substituted imidazoquinoline amines, sulfonamido ether substituted imidazoquinoline amines, urea substituted imidazoquinoline ethers, thioether substituted imidazoquinoline amines, and 6-, 7-, 8-, or 9-aryl or heteroaryl substituted imidazoquinoline amines; tetrahydroimidazoquinoline amines including, but not limited to, amide substituted tetrahydroimidazoquinoline amines, sulfonamide substituted tetrahydroimidazoquinoline amines, urea substituted tetrahydroimidazoquinoline amines, aryl ether substituted tetrahydroimidazoquinoline amines, heterocyclic ether substituted tetrahydroimidazoquinoline amines, amido ether substituted tetrahydroimidazoquinoline amines, sulfonamido ether substituted tetrahydroimidazoquinoline amines, urea substituted tetrahydroimidazoquinoline ethers, and thioether substituted tetrahydroimidazoquinoline amines; imidazopyridine amines including, but not limited to, amide substituted imidazopyridine amines, sulfonamide substituted imidazopyridine amines, urea substituted imidazopyridine amines, aryl ether substituted imidazopyridine amines, heterocyclic ether substituted imidazopyridine amines, amido ether substituted imidazopyridine amines, sulfonamido ether substituted imidazopyridine amines, urea substituted imidazopyridine ethers, and thioether substituted imidazopyridine amines; 1,2-bridged imidazoquinoline amines; 6,7-fused cycloalkylimidazopyridine amines; imidazonaphthyridine amines; tetrahydroimidazonaphthyridine amines; oxazoloquinoline amines; thiazoloquinoline amines; oxazolopyridine amines; thiazolopyridine amines; oxazolonaphthyridine amines; thiazolonaphthyridine amines; and 1H-imidazo dimers fused to pyridine amines, quinoline amines, tetrahydroquinoline amines, naphthyridine amines, or tetrahydronaphthyridine amines.

Additional examples of small molecule IRMs said to induce interferon (among other things), include purine derivatives (such as those described in U.S. Pat. Nos. 6,376,501, and 6,028,076), imidazoquinoline amide derivatives (such as those described in U.S. Pat. No. 6,069,149), 1H-imidazopyridine derivatives (such as those described in Japanese Patent Application No. 9-255926), benzimidazole derivatives (such as those described in U.S. Pat. No. 6,387,938), derivatives of a 4-aminopyrimidine fused to a five membered nitrogen containing heterocyclic ring (such as adenine derivatives described in U.S. Pat. Nos. 6,376,501; 6,028,076 and 6,329,381; and in International Publication No. WO 02/08595), and certain 3-.beta.-D-ribofuranosylthiazolo[4,5-d]pyri- midine derivatives (such as those described in U.S. Patent Publication No. 2003/0199461). 1H-imidazopyridine derivatives (such as those described in U.S. Pat. No. 6,518,265 and European Patent Application EP No. 1 256 582)) are said to inhibit TNF and IL-1 cytokines.

Examples of small molecule IRMs that comprise a 4-aminopyrimidine fused to a five-membered nitrogen-containing heterocyclic ring include adenine derivatives (such as those described in U.S. Pat. Nos. 6,376,501; 6,028,076 and 6,329,381; and in International Publication No. WO 02/08595).

Examples of particular IRM compounds include 2-propyl[1,3]thiazolo[4,5-c]quinolin-4-amine, which is considered predominantly a TLR 8 agonist (and not a substantial TLR 7 agonist), 4-amino-.alpha.,.alpha.-dimethyl-1H-imidazo[4,5-c]quinoline-1-ethanol, which is considered predominantly a TLR 7 agonist (and not a substantial TLR 8 agonist), and 4-amino-2-(ethoxymethyl)-alpha,alpha.-dimethyl-6,7,8,9-tetrahydro-1H-imidazo[4,5-c]quinolines-1-ethanol, which is a TLR 7 and TLR 8 agonist. In addition to its TLR 7 activity (and TLR 6 activity, but low TLR 8 activity), 4-amino-alpha,alpha-dimethyl-1H-imidazo[4,5-c]quinoline-1-ethanol has beneficial characteristics, including that it has a much lower CNS effect when delivered systemically compared to imiquimod. Other examples of specific IRM compounds include, e.g., N-[4-(4-amino-2-butyl-1H-imidazo[4,5-c][1,5]naphthyridin-1-yl)butyl]-N'-cyclohexylurea, 2-methyl-1-(2-methylpropyl)-1H-imidazo[4,5-c][1,5]naphthyridin-4-amine, 1-(2-methylpropyl)-1H-imidazo[4,5-c][1,5]naphthyridin-4-amine, N-{2-[4-amino-2-(ethoxymethyl)-1H-imidazo[4,5-c]quinolin-1-yl]-1,1-dimethylethyl}methanesulfonamide, N-[4-(4-amino-2-ethyl-1H-imidazo[4,5-c]quinolin-1-yl)butyl]methanesulfonamide, 2-methyl-1-[5-(methylsulfonyl)pentyl]-1H-imidazo[4,5-c]quinolin-4-amine, N-[4-(4-amino-2-propyl-1H-imidazo[4,5-c]quinolin-1-yl)butyl]methanesulfonamide, 2-butyl-1-[3-(methylsulfonyl)propyl-]-1H-imidazo[4,5-c]quinoline-4-amine, 2-butyl-1-{2-[(1-methylethyl)sulfonyl]ethyl}-1H-imidazo-[4,5-c]quinolin-4-amine, N-{2-[4-amino-2-(ethoxymethyl)- -1H-imidazo[4,5-c]quinolin-1-yl]-1,1-dimethylethyl}-N'-cyclohexylurea, N-{2-[4-amino-2-(ethoxymethyl)-1H-imidazo[4,5-c]quinolin-1-yl]-1,1-dimethylethyl}cyclohexanecarboxamide, N-{2-[4-amino-2-(ethoxymethyl)-1H-imidazo[-4,5-c]quinolin-1-yl]ethyl}-N'-isopropylurea. Resiquimod, 4-amino-2-ethoxymethyl-.alpha.,.alpha.-dimethyl-1H-imidazo[4,5-c]quinoline-1-ethanol, may also be used in certain situations where a combination TLR 7 and TLR 8 agonist is desired.

Other IRMs include large biological molecules such as oligonucleotide sequences. Some IRM oligonucleotide sequences contain cytosine-guanine dinucleotides (CpG) and are described, for example, in U.S. Pat. Nos. 6,194,388; 6,207,646; 6,239,116; 6,339,068; and 6,406,705. Some CpG-containing oligonucleotides can include synthetic immunomodulatory structural motifs such as those described, for example, in U.S. Pat. Nos. 6,426,334 and 6,476,000. Other IRM nucleotide sequences lack CpG and are described, for example, in International Patent Publication No. WO 00/75304. IRMs are delivered agents in embodiments of the transdermal delivery compositions of the present invention.

Embodiments of the invention are also useful for delivery of compounds used to facilitate imaging of tissues and organs within the body. Several imaging methods commonly used include Xray, CT scans, ultrasound, and magnetic resonance imaging. Various compounds are administered to individuals that facilitate the imaging process. Thus, other embodiments are useful for the delivery of diagnostic or contrast components useful in imaging methods now known or later discovered include iohexol, technetium, Tc99M, sestamibi, iomeprol, gadodiamide, oiversol, iopromide, alsactide, americium, betazole, histamine, mannitol, metyraphone, petagastrin, phentolamine, radioactive B12, gadodiamide, gadopentetic acid, gadoteridol, or perflubron as delivered agents.

In addition to low molecular weight delivered agents and medium molecular weight delivered agents, several high molecular weight delivered agents (*e.g*., glycoproteins) can be delivered to cells in the body by using an embodiment of the transdermal delivery composition. Glycoproteins are high molecular weight compounds, which are generally characterized as conjugated proteins containing one or more heterosaccharides as prosthetic groups. The heterosaccharides are usually branched but have a relatively low number of sugar residues, lack a serially repeating unit, and are covalently bound to a polypeptide chain. Several forms of glycoproteins are found in the body. For example, many membrane bound proteins are glycoproteins, the substances that fill the intercellular spaces (*e.g*., extracellular matrix proteins) are glycoproteins, and the compounds that compose collagens, proteoglycans, mucopolysaccharides, glycosaminoglycans, and ground substance are glycoproteins. A delivery system that can administer glycoproteins to cells of the body has several pharmaceutical and cosmetic uses, including but not limited to, the restoration of skin elasticity and firmness (*e.g*., the reduction in the appearance of fine lines and wrinkles by transdermal delivery of collagen) and the restoration of flexible and strong joints (*e.g*., water retention in joints can be increased by transdermal delivery of proteoglycans).

Accordingly, in another aspect of the invention, a transdermal delivery composition that can administer a high molecular weight compound (*e.g*., a form of collagen or fragment thereof) to cells of the body is provided. As described above, many different high molecular weight compounds can be administered by using an embodiment of a transdermal delivery composition of the invention and the use of a high molecular weight collagen as a delivered agent is intended to demonstrate that embodiments of the invention can deliver many high molecular weight compounds to cells of the body.

Collagens exist in many forms and can be isolated from a number of sources. Additionally, several forms of collagen can be obtained commercially (*e.g*., Brooks Industries Inc., New Jersey). Many low molecular weight collagens can be made, for example, by hydrolysis. Several transdermal delivery compositions of the invention can deliver collagens having molecular weights below 6,000 daltons. Additionally, several high molecular weight collagens exist. Some are isolated from animal or plant sources and some are synthesized or produced through techniques common in molecular biology. Several transdermal delivery compositions of the invention can deliver collagens having molecular weights of 1,000 daltons to greater than 2,000,000 daltons. That is, embodiments of the transdermal delivery compositions can deliver collagens having molecular weights of less than or equal to or greater than 1,000, 1,500, 2,000, 2,500, 3,000, 3,500, 4,000, 4,500, 5,000, 5,500, 6,000, 7,000, 8,000, 9,000, 10,000, 11,000, 12,000, 13,000, 14,000, 15,000, 16,000, 17,000, 18,000, 19,000, 20,000, 21,000, 22,000, 23,000, 24,000, 25,000, 26,000, 27,000, 28,000, 29,000, 30,000, 31,000, 32,000, 33,000, 34,000, 35,000, 36,000, 37,000, 38,000, 39,000, 40,000, 41,000, 42,000, 43,000, 44,000, 45,000, 46,000, 47,000, 48,000, 49,000, 50,000, 51,000, 52,000, 53,000, 54,000, 55,000, 56,000, 57,000, 58,000, 59,000, 60,000, 61,000, 62,000, 63,000, 64,000, 65,000, 66,000, 67,000, 68,000, 69,000, 70,000, 75,000, 80,000, 85,000, 90,000, 95,000, 100,000, 125,000, 150,000, 175,000, 200,000, 225,000, 250,000, 275,000, 300,000, 350,000, 400,000, 450,000, 500,000, 600,000, 700,000, 800,000, 900,000, 1,000,000, 1,500,000, 1,750,000, and 2,000,000 daltons.

In some embodiments, the commercially available collagen "Hydrocoll EN-55" was provided as the delivered agent and was delivered to cells of a test subject. This form of collagen is hydrolyzed collagen and has a molecular weight of 2,000 daltons. In another embodiment, the commercially available "Ichtyocollagene" or marine collagen (Sederma or Croda of Parsippany, New Jersey) was provided as the delivered agent and was delivered to a test subject. This form of soluble collagen has a molecular weight of greater than 100,000 daltons. In another embodiment, the commercially available collagen "Solu-Coll" was provided as the delivered agent and was delivered to cells of a test subject. This form of collagen is a soluble collagen having a molecular weight of 300,000 daltons. An additional embodiment includes the commercially available collagen "Plantsol", which is obtained from yeast and has a molecular weight of 500,000 daltons. This collagen was also provided as a delivered agent and was delivered to cells of a test subject.

The transdermal delivery compositions that contain a form of collagen or fragment thereof desirably comprise by weight or volume between 0.1% to 85.0% of the delivered agent depending on the type and form of the collagen, its solubility, and the intended application. That is, some transdermal delivery compositions comprise by weight or volume less than or equal to or greater than 0.1%, 0.15%, 0.2%, 0.25%, 0.3%, 0.35%, 0.4%, 0.45%, 0.5%, 0.55%, 0.6%, 0.65%, 0.7%, 0.75%, 0.8%, 0.85%, 0.9%, 0.95%, 1.0%, 1.25%, 1.5%, 1.75%, 2.0%, 2.25%, 2.5%, 2.75%, 3.0%, 3.25%, 3.5%, 3.75%, 4.0%,. 4.25%, 4.5%, 4.75%, 5.0%, 5.25%, 5.5%, 5.75%, 6.0%, 6.25%, 6.5%, 6.75%, 7.0%, 7.25%, 7.5%, 7.75%, 8.0% 8.25%, 8.5%, 8.75%, 9.0%, 9.25%, 9.5%, 9.75%, 10.0%, 10.25%, 10.5%, 10.75%, 11.0%,. 11.25%, 11.5%, 11.75%, 12.0%, 12.25%, 12.5%, 12.75%, 13.0%, 13.25%, 13.5%, 13.75%, 14.0%, 14.25%, 14.5%, 14.75%, 15.0%, 15.5%, 16.0%, 16.5%, 17.0%, 17.5%, 18.0%, 18.5%, 19.0%, 19.5%, 20.0%, 20.5%, 21.0%, 21.5%, 22.0%, 22.5%, 23.0%, 23.5%, 24.0%, 24.5%, 25.0%, 25.5%, 26.0%, 26.5%, 27.0%, 27.5%, 28.0%, 28.5%, 29.0%, 29.5%, 30.0%, 30.5%, 31.0%, 31.5%, 32.0%, 32.5%, 33.0%, 33.5%, 34.0%, 34.5%, 35.0%, 35.5%, 36.0%, 36.5%, 37.0%, 37.5%, 38.0%, 38.5%, 39.0%, 39.5%, 40.0%, 41.0%, 42.0%, 43.0%, 44.0%, 45.0%, 46.0%, 47.0%, 48.0%, 49.0%, 50.0%, 51%, 52%, 53%, 54%, 55%, 56%, 57%, 58%, 59%, 69%, 70%, 71%, 72%, 73%, 74%, 75%, 76%, 77%, 78%, 79%, 80%, 81%, 82%, 83%, 84%, or 85% collagen or fragment thereof.

For example, embodiments having Hydrocoll-EN55 can comprise by weight or volume less than or equal to or greater than 1.0%, 1.25%, 1.5%, 1.75%, 2.0%, 2.25%, 2.5%, 2.75%, 3.0%, 3.25%, 3.5%, 3.75%, 4.0%,. 4.25%, 4.5%, 4.75%, 5.0%, 5.25%, 5.5%, 5.75%, 6.0%, 6.25%, 6.5%, 6.75%, 7.0%, 7.25%, 7.5%, 7.75%, 8.0% 8.25%, 8.5%, 8.75%, 9.0%, 9.25%, 9.5%, 9.75%, 10.0%, 10.25%, 10.5%, 10.75%, 11.0%,. 11.25%, 11.5%, 11.75%, 12.0%, 12.25%, 12.5%, 12.75%, 13.0%, 13.25%, 13.5%, 13.75%, 14.0%, 14.25%,-14.5%, 14.75%, 15.0%, 15.5%, 16.0%, 16.5%, 17.0%, 17.5%, 18.0%, 18.5%, 19.0%, 19.5%, 20.0%, 20.5%, 21.0%, 21.5%, 22.0%, 22.5%, 23.0%, 23.5%, 24.0%, 24.5%, 25.0%, 25.5%, 26.0%, 26.5%, 27.0%, 27.5%, 28.0%, 28.5%, 29.0%, 29.5%, 30.0%, 30.5%, 31.0%, 31.5%, 32.0%, 32.5%, 33.0%, 33.5%, 34.0%, 34.5%, 35.0%, 35.5%, 36.0%, 36.5%, 37.0%, 37.5%, 38.0%, 38.5%, 39.0%, 39.5%, 40.0%, 41.0%, 42.0%, 43.0%, 44.0%, 45.0%, 46.0%, 47.0%, 48.0%, 49.0%, 50.0%, 51%, 52%, 53%, 54%, 55%, 56%, 57%, 58%, 59%, 69%, 70%, 71%, 72%, 73%, 74%, 75%, 76%, 77%, 78%, 79%, 80%, 81%, 82%, 83%, 84%, or 85% Hydrocoll-EN-55.

Embodiments having marine collagen can comprise by weight or volume less than or equal to or greater than 1.0%, 1.25%, 1.5%, 1.75%, 2.0%, 2.25%, 2.5%, 2.75%, 3.0%, 3.25%, 3.5%, 3.75%, 4.0%,. 4.25%, 4.5%, 4.75%, 5.0%, 5.25%, 5.5%, 5.75%, 6.0%, 6.25%, 6.5%, 6.75%, 7.0%, 7.25%, 7.5%, 7.75%, 8.0% 8.25%, 8.5%, 8.75%, 9.0%, 9.25%, 9.5%, 9.75%, 10.0%, 10.25%, 10.5%, 10.75%, 11.0%,. 11.25%, 11.5%, 11.75%, 12.0%, 12.25%, 12.5%, 12.75%, 13.0%, 13.25%, 13.5%, 13.75%, 14.0%, 14.25%, 14.5%, 14.75%, 15.0%, 15.5%, 16.0%, 16.5%, 17.0%, 17.5%, 18.0%, 18.5%, 19.0%, 19.5%, 20.0%, 20.5%, 21.0%, 21.5%, 22.0%, 22.5%, 23.0%, 23.5%, 24.0%, 24.5%, 25.0%, 25.5%, 26.0%, 26.5%, 27.0%, 27.5%, 28.0%, 28.5%, 29.0%, 29.5%, 30.0%, 30.5%, 31.0%, 31.5%, 32.0%, 32.5%, 33.0%, 33.5%, 34.0%, 34.5%, 35.0%, 35.5%, 36.0%, 36.5%, 37.0%, 37.5%, 38.0%, 38.5%, 39.0%, 39.5%, 40.0%, 41.0%, 42.0%, 43.0%, 44.0%, 45.0%, 46.0%, 47.0%, 48.0%, 49.0%, 50.0%, 51%, 52%, 53%, 54%, 55%, 56%, 57%, 58%, 59%, 69%, 70%, 71%, 72%, 73%, 74%, 75%, 76%, 77%, 78%, 79%, 80%, 81%, 82%, 83%, 84%, or 85% marine collagen.

Further, transdermal delivery compositions that contain Solu-Coll can comprise by weight or volume less than or equal to or greater than 0.1%, 0.15%, 0.2%, 0.25%, 0.3%, 0.35%, 0.4%, 0.45%, 0.5%, 0.55%, 0.6%, 0.65%, 0.7%, 0.75%, 0.8%, 0.85%, 0.9%, 0.95%, 1.0%, 1.1%, 1.15%, 1.2%, 1.25%, 1.3%, 1.35%, 1.4%, 1.45%, 1.5%, 1.55%, 1.6%, 1.65%, 1.7%, 1.75%, 1.8%, 1.85%, 1.9%, 1.95%, or 2.0% Solu-Coll.

Additionally, transdermal delivery compositions that contain Plantsol can comprise by weight or volume less than or equal to or greater than 0.1%, 0.15%, 0.2%, 0.25%, 0.3%, 0.35%, 0.4%, 0.45%, 0.5%, 0.55%, 0.6%, 0.65%, 0.7%, 0.75%, 0.8%, 0.85%, 0.9%, 0.95%, 1.0%, 1.1%, 1.15%, 1.2%, 1.25%, 1.3%, 1.35%, 1.4%, 1.45%, 1.5%, 1.55%, 1.6%, 1.65%, 1.7%, 1.75%, 1.8%, 1.85%, 1.9%, 1.95%, 2.0%, 2.1%, 2.15%, 2.2%, 2.25%, 2.3%, 2.35%, 2.4%, 2.45%, 2.5%, 2.55%, 2.6%, 2.65%, 2.7%, 2.75%, 2.8%, 2.85%, 2.9%, 2.95%, 3.0%, 3.1%, 3.15%, 3.2%, 3.25%, 3.3%, 3.35%, 3.4%, 3.45%, 3.5%, 3.55%, 3.6%, 3.65%, 3.7%, 3.75%, 3.8%, 3.85%, 3.9%, 3.95%, or 4.0% Plantsol.

In other embodiments of the invention, a transdermal delivery composition that can provide a collagen solution comprising two or more forms of collagen (e.g., Hydro-Coll EN-55, marine collagen, Solu-coll, or Plantsol) is provided. The transdermal delivery compositions that include two or more forms of collagen desirably comprise an amount of delivered agent that can be included in a delivered agent having the specific type of collagen by itself. For example, if the mixture of delivered agents comprises Hydro-Coll EN55, the amount of Hydro-Coll EN55 in the transdermal delivery composition can comprise by weight or volume less than or equal to or greater than 1.0%, 1.25%, 1.5%, 1.75%, 2.0%, 2.25%, 2.5%, 2.75%, 3.0%, 3.25%, 3.5%, 3.75%, 4.0%,. 4.25%, 4.5%, 4.75%, 5.0%, 5.25%, 5.5%, 5.75%, 6.0%, 6.25%, 6.5%, 6.75%, 7.0%, 7.25%, 7.5%, 7.75%, 8.0% 8.25%, 8.5%, 8.75%, 9.0%, 9.25%, 9.5%, 9.75%, 10.0%, 10.25%, 10.5%, 10.75%, 11.0%,. 11.25%, 11.5%, 11.75%, 12.0%, 12.25%, 12.5%, 12.75%, 13.0%, 13.25%, 13.5%, 13.75%, 14.0%, 14.25%, 14.5%, 14.75%, 15.0%, 15.5%, 16.0%, 16.5%, 17.0%, 17.5%, 18.0%, 18.5%, 19.0%, 19.5%, 20.0%, 20.5%, 21.0%, 21.5%, 22.0%, 22.5%, 23.0%, 23.5%, 24.0%, 24.5%, 25.0%, 25.5%, 26.0%, 26.5%, 27.0%, 27.5%, 28.0%, 28.5%, 29.0%, 29.5%, 30.0%, 30.5%, 31.0%, 31.5%, 32.0%, 32.5%, 33.0%, 33.5%, 34.0%, 34.5%, 35.0%, 35.5%, 36.0%, 36.5%, 37.0%, 37.5%, 38.0%, 38.5%, 39.0%, 39.5%, 40.0%, 41.0%, 42.0%, 43.0%, 44.0%, 45.0%, 46.0%, 47.0%, 48.0%, 49.0%, 50.0%, 51%, 52%, 53%, 54%, 55%, 56%, 57%, 58%, 59%, 69%, 70%, 71%, 72%, 73%, 74%, 75%, 76%, 77%, 78%, 79%, 80%, 81%, 82%, 83%, 84%, or 85% Hydrocoll-EN-55.

If the mixture of delivered agents has marine collagen, then the amount of marine collagen in the delivery system can comprise by weight or volume less than or equal to or greater than 1.0%, 1.25%, 1.5%, 1.75%, 2.0%, 2.25%, 2.5%, 2.75%, 3.0%, 3.25%, 3.5%, 3.75%, 4.0%,. 4.25%, 4.5%, 4.75%, 5.0%, 5.25%, 5.5%, 5.75%, 6.0%, 6.25%, 6.5%, 6.75%, 7.0%, 7.25%, 7.5%, 7.75%, 8.0% 8.25%, 8.5%, 8.75%, 9.0%, 9.25%, 9.5%, 9.75%, 10.0%, 10.25%, 10.5%, 10.75%, 11.0%,. 11.25%, 11.5%, 11.75%, 12.0%, 12.25%, 12.5%, 12.75%, 13.0%, 13.25%, 13.5%, 13.75%, 14.0%, 14.25%, 14.5%, 14.75%, 15.0%, 15.5%, 16.0%, 16.5%, 17.0%, 17.5%, 18.0%, 18.5%, 19.0%, 19.5%, 20.0%, 20.5%, 21.0%, 21.5%, 22.0%, 22.5%, 23.0%, 23.5%, 24.0%, 24.5%, 25.0%, 25.5%, 26.0%, 26.5%, 27.0%, 27.5%, 28.0%, 28.5%, 29.0%, 29.5%, 30.0%, 30.5%, 31.0%, 31.5%, 32.0%, 32.5%, 33.0%, 33.5%, 34.0%, 34.5%, 35.0%, 35.5%, 36.0%, 36.5%, 37.0%, 37.5%, 38.0%, 38.5%, 39.0%, 39.5%, 40.0%, 41.0%, 42.0%, 43.0%, 44.0%, 45.0%, 46.0%, 47.0%, 48.0%, 49.0%, 50.0%, 51%, 52%, 53%, 54%, 55%, 56%, 57%, 58%, 59%, 69%, 70%, 71%, 72%, 73%, 74%, 75%, 76%, 77%, 78%, 79%, 80%, 81%, 82%, 83%, 84%, or 85% marine collagen.

Similarly if the mixture of delivered agents has Solu-coll, then the amount of Solu-coll in the delivery system can comprise by weight or volume less than or equal to or greater than 0.1%, 0.15%, 0.2%, 0.25%, 0.3%, 0.35%, 0.4%, 0.45%, 0.5%, 0.55%, 0.6%, 0.65%, 0.7%, 0.75%, 0.8%, 0.85%, 0.9%, 0.95%, 1.0%, 1.1%, 1.15%, 1.2%, 1.25%, 1.3%, 1.35%, 1.4%, 1.45%, 1.5%, 1.55%, 1.6%, 1.65%, 1.7%, 1.75%, 1.8%, 1.85%, 1.9%, 1.95%, or 2.0% or Solu-Coll. Further, if the mixture of delivered agents has Plantsol, then the amount of Plantsol in the delivery system can comprise by weight or volume less than or equal to or greater than 0.1%, 0.15%, 0.2%, 0.25%, 0.3%, 0.35%, 0.4%, 0.45%, 0.5%, 0.55%, 0.6%, 0.65%, 0.7%, 0.75%, 0.8%, 0.85%, 0.9%, 0.95%, 1.0%, 1.1%, 1.15%, 1.2%, 1.25%, 1.3%, 1.35%, 1.4%, 1.45%, 1.5%, 1.55%, 1.6%, 1.65%, 1.7%, 1.75%, 1.8%, 1.85%, 1.9%, 1.95%, 2.0%, 2.1%, 2.15%, 2.2%, 2.25%, 2.3%, 2.35%, 2.4%, 2.45%, 2.5%, 2.55%, 2.6%, 2.65%, 2.7%, 2.75%, 2.8%, 2.85%, 2.9%, 2.95%, 3.0%, 3.1%, 3.15%, 3.2%, 3.25%, 3.3%, 3.35%, 3.4%, 3.45%, 3.5%, 3.55%, 3.6%, 3.65%, 3.7%, 3.75%, 3.8%, 3.85%, 3.9%, 3.95%, or 4.0% Plantsol.

Additionally, modified or stabilized collagens or collagen derivatives are contemplated for use in some of the embodiments described herein. Particularly preferred are collagens that are resistant to proteases. Recombinant engineering can be used to generate collagens or fragments thereof that lack protease cleavage sites for example. Resistant collagens or fragments thereof can also be prepared by incorporating D-amino acids in synthetically prepared collagens or fragments thereof. Cross-linked collagens can also be used. (*See e.g.,* Charulatha, Biomaterials Feb;24(5):759-67 (2003)). Still further, amidated collagen or collagen fragments can be prepared using synthetic chemistry and these collagen derivatives can be mixed with an ethoxylated oil with or without water or alcohol so as to form a transdermal delivery composition containing collagen. Several techniques to create synthetic, recombinant, or cross-linked collagens are known to those of skill in the art and many are commercially available.

Still further, protease resistant fragments of collagen can be prepared and isolated using conventional techniques. By one approach, marine collagen, procollagen, or collagen obtained from human placenta is incubated with bovine serum, pepsin, or bacterial collagenase for one hour and the preparation is then separated by gel electrophoresis, size exclusion, reverse phase, or ionic exchange chromatography (*e.g.,* FPLC or HPLC). Protease resistant fragments of collagen (*e.g.,* 15 kDa or 30kDa; *see e.g.,* Tasab et al., JBC 277(38):35007 (2002) or 38kDa *see e.g.,* Odermatt et al., Biochem J. May 1;211(2):295-302 (1983) both of which are herein expressly incorporated by reference in their entireties) are separated from the hydrolytic products and these fragments are isolated from the column and concentrated (*e.g*., centricon filters) or lyophilized using conventional techniques. The protease resistant fragments of collagen are then incorporated into a transdermal delivery composition, as described herein. Alternatively, the protease resistant domain of collagen can be prepared synthetically or obtained commercially (*e.g*., pepsinized collagens can also be obtained from Chemicon of Temecula, CA).

An additional delivered agent that can be included in a transdermal delivery composition is Etioline (Sederma or Croda of Parsippany, New Jersey). Etioline is a tyrosinase inhibitor made from the extract *Mitracarpe* and bearberry that effectively whitens the skin. Formulations of a transdermal delivery composition described herein containing Etioline (*e.g*., at 1%, 2%, 3%, 4%, 5%, 6%, 7%, 8%, 9%, 10%, 11%, 12%, 13%, 14%, 15%, 16%, 17%, 18%, 19%, or 20%) are also embodiments of the invention. Another skin brightening or whitening formulation of a transdermal delivery composition comprises Melaslow (Sederma of Parsippany, New Jersey). Melaslow is an extract made from *Citrus reticulate Blanco* var. Unshiu. Melaslow is also an inhibitor of melanogenesis and formulations of a transdermal delivery composition described herein containing Melaslow (*e.g*., at 1%, 2%, 3%, 4%, 5%, 6%, 7%, 8%, 9%, 10%, 11%, 12%, 13%, 14%, 15%, 16%, 17%, 18%, 19%, or 20%) are also embodiments of the invention. An additional delivered agent that can be included in a transdermal delivery composition is Matrixyl (Sederma or Croda of Parsippany, New Jersey). Matrixyl is a compound comprising the peptide KTTKS (SEQ. ID. NO:2), which has been shown to stimulate collagen synthesis. *See* Katayama et al., J. Biol. Chem. 268, 9941 (1993). Formulations of a transdermal delivery composition described herein containing Matrixyl or the peptide KTTKS (SEQ. ID. NO:2) (*e.g.,* at 1%, 2%, 3%, 4%, 5%, 6%, 7%, 8%, 9%, 10%, 11%, 12%, 13%, 14%, 15%, 16%, 17%, 18%, 19%, or 20%) are also embodiments of the invention. The section below describes the manufacture and use of several penetration enhancers that deliver both low and high molecular weight molecules to cells of the body.

### Penetration Enhancers

A penetration enhancer included in many embodiments of the invention is comprised of two components -- a hydrophobic component and a hydrophilic component. Desirably, the hydrophobic component comprises a polyether compound, such as an ethoxylated fatty moiety, preferably, an ethoxylated oil, such as vegetable, nut, synthetic, or animal oil, which has the ability to reduce the surface tension of materials that are dissolved into it. Not wanting to be tied to any particular mechanism or mode of action and offered only to expand the knowledge in the field, it is contemplated that the attachment of poly (ethylene oxide) to the components of a particular oil occurs not on a particular functional group but rather the polyethylene oxide chains begin to grow from unsaturated C=C bonds and from the occasional glycerol unit. Because an ethoxylated oil, such as ethoxylated macadamia nut oil, is a mixture of various fatty acids, fatty alcohols, and fatty amines, the components of the oil may have varying amounts of ethoxylation. Accordingly, measurements of ethoxylation/molecule (*e.g.,* 16 ethoxylations/molecule) are an average of the amount of ethoxylation present on the components of the oil rather than on any specific component itself.

Preferred ethoxylated oils can be obtained or created from, for example, macadamia nut oil, meadowfoam, castor oil, jojoba oil, corn oil, sunflower oil, sesame oil, and emu oil. Many of these oils are commercially available from Floratech of Gilbert, Arizona or other suppliers. Alternatively, ethoxylated oils can be prepared by reacting the oil with ethylene oxide. Pure carrier oils that are suitable for ethoxylation so as to create a penetration enhancer for use with the transdermal delivery compositions described herein are included in **TABLES 3-17** and can be obtained from Esoteric oils Pty. Ltd., Pretoria South Africa. **TABLES 3-17** also list the component fatty acids of these oils, all of which are individually suitable for ethoxylation and incorporation into an embodiment of a transdermal delivery composition. That is, it is contemplated that ethoxylated fatty acids, ethoxylated fatty alcohols, and ethoxylated fatty amines, in particular ethoxylated fatty acids, ethoxylated fatty alcohols, and ethoxylated fatty amines that contain 12, 13, 14, 15, 16, 17, 18, or 19 ethoxylations are suitable penetration enhancers for use in the transdermal delivery compositions described herein. These ethoxylated oil components can be used individually as penetration enhancers or as supplements to other penetration enhancers (*e.g*., ethoxylated macadamia nut oil).

**TABLE 3**

| MACADAMIA NUT OIL | | |
|---|---|---|
| Fatty acids | | Range |
| Myristic | C14 | 0.6 - 1.6 % |
| Palmitic | C16 | 7.0 - 11.0 % |
| Palmitoleic | C16:1 | 18.0 -25.0 % |
| Stearic | C18 | 2.0 - 4.0 % |
| Oleic | C18:1 | 55.0 - 62.0 % |
| Linoleic | C18:2 | 1.0 -4.0 % |
| Arachidic | C20 | 2.0 - 4.0 % |
| Eicosenoic | C20:1 | 2.0 - 4.0 % |

**TABLE 4**

| APRICOT KERNEL OIL | | | |
|---|---|---|---|
| Fatty acids | | Range | Typical |
| Palmitic | C16:0 | 3.0 - 6.0 % | 4.28 % |
| Palmitoleic | C16:1 | trace - 1.4 % | 0.70 % |
| Stearic | C18:0 | trace - 2.0 % | 1.12 % |
| Oleic | C18:1 | 55.0-70.0% | 69.62 % |
| Linoleic | C18:2 | 20.0-35.0 % | 23.34 % |
| Linolenic | C18:3 | trace - 1.0 % | 0.22 % |
| Eicosenoic | C20:1 | trace - 1.0 % | 0.18 % |

**TABLE 5**

| AVOCADO OIL | | | |
|---|---|---|---|
| Fatty acids | | Range | Typical |
| Palmitic | C16:0 | 12.0 - 20.0 % | 14.25 % |
| Palmitoleic | C16:1 | 2.0 - 10.0 % | 5.84 % |
| Stearic | C18:0 | 0.1 - 2.0 % | 0.1 % |
| Oleic | C18:1 | 55.0 - 75.0 % | 65.4 % |
| Linoleic | C18:2 | 9.0 - 17.0 % | 14.74 % |
| Linolenic | C18:3 | 0.1 - 2.0 % | 0.8 % |

**TABLE 6**

| EVENING PRIMROSE OIL | | | |
|---|---|---|---|
| Fatty acids | | Range | Typical |
| Palmitic | C16:0 | 5.5 - 7.0 % | 5.9 % |
| Stearic | C18:0 | 1.5 - 2.5 % | 1.7 % |
| Oleic | C18:1 | 5.0 - 11.0 % | 5.8 % |
| Linoleic | C18:2 | 70.0 - 77.0 % | 75.1 % |
| Gamma Linolenic | C18:3 | 9.0 - 10.9 % | 10.6 % |
| Alpha Linolenic | C18:3 | 1.0 % max | 0.4 % |
| Icosanoic | C20:0 | 1.0 % max | 0.2 % |
| Icosenoic | C20:1 | 1.0 % max | .01 % |

**TABLE 7**

| GRAPE SEED OIL | | | |
|---|---|---|---|
| Fatty acids | | Range | Typical |
| Palmitic | C16:0 | 6.0 -9.0 % | 6.5 % |
| Palmitoleic | C16:1 | less 1 % | 0.2 % |
| Stearic | C18:0 | 3.0 -6.0 % | 3.7 % |
| Oleic | C18:1 | 12.0 - 25.0 % | 23.4 % |
| Linoleic | C18:2 | 60.0 - 75.0 % | 65.3 % |
| Alpha Linolenic | C18:3 | less than 1.5 % | 0.2 % |
| Icosanoic | C20:0 | less than 0.5 % | 0.2 % |
| Icosenoic | C20:1 | less than 0.5 % | 0.2 % |
| Docosanoic | C22:0 | less than 0.3 % | 0.2 % |

**TABLE 8**

| HAZELNUT OIL | | |
|---|---|---|
| Fatty acids | | Range |
| Palmitic | C16:0 | 4.0- 8.0 % |
| Palmitoleic | C16:1 | 0.1 - 0.6 % |
| Stearic | C18:0 | 1.5 - 3.5 % |
| Oleic | C18:1 | 68.0 -85.0 % |
| Linoleic | C18:2 | 7.0 -15.0 % |
| Linolenic | C18:3 | 0.1 - 0.5 % |
| Arachidic | C20:0 | 0.1 - 0.5 % |
| Gadoleic | C20:1 | 0.1 - 0.3 % |
| Behenic | C22:0 | 3.0 % MAX |

**TABLE 9**

| JOJOBA OIL | | |
|---|---|---|
| Fatty acids | | Range |
| Palmitic | C16:0 | 3.0 max |
| Palmitoleic | C16:1 | 1.0 %max |
| Stearic | C18:0 | 1.0 %max |
| Oleic | C18:1 | 5.0 - 15.0 % |
| Linoleic | C18:2 | 5.0 % max |
| Linolenic | C18:3 | 1.0 % max |
| Arachidic | C20:0 | 0.5 % max |
| Eicosenoic | C20:1 | 65.0 - 80.0 % max |
| Behenic | C22:0 | 0.5 % max |
| Erucic | C22:1 | 10.0 - 20.0 % max |
| Lignoceric | C24:0 | 5.0 % max |

**TABLE 10**

| OLIVE OIL | | |
|---|---|---|
| Fatty acids | | Range |
| Palmitic | C16:0 | 5.0 - 12.0 % |
| Palmitoleic | C16:1 | 1.0 % max |
| Stearic | C18:0 | 3.5 % max |
| Oleic | C18:1 | 65.0 - 80.0 % |
| Linoleic | C18:2 | 6.0 - 25.0 % |
| Linolenic | C18:3 | 1.0 % max |
| Arachidic | C20:0 | 0.6 % max |
| Gadoleic | C20: 1 | 0.5 % max |
| Behenic | C22:0 | 0.3 % max |
| Erucic | C22:1 | 0.2 % max |

**TABLE 11**

| PUMPKIN SEED OIL | | |
|---|---|---|
| Fatty acids | | Range |
| Palmitic | C16:0 | 6.0 - 21.0 % |
| Stearic | C18:0 | 3.0 - 8.0 % |
| Oleic | C18:1 | 24.0 - 41.0 % |
| Linoleic | C18:2 | 42.0 - 60.0 % |
| Linolenic | C18:3 | 2.0 % max |
| Others | | 2.0 % max |

**TABLE 12**

| ROSE HIP OIL | | |
|---|---|---|
| Fatty acids | | Range |
| Mirystic | C14:0 | 0.0 -0.3 % |
| Palmitic | C16:0 | 3.4 - 4.4 % |
| Palmitoleic | C16:1 | 0.1 - 0.18 % |
| Stearic | C18:0 | 1.5 - 2.5 % |
| Oleic | C18:1 | 14.0 - 16.0 % |
| Linoleic | C18:2 | 43.0 - 46.0 % |
| Linolenic | C18:3 | 31.0 -34.0 % |
| Arachidic | C20:0 | 0.1 - 0.9 % |
| Gadoleic | C20:1 | 0.0 - 0.5 % |
| Eicosenoic | C20:2 | 0.0 - 0.5 % |
| Behenic | C22:0 | 0.1 -0.4 % |

**TABLE 13**

| SAFFLOWER OIL | | |
|---|---|---|
| Fatty acids | | Range |
| Palmitic | C16:0 | 4.0 - 9.0 % |
| Palmitoleic | C16:1 | Trace |
| Stearic | C18:0 | trance - 2.5 % |
| Oleic | C18:1 | 72.0 - 80.0 % |
| Linolenic | C18:2 | 12.0 - 16.0 % |
| Linolenic | C18:3 | trace - 0.5 % |

**TABLE 14**

| SESAME OIL | | |
|---|---|---|
| Fatty acids | | Range |
| Palmitic | C16:0 | 7.0 - 12.0 % |
| Palmitoleic | C16:1 | trace - 0.5 % |
| Stearic | C18:0 | 3.5 - 6.0 % |
| Oleic | C18:1 | 35.0 - 50.0 % |
| Linoleic | C18:2 | 35.0 - 50.0 % |
| Linolenic | C18:3 | track - 1.0 % |
| Eicosenoic | C20:1 | trace - 1.0 % |

**TABLE 15**

| SUNFLOWER OIL | | |
|---|---|---|
| Fatty acids | | Range |
| Palmitic | C16:0 | 5.8 % |
| Palmitoleic | C16:1 | 0.1 % |
| Stearic | C18:0 | 3.9 % |
| Oleic | C18:1 | 15.9 % |
| Linoleic | C18:2 | 71.7 % |
| Alpha Linolenic | C18:3 | 0.6 % |
| Gamma Linolenic | C18:3 | 0.1 % |
| Arachidic | C20:0 | 0.3 % |
| Gadoleic | C20:1 | 0.2 % |
| Tetracosanoic | C24:0 | 0.5 % |
| Behenic | C22:0 | 0.7 % |

**TABLE 16**

| WALNUT OIL | | | |
|---|---|---|---|
| Fatty acids | | Range | Typical |
| Palmitic | C16:0 | 5.0 - 8.0 % | 6.0 % |
| Palmitoleic | C16:1 | less than 1.0 % | 0.1 % |
| Stearic | C18:0 | 3.0 - 7.0 % | 4.0 % |
| Oleic | C18:1 | 25.0 - 35.0 % | 29.8 % |
| Linoleic | C18:2 | 45.0 - 60.0 % | 58.5 % |
| Alpha Linolenic | C18:3 | less than 0.8 % | 0.4 % |
| Arachidic | C20:0 | less than 0.5 % | 0.3 % |
| Eicosenoic | C20:1 | less than 0.5 % | 0.2 % |

**TABLE 17**

| WHEAT GERM OIL | | | |
|---|---|---|---|
| Fatty acids | | Range | Typical |
| Palmitic | C16:0 | 11.0 -16.0 % | 12.5 % |
| Palmitoleic | C16:1 | 1.0 % max | 0.2 % |
| Stearic | C18:0 | 2.0 - 6.0% | 2.5 % |
| Oleic | C18:1 | 12.0 - 39.0 % | 27.3 % |
| Linoleic | C18:2 | 30.0 -57.0 % | 53.7 % |
| Linolenic | C18:3 | 2.0 - 10.0 % | 3.0 % |
| Arachidic | C20:0 | 1.0 % max | 0.4 % |
| Gadoleic | C20:1 | 0.5 % max | 0.2 % |
| Behenic | C22:0 | 1.0 % max | 0.1 % |

In some embodiments, an ethoxylated oil comprises a molar ratio of ethylene oxide:oil of 35:1. A 99% pure ethylene oxide/castor oil having such characteristics can be obtained commercially (BASF) or such an ethoxylated compound can be synthesized using conventional techniques. In other embodiments, the ethoxylated oil is itself the penetration enhancer. That is, it has been discovered that oils that have been ethoxylated 10, 11, 12, 13, 14, 15, 16, 17, 18, or 19 ethoxylations/molecule are sufficiently hydrophobic and sufficiently hydrophilic to allow for transdermal delivery of a variety of delivered agents without water, alcohol, or an aqueous adjuvant. Although the ethoxylated oil can comprise at least 20-25 ethoxylations per molecule or more, preferably, the ethoxylated lipid comprises less than 20 ethoxylations per molecule, *e.g*., 19, 18, 17, 16, 15, 14, 13, 12, 11, or 10 ethoxylations per molecule.

By using a light, ethoxylated oil (*e.g*., macadamia nut oil containing approximately 16 ethoxylations/molecule) efficient transdermal delivery of high molecular weight collagen was observed in the absence of *Aloe Vera* and alcohol. Formulations of a transdermal delivery composition that contain *Aloe Vera* and an oil with 20-30 ethoxylations/molecule are not as effective as formulations of a transdermal delivery composition that contain an oil with 10-19 ethoxylations/molecule (*e.g.*, 16 ethoxylations/molecule) but lacking *Aloe Vera* and alcohol. A greater reduction of fine lines and wrinkles was observed with a transdermal delivery composition composed of macadamia nut oil (16 ethoxylations/molecule) and water as compared with a transdermal delivery composition composed of castor oil (25 ethoxylations/molecule), water, alcohol, and *Aloe Vera,* for example.

Unexpectedly, it was discovered that a reduction in the number of ethoxylations on a light oil produced a superior transdermal delivery product. This was unexpected because as the amount of ethoxylations on a molecule of oil decreases its miscibility with the aqueous components of the delivery system decreases. Surprisingly, formulations containing 10 - 19 ethoxylations/ molecule were not only miscible but provided very efficient transdermal delivery in the absence of *Aloe Vera.*

Desirable compounds often found in ethoxylated oils that are beneficial for some embodiments and methods described herein are glycerol-polyethylene glycol ricinoleate, the fatty esters of polyethylene glycol, polyethylene glycol, and ethoxylated glycerol. Some of these desirable compounds exhibit hydrophilic properties and the hydrophilic-lipophilic balance (HLB) is preferably maintained between 10 and 18. Any number of methods have been devised to characterize HLB, but perhaps the most widely used is the octanol/water coefficient. (*See* Calculating log Poet from Structures", by Albert J. Leo, Chemical Reviews, vol 93, pp 1281).

Accordingly, some of the components of the oils in the table above and related fatty acids, fatty alcohols, and fatty amines can be ethoxylated and used as a penetration enhancer or to enhance another penetration enhancer (*e.g*., ethoxylated macadamia nut oil). For example, some embodiments comprise a penetration enhancer that consists of, consists essentially of, or comprises ethoxylated palmitoleic acid, ethoxylated oleic acid, ethoxylated gondoic acid, or ethoxylated erucic acid. These compounds can be prepared synthetically or isolated or purified from oils that contain large quantities of these fatty acids and the synthesized, isolated, or purified fatty acids can then be reacted with ethylene oxide.

That is, a transdermal delivery composition of the invention can comprise a penetration enhancer that contains, for example, ethoxylated palmitoleic acid, ethoxylated oleic acid, ethoxylated gondoic acid, or ethoxylated erucic acid, wherein the amount of one or more of the fatty acids is at least, less than, more than, or an amount equal to 0.1%, 0.15%, 0.2%, 0.25%, 0.3%, 0.35%, 0.4%, 0.45%, 0.5%, 0.55%, 0.6%, 0.65%, 0.7%, 0.75%, 0.8%, 0.85%, 0.9%, 0.95%, 1.0%, 1.25%, 1.5%, 1.75%, 2.0%, 2.25%, 2.5%, 2.75%, 3.0%, 3.25%, 3.5%, 3.75%, 4.0%, 4.25%, 4.5%, 4.75%, 5.0%, 5.25%, 5.5%, 5.75%, 6.0%, 6.25%, 6.5%, 6.75%, 7.0%, 7.25%, 7.5%, 7.75%, 8.0% 8.25%, 8.5%, 8.75%, 9.0%, 9.25%, 9.5%, 9.75%, 10.0%, 10.25%, 10.5%, 10.75%, 11.0%, 11.25%, 11.5%, 11.75%, 12.0%, 12.25%, 12.5%, 12.75%, 13.0%, 13.25%, 13.5%, 13.75%, 14.0%, 14.25%, 14.5%, 14.75%, 15.0%, 15.5%, 16.0%, 16.5%, 17.0%, 17.5%, 18.0%, 18.5%, 19.0%, 19.5%, 20.0%, 20.5%, 21.0%, 21.5%, 22.0%, 22.5%, 23.0%, 23.5%, 24.0%, 24.5%, 25.0%, 25.5%, 26.0%, 26.5%, 27.0%, 27.5%, 28.0%, 28.5%, 29.0%, 29.5%, 30.0%, 30.5%, 31.0%, 31.5%, 32.0%, 32.5%, 33.0%, 33.5%, 34.0%, 34.5%, 35.0%, 35.5%, 36.0%, 36.5%, 37.0%, 37.5%, 38.0%, 38.5%, 39.0%, 39.5%, 40.0%, 40.25%, 40.5%, 40.75%, 41.0%, 41.25%, 41.5%, 41.75%, 42.0%, 42.25%, 42.5%, 42.75%, 43.0%, 43.25%, 43.5%, 43.75%, 44.0%, 44.25%, 44.5%, 44.75%, 45.0%, 45.25%, 45.5%, 45.75%, 46.0%, 46.25%, 46.5%, 46.75%, 47.0% 47.25%, 47.5%, 47.75%, 48.0%, 48.25%, 48.5%, 48.75%, 49.0%, 49.25%, 49.5%, 49.75%, 50.0%, 50.25%, 50.5%, 50.75%, 51.0%, 51.25%, 51.5%, 51.75%, 52.0%, 52.25%, 52.5%, 52.75%, 53.0%, 53.25%, 53.5%, 53.75%, 54.0%, 54.5%, 54.0%, 54.5%, 55.0%, 55.5%, 56.0%, 56.5%, 57.0%, 57.5%, 58.0%, 58.5%, 59.0%, 59.5%, 60.0%, 60.5%, 61.0%, 61.5%, 62.0%, 62.5%, 63.0%, 63.5%, 64.0%, 64.5%, 65.0%, 65.5%, 66.0%, 66.5%, 67.0%, 67.5%, 68.0%, 68.5%, 69.0%, 69.5%, 70.0%, 70.5%, 71.0%, 71.5%, 72.0%, 72.5%, 73.0%, 73.5%, 74.0%, 74.5%, 75.0%, 75.5%, 76.0%, 76.5%, 77.0%, 77.5%, 78.0%, 78.5%, 79.0%, 79.5%, 80.0%, 80.5%, 81%, 81.5%, 82%, 82.5%, 83%, 83.5%, 84%, 84.5%, 85%. 85.5%, 86%, 86.5%, 87%, 87.5%, 88%, 88.5%, 89%, 89.5%, 90%, 90.5%, 91%, 91.5%, 92%, 92.5%, 93%, 93.5%, 94%, 94.5%, 95%, 95.5%, 96%, 96.5%, 97%, 97.5%, 98%, 98.5%, 99%, or 100% of the total fatty acid content in the composition. In some embodiments, more than one ethoxylated compound is added or another hydrophobic compound is added (*e.g*., Y-Ling-Y-Lang oil; Young Living Essential Oils, Lehl, Utah)) to balance or enhance the penetration enhancer. Preferred embodiments include ethoxylated macadamia nut oil that has been supplemented with ethoxylated palmitoleic acid, ethoxylated oleic acid, ethoxylated gondoic acid, or ethoxylated erucic acid.

Depending on the type of delivered agent and the intended application, the amount of ethoxylated lipid(s) in the delivery system can vary. For example, delivery systems of the invention can comprise between 0.1% and 99% by weight or volume ethoxylated compound(s). That is, embodiments of the invention can comprise by weight or volume at least, less than, or equal to or greater than 0.1%, 0.15%, 0.2%, 0.25%, 0.3%, 0.35%, 0.4%, 0.45%, 0.5%, 0.55%, 0.6%, 0.65%, 0.7%, 0.75%, 0.8%, 0.85%, 0.9%, 0.95%, 1.0%, 1.25%, 1.5%, 1.75%, 2.0%, 2.25%, 2.5%, 2.75%, 3.0%, 3.25%, 3.5%, 3.75%, 4.0%, 4.25%, 4.5%, 4.75%, 5.0%, 5.25%, 5.5%, 5.75%, 6.0%, 6.25%, 6.5%, 6.75%, 7.0%, 7.25%, 7.5%, 7.75%, 8.0% 8.25%, 8.5%, 8.75%, 9.0%, 9.25%, 9.5%, 9.75%, 10.0%, 10.25%, 10.5%, 10.75%, 11.0%, 11.25%, 11.5%, 11.75%, 12.0%, 12.25%, 12.5%, 12.75%, 13.0%, 13.25%, 13.5%, 13.75%, 14.0%, 14.25%, 14.5%, 14.75%, 15.0%, 15.5%, 16.0%, 16.5%, 17.0%, 17.5%, 18.0%, 18.5%, 19.0%, 19.5%, 20.0%, 20.5%, 21.0%, 21.5%, 22.0%, 22.5%, 23.0%, 23.5%, 24.0%, 24.5%, 25.0%, 25.5%, 26.0%, 26.5%, 27.0%, 27.5%, 28.0%, 28.5%, 29.0%, 29.5%, 30.0%, 30.5%, 31.0%, 31.5%, 32.0%, 32.5%, 33.0%, 33.5%, 34.0%, 34.5%, 35.0%, 35.5%, 36.0%, 36.5%, 37.0%, 37.5%, 38.0%, 38.5%, 39.0%, 39.5%, 40.0%, 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% ethoxylated lipid(s), preferably an ethoxylated oil or fatty acid or combination of fatty acids.

The hydrophilic component of the penetration enhancer can comprise an alcohol, a non-ionic solubilizer, or an emulsifier. Compounds such as ethylene glycol, propylene glycol, dimethyl sulfoxide (DMSO), dimethyl polysiloxane (DMPX), oleic acid, caprylic acid, isopropyl alcohol, 1-octanol, ethanol (denatured or anhydrous), and other pharmaceutical grade or absolute alcohols with the exception of methanol can be used. Preferred embodiments comprise an alcohol (*e.g.*, absolute isopropyl alcohol), which is commercially available. As above, the amount of hydrophilic component in the penetration enhancer depends on the type of the delivered agent and the intended application. The hydrophilic component of a penetration enhancer of the invention can comprise between 0.1% and 50% by weight or volume. That is, a delivery system of the invention can comprise by weight or volume at least, less than or equal to or greater than 0.1%, 0.15%, 0.2%, 0.25%, 0.3%, 0.35%, 0.4%, 0.45%, 0.5%, 0.55%, 0.6%, 0.65%, 0.7%, 0.75%, 0.8%, 0.85%, 0.9%, 0.95%, 1.0%, 1.25%, 1.5%, 1.75%, 2.0%, 2.25%, 2.5%, 2.75%, 3.0%, 3.25%, 3.5%, 3.75%, 4.0%,. 4.25%, 4.5%, 4.75%, 5.0%, 5.25%, 5.5%, 5.75%, 6.0%, 6.25%, 6.5%, 6.75%, 7.0%, 7.25%, 7.5%, 7.75%, 8.0% 8.25%, 8.5%, 8.75%, 9.0%, 9.25%, 9.5%, 9.75%, 10.0%, 10.25%, 10.5%, 10.75%, 11.0%, 11.25%, 11.5%, 11.75%, 12.0%, 12.25%, 12.5%, 12.75%, 13.0%, 13.25%, 13.5%, 13.75%, 14.0%, 14.25%, 14.5%, 14.75%, 15.0%, 15.5%, 16.0%, 16.5%, 17.0%, 17.5%, 18.0%, 18.5%, 19.0%, 19.5%, 20.0%, 20.5%, 21.0%, 21.5%, 22.0%, 22.5%, 23.0%, 23.5%, 24.0%, 24.5%, 25.0%, 25.5%, 26.0%, 26.5%, 27.0%, 27.5%, 28.0%, 28.5%, 29.0%, 29.5%, 30.0%, 30.5%, 31.0%, 31.5%, 32.0%, 32.5%, 33.0%, 33.5%, 34.0%, 34.5%, 35.0%, 35.5%, 36.0%, 36.5%, 37.0%, 37.5%, 38.0%, 38.5%, 39.0%, 39.5%, 40.0%, 41.0%, 42.0%, 43.0%, 44.0%, 45.0%, 46.0%, 47.0%, 48.0%, 49.0%, or 50.0% hydrophilic component.

In addition to a delivered agent and penetration enhancer, the transdermal delivery compositions described herein can comprise an aqueous adjuvant. The section below describes the incorporation of aqueous adjuvants in formulations of transdermal delivery compositions, in particular, *Aloe Vera,* which can enhance the delivery of both low and high molecular weight molecules to the skin cells of the body.

### Aqueous Adjuvants

Several embodiments of the transdermal delivery composition described herein comprise an aqueous adjuvant such as *Aloe Vera* juice or water or both. The term *"Aloe"* refers to the genus of South African plants of the *Liliaceae* family, of which the *Aloe barbadensis* plant is a species. *Aloe* is an intricate plant, which contains many biologically active substances. (Cohen, et al. in Wound Healing/Biochemical and Clinical Aspects, 1st ed. WB Saunders, Philadelphia (1992)). Over 300 species of *Aloe* are known, most of which are indigenous to Africa. Studies have shown that the biologically active substances are located in three separate sections of the *Aloe* leaf--a clear gel fillet located in the center of the leaf, in the leaf rind or cortex of the leaf and in a yellow fluid contained in the pericyclic cells of the vascular bundles, located between the leaf rind and the internal gel fillet, referred to as the latex. Historically, *Aloe* products have been used in dermatological applications for the treatment of burns, sores and other wounds. These uses have stimulated a great deal of research in identifying compounds from *Aloe* plants that have clinical activity, especially anti-inflammatory activity. (*See e.g.*, Grindlay and Reynolds (1986) J. of Ethnopharmacology 16:117-151; Hart, et al. (1988) J. of Ethnopharmacology 23:61-71). As a result of these studies there have been numerous reports of *Aloe* compounds having diverse biological activities, including anti-tumor activity, anti-gastric ulcer, anti-diabetic, anti-tyrosinase activity, (*See e.g.*, Yagi, et al. (1977) Z. Naturforsch. 32c:731-734), and antioxidant activity (International Application Serial No. PCT/US95/07404).

Recent research has also shown that *Aloe Vera,* a term used to describe the extract obtained from processing the entire leaf, isolated from the *Aloe Vera* species of *Aloe,* can be used as a vehicle for delivering hydrocortisone, estradiol, and testosterone propionate. (*See* Davis, et al, JAPMA 81:1 (1991) and U.S. Pat. No. 5,708,038 to Davis)). As set forth in Davis (U.S. Pat. No. 5,708,308), one embodiment of *"Aloe Vera"* can be prepared by "whole-leaf processing" of the whole leaf of the *Aloe barbadensis* plant. Briefly, whole leaves obtained from the *Aloe barbadensis* plant are ground, filtered, treated with cellulase (optional) and activated carbon and lyophilized. The lyophilized powder is then reconstituted with water prior to use.

*Aloe Vera* can be obtained commercially through Aloe Laboratories, for example. In other embodiments, the *Aloe Vera* is made as follows. First, the leaves are manually harvested. Next, the leaves are washed with water and the thorns on both ends are cut. The leaves are then hand-filleted so as to extract the inner part of the leaf. The inner gel is passed through a grinder and separator to remove fiber from the gel. Then the gel is put into a pasteurizing tank where L-Ascorbic Acid (Vitamin C) and preservatives are added. The gel is pasteurized at 85°C for 30 minutes. After pasteurization, the gel is put into a holding tank for about one or two days, after which the gel is sent through a ½ micron filter. Finally, the gel is cooled down through a heat exchanger and stored in a steamed, sanitized and clean 55 gallon drum. The above described sources and manufacturing methods of *Aloe Vera* are given as examples and not intended to limit the scope of the invention. One of ordinary skill in the art will recognize that *Aloe Vera* is a well known term of art, and that *Aloe Vera* is available from various sources and manufactured according to various methods.

Absolute *Aloe Vera* (100% pure) can also be obtained from commercial suppliers (Lily of the Desert, Irving, Texas). *Aloe Vera* juice, prepared from gel fillet, has an approximate molecular weight of 200,000 to 1,400,000 daltons. Whole leaf *Aloe Vera* gel has a molecular weight of 200,000 to 3,000,000 depending on the purity of the preparation. Although, preferably, the embodiments of the invention having *Aloe Vera* comprise *Aloe Vera* juice, other extracts from a member of the *Liliaceae* family can be used (*e.g.*, an extract from another *Aloe* species).

Transdermal delivery compositions having *Aloe Vera* can comprise between 0.1% to 85.0% by weight or volume *Aloe Vera.* That is, embodiments of the invention can comprise by weight or volume at least, less than or equal to or greater than 0.1%, 0.15%, 0.2%, 0.25%, 0.3%, 0.35%, 0.4%, 0.45%, 0.5%, 0.55%, 0.6%, 0.65%, 0.7%, 0.75%, 0.8%, 0.85%, 0.9%, 0.95%, 1.0%, 1.25%, 1.5%, 1.75%, 2.0%, 2.25%, 2.5%, 2.75%, 3.0%, 3.25%, 3.5%, 3.75%, 4.0%, 4.25%, 4.5%, 4.75%, 5.0%, 5.25%, 5.5%, 5.75%, 6.0%, 6.25%, 6.5%, 6.75%, 7.0%, 7.25%, 7.5%, 7.75%, 8.0% 8.25%, 8.5%, 8.75%, 9.0%, 9.25%, 9.5%, 9.75%, 10.0%, 10.25%, 10.5%, 10.75%, 11.0%, 11.25%, 11.5%, 11.75%, 12.0%, 12.25%, 12.5%, 12.75%, 13.0%, 13.25%, 13.5%, 13.75%, 14.0%, 14.25%, 14.5%, 14.75%, 15.0%, 15.5%, 16.0%, 16.5%, 17.0%, 17.5%, 18.0%, 18.5%, 19.0%, 19.5%, 20.0%, 20.5%, 21.0%, 21.5%, 22.0%, 22.5%, 23.0%, 23.5%, 24.0%, 24.5%, 25.0%, 25.5%, 26.0%, 26.5%, 27.0%, 27.5%, 28.0%, 28.5%, 29.0%, 29.5%, 30.0%, 30.5%, 31.0%, 31.5%, 32.0%, 32.5%, 33.0%, 33.5%, 34.0%, 34.5%, 35.0%, 35.5%, 36.0%, 36.5%, 37.0%, 37.5%, 38.0%, 38.5%, 39.0%, 39.5%, 40.0%, 40.25%, 40.5%, 40.75%, 41.0%, 41.25%, 41.5%, 41.75%, 42.0%, 42.25%, 42.5%, 42.75%, 43.0%, 43.25%, 43.5%, 43.75%, 44.0%, 44.25%, 44.5%, 44.75%, 45.0%, 45.25%, 45.5%, 45.75%, 46.0%, 46.25%, 46.5%, 46.75%, 47.0% 47.25%, 47.5%, 47.75%, 48.0%, 48.25%, 48.5%, 48.75%, 49.0%, 49.25%, 49.5%, 49.75%, 50.0%, 50.25%, 50.5%, 50.75%, 51.0%, 51.25%, 51.5%, 51.75%, 52.0%, 52.25%, 52.5%, 52.75%, 53.0%, 53.25%, 53.5%, 53.75%, 54.0%, 54.5%, 54.0%, 54.5%, 55.0%, 55.5%, 56.0%, 56.5%, 57.0%, 57.5%, 58.0%, 58.5%, 59.0%, 59.5%, 60.0%, 60.5%, 61.0%, 61.5%, 62.0%, 62.5%, 63.0%, 63.5%, 64.0%, 64.5%, 65.0%, 65.5%, 66.0%, 66.5%, 67.0%, 67.5%, 68.0%, 68.5%, 69.0%, 69.5%, 70.0%, 70.5%, 71.0%, 71.5%, 72.0%, 72.5%, 73.0%, 73.5%, 74.0%, 74.5%, 75.0%, 75.5%, 76.0%, 76.5%, 77.0%, 77.5%, 78.0%, 78.5%, 79.0%, 79.5%, 80.0%, 80.5%, 81%, 81.5%, 82%, 82.5%, 83%, 83.5%, 84%, 84.5%, and 85% *Aloe Vera.*

The amount of water in the delivery system generally depends on the amount of other reagents (*e.g*., delivered agent, penetration enhancer, and other aqueous adjuvants or fillers). Although water is used as the sole aqueous adjuvant in some embodiments, preferred embodiments use enough water to make the total volume of a particular preparation of a delivery system such that the desired concentrations of reagents in the penetration enhancer, aqueous adjuvant, and delivered agent are achieved. Suitable forms of water are deionized, distilled, filtered or otherwise purified. Clearly, however, any form of water can be used as an aqueous adjuvant.

Transdermal delivery compositions having water can comprise between 0.1% to 85.0% by weight or volume water. That is, embodiments of the invention can comprise by weight or volume at least, less than or equal to or greater than 0.1%, 0.15%, 0.2%, 0.25%, 0.3%, 0.35%, 0.4%, 0.45%, 0.5%, 0.55%, 0.6%, 0.65%, 0.7%, 0.75%, 0.8%, 0.85%, 0.9%, 0.95%, 1.0%, 1.25%, 1.5%, 1.75%, 2.0%, 2.25%, 2.5%, 2.75%, 3.0%, 3.25%, 3.5%, 3.75%, 4.0%, 4.25%, 4.5%, 4.75%, 5.0%, 5.25%, 5.5%, 5.75%, 6.0%, 6.25%, 6.5%, 6.75%, 7.0%, 7.25%, 7.5%, 7.75%, 8.0% 8.25%, 8.5%, 8.75%, 9.0%, 9.25%, 9.5%, 9.75%, 10.0%, 10.25%, 10.5%, 10.75%, 11.0%, 11.25%, 11.5%, 11.75%, 12.0%, 12.25%, 12.5%, 12.75%, 13.0%, 13.25%, 13.5%, 13.75%, 14.0%, 14.25%, 14.5%, 14.75%, 15.0%, 15.5%, 16.0%, 16.5%, 17.0%, 17.5%, 18.0%, 18.5%, 19.0%, 19.5%, 20.0%, 20.5%, 21.0%, 21.5%, 22.0%, 22.5%, 23.0%, 23.5%, 24.0%, 24.5%, 25.0%, 25.5%, 26.0%, 26.5%, 27.0%, 27.5%, 28.0%, 28.5%, 29.0%, 29.5%, 30.0%, 30.5%, 31.0%, 31.5%, 32.0%, 32.5%, 33.0%, 33.5%, 34.0%, 34.5%, 35.0%, 35.5%, 36.0%, 36.5%, 37.0%, 37.5%, 38.0%, 38.5%, 39.0%, 39.5%, 40.0%, 40.25%, 40.5%, 40.75%, 41.0%, 41.25%, 41.5%, 41.75%, 42.0%, 42.25%, 42.5%, 42.75%, 43.0%, 43.25%, 43.5%, 43.75%, 44.0%, 44.25%, 44.5%, 44.75%, 45.0%, 45.25%, 45.5%, 45.75%, 46.0%, 46.25%, 46.5%, 46.75%, 47.0% 47.25%, 47.5%, 47.75%, 48.0%, 48.25%, 48.5%, 48.75%, 49.0%, 49.25%, 49.5%, 49.75%, 50.0%, 50.25%, 50.5%, 50.75%, 51.0%, 51.25%, 51.5%, 51.75%, 52.0%, 52.25%, 52.5%, 52.75%, 53.0%, 53.25%, 53.5%, 53.75%, 54.0%, 54.5%, 54.0%, 54.5%, 55.0%, 55.5%, 56.0%, 56.5%, 57.0%, 57.5%, 58.0%, 58.5%, 59.0%, 59.5%, 60.0%, 60.5%, 61.0%, 61.5%, 62.0%, 62.5%, 63.0%, 63.5%, 64.0%, 64.5%, 65.0%, 65.5%, 66.0%, 66.5%, 67.0%, 67.5%, 68.0%, 68.5%, 69.0%, 69.5%, 70.0%, 70.5%, 71.0%, 71.5%, 72.0%, 72.5%, 73.0%, 73.5%, 74.0%, 74.5%, 75.0%, 75.5%, 76.0%, 76.5%, 77.0%, 77.5%, 78.0%, 78.5%, 79.0%, 79.5%, 80.0%, 80.5%, 81%, 81.5%, 82%, 82.5%, 83%, 83.5%, 84%, 84.5%, and 85% water. In addition to the aforementioned compositions, methods of making and using the transdermal delivery compositions are described in the following section.

### Preparing Transdermal delivery compositions

In general, transdermal delivery compositions are prepared by combining an ethoxylated fatty moiety or a penetration enhancer with a delivered agent and, optionally, an aqueous adjuvant. Depending on the solubility of the delivered agent, the delivered agent can be solubilized in either the hydrophobic or hydrophilic components of the penetration enhancer. In some formulations, (*e.g.*, formulations containing oil soluble delivered agents such as steroid hormones), the delivered agent readily dissolves in the ethoxylated oil without water, alcohol, or an aqueous adjuvant. In other formulations, the delivered agent (*e.g*., an NSAID or collagen or fragments thereof) readily dissolves in water, which is then mixed with the ethoxylated oil. Additionally, some delivered agents can be solubilized in the aqueous adjuvant prior to mixing with the penetration enhancer. Desirably, the pH of the mixture is maintained between 3 and 11 and preferably between 5 and 9. That is, during preparation and after preparation the pH of the solution is desirably maintained at less than, more than, at least, or equal to 3.0, 3.25, 3.5, 3.75, 4.0, 4.25, 4.5, 4.75, 5.0, 5.25, 5.5, 5.75, 6, 6.25, 6.5, 6.75, 7.0, 7.25, 7.5, 7.75, 8.0, 8.25, 8.5, 8.75, 9.0, 9.25, 9.5, 9.75, 10.0, 10.25, 10.5, 10.75, or 11.0.

Several physical mixing techniques can be employed to help the delivery system coalesce. For example, a magnetic stir plate and bar can be used, however, the speed of stirring is preferably minimized so as not to drive air into the mixture and/or destroy the delivered agent (*e.g*., when the delivered agent is a peptide or a protein). Additionally, a rocker can be used to bring components of the delivery system together. Heat can also be applied to help coalesce the mixture but desirably, the temperature is not raised above 40°C so that labile aqueous adjuvants or labile delivered agents are not degraded. Preferably, once the delivery system has coalesced, other components such as fragrances and colors are added or the delivery system is incorporated into a cream or ointment or a device for applying the delivery system.

Several formulations of delivery system are within the scope of aspects of the invention. In embodiments that include an aqueous adjuvant, the ratio of hydrophilic component:hydrophobic component:aqueous adjuvant is desirably 3:4:3, but preferred formulations comprise 1:1:4, 1:1:14, and 1:10:25. As described above, a sufficient amount of delivered agent to suit the intended purpose is incorporated into the delivery system. The amount of delivered agent that is incorporated into the penetration enhancer depends on the compound, desired dosage, and application.

In some embodiments, the transdermal delivery composition is made by providing an ethoxylated oil, mixing the ethoxylated oil with an alcohol, non-ionic solubilizer, or emulsifier so as to form a penetration enhancer, mixing the penetration enhancer with an aqueous adjuvant (*e.g.*, an extract from a plant of the *Liliaeacae* family), and mixing the penetration enhancer and aqueous adjuvant with a delivered agent and thereby making the transdermal delivery composition. For example, an embodiment of a transdermal delivery composition comprising a pain relief solution is manufactured as follows. A solution of 2.0% to 7.0% oleoresin capsicum, 2.5 grams of Boswellin is mixed with 400ml of absolute carpilic alcohol or isopropyl alcohol, 300ml of ethoxylated castor oil, and 300ml of a 100% solution of *Aloe Vera*. This transdermal delivery composition has been observed to alleviate pain when rubbed on a targeted area.

The transdermal delivery compositions having a form of Hepsyl as a delivered agent desirably are comprised by weight or volume of between 0.005% to 12.0% Hepsyl, depending on the type of Hepsyl, its solubility, and the intended application. For example, embodiments having Hepsyl CA 1501C. Hepsyl CGA 1501K., and Hepsyl RA 150K can be comprised by weight or volume of 0.01-2 grams of Hepsyl delivered agent, 0-50 mL of hydrophobic penetration enhancers (*e.g*., ethoxylated castor oil, jojoba oil, etc.), 0-50 mL of hydrophilic penetration enhancers, nonionic solubilizers, or emulsifiers (*e.g*., isopropyl. alcohol, DMSO, etc.), and 0-50 mL of aqueous adjuvant (*e.g.*, water, *Aloe Vera* extract, etc.). A particularly desirable embodiment of the invention is comprised of 0.1-0.5 gram of Hepsyl, 5-10 mL of ethoxylated castor oil, 5-10 mL of isopropyl alcohol, and 5-10 mL of *Aloe Vera* extract. By using these formulations, other delivered agents can be incorporated into a transdermal delivery composition. Formulations of transdermal delivery compositions having collagens are described in the examples. The following section describes several therapeutic, prophylactic and cosmetic applications.

### Therapeutic, Prophylactic, and Cosmetic Applications

Many embodiments are suitable for treatment of subjects either as a preventive measure (*e.g*., to avoid pain or skin disorders) or as a therapeutic to treat subjects already afflicted with skin disorders or who are suffering pain. In general, most drugs, chemicals, and cosmetic agents that can be incorporated into a pharmaceutical or cosmetic can be formulated into a transdermal delivery composition of the invention. Because the various formulations of transdermal delivery composition described herein have a considerable range in hydrophobic and hydrophilic character, most drugs, chemicals, and cosmetic preparations can be incorporated therein. That is, by adjusting the amount of ethoxylation, alcohol, and water in a particular formulation most pharmaceutical and cosmetic agents are solubilized in a transdermal delivery composition with little effort. Furthermore, because the transdermal delivery compositions described herein can deliver a wide range of materials of both high and low molecular weight to skin cells, the utility of the transdermal delivery compositions described herein is incredibly broad. The aspects of the invention that follow are for exemplary purposes only, and one of skill in the art can readily appreciate the wide spread applicability of a transdermal delivery composition described herein and the incorporation of other delivered agents into a formulation of transdermal delivery composition is straight forward.

In one embodiment, for example, a method of treatment or prevention of inflammation, pain, or human diseases, such as cancer, arthritis, and Alzheimer's disease, comprises using a transdermal delivery composition described herein that has been formulated with an NSAID. Because delivered agents such as NSAIDs, capsaicin, and Boswellin interfere and/or inhibit cyclooxygenase enzymes (COX-1 and COX-2), they provide a therapeutically beneficial treatment for cancer and Alzheimer's disease when administered by a transdermal delivery composition described herein. (*See* U.S. Pat. No. 5,840,746 to Ducharme et al., and U.S. Pat. No. 5,861,268 to Tang et al.).

By one approach, a transdermal delivery composition comprising a delivered agent that is effective at reducing pain or inflammation (*e.g*., NSAIDS, capsaicin, Boswellin, or any combination thereof) is administered to a subject in need and the reduction in pain or inflammation is monitored. An additional approach involves identifying a subject in need of a COX enzyme inhibitor (*e.g*., a subject suffering from cancer or Alzheimer's disease) and administering a transdermal delivery composition comprising a delivered agent that inhibits a COX enzyme (*e.g*., NSAIDS, capsaicin, Boswellin, or any combination thereof). Although many individuals can be at risk for contracting cancer or Alzheimer's disease, those with a family history or a genetic marker associated with these maladies are preferably identified. Several diagnostic approaches to identify persons at risk of developing these diseases have been reported. (See *e.g.*, U.S. Pat. Nos., 5,891,857; 5,744,368; 5,891,651; 5,837,853; and 5,571,671). The transdermal delivery composition is preferably applied to the skin at a region of inflammation or an area associated with pain or the particular condition and treatment is continued for a sufficient time to reduce inflammation, pain, or inhibit the progress of the disease. Typically, pain and inflammation will be reduced in 5-20 minutes after application. Cancer and Alzheimer's disease can be inhibited or prevented with prolonged use.

In another method, an approach to reduce wrinkles and increase skin tightness and flexibility (collectively referred to as "restoring skin tone") is provided. Accordingly, a transdermal delivery composition comprising a form of collagen or fragment thereof as a delivered agent is provided and contacted with the skin of a subject in need of treatment. By one approach, a subject in need of skin tone restoration is identified, a transdermal delivery composition comprising collagen or a fragment thereof is administered to the subject, and the restoration of the skin tone is monitored. Identification of a person in need of skin restoration can be based solely on visible inspection and the desire to have tight, smooth, and flexible skin. Treatment with the delivery system is continued until a desired skin tone is achieved. Typically a change in skin tone will be visibly apparent in 15 days but prolonged use may be required to retain skin tightness and flexibility. The form of collagen in the delivered agent can be from various sources and can have many different molecular weights, as detailed above. Preferably, high molecular weight natural collagens are used, however, recombinant collagens, modified collagens, protease resistant collagens, and fragments thereof may be used with some of the transdermal delivery compositions described herein.

The transdermal delivery compositions described herein can be processed in accordance with conventional pharmacological, veterinary and cosmetological methods to produce medicinal, veterinary, and cosmetic agents for administration to animals and humans in need thereof (*e.g*., mammals including humans, dogs, cats, horses, cattle, and other companion or farm animals). The transdermal delivery compositions described herein can be incorporated into a pharmaceutical or cosmetic product with or without modification. The compositions of the invention can be employed in admixture with conventional excipients, *e.g.*, pharmaceutically acceptable organic or inorganic carrier substances suitable for topical application that do not deleteriously react with the molecules that assemble the delivery system. The preparations can be sterilized and if desired mixed with auxiliary agents, *e.g.*, lubricants, preservatives, stabilizers, coloring, aromatic substances and the like that do not deleteriously react with the active compounds. They can also be combined where desired with other active agents. Embodiments described herein can be made according to good manufacturing processes (e.g., certified GMP), can be approved by a governmental body, such as the Food and Drug Administration, and may have indicia that indicates that said compositions were manufactured GMP or were approved by a governmental body, with or without structure-function indicia (e.g., indicia that indicates the product's usefulness for improvement of one's appearance or the general health and welfare of individuals that use the product).

The effective dose and method of administration of a transdermal delivery system formulation can vary based on the individual patient and the stage of the disease, as well as other factors known to those of skill in the art. Although several doses of delivered agents have been indicated above, the therapeutic efficacy and toxicity of such compounds in a delivery system of the invention can be determined by standard pharmaceutical or cosmetological procedures with experimental animals, *e.g.*, ED50 (the dose therapeutically effective in 50% of the population) and LD50 (the dose lethal to 50% of the population). The dose ratio of toxic to therapeutic effects is the therapeutic index, and it can be expressed as the ratio, LD50/ED50. Pharmaceutical and cosmetological compositions that exhibit large therapeutic indices are preferred. The data obtained from animal studies is used in formulating a range of dosages for human use. The dosage of such compounds lies preferably within a range of circulating concentrations that include the ED50 with little or no toxicity. The dosage varies within this range depending upon the dosage form employed, sensitivity of the patient, and the route of administration.

The exact dosage is chosen by the individual physician in view of the patient to be treated. Dosage and administration are adjusted to provide sufficient levels of the active moiety or to maintain the desired effect. Additional factors that may be taken into account include the severity of the disease state, age, weight and gender of the patient; diet, time and frequency of administration, drug combination(s), reaction sensitivities, and tolerance/response to therapy. Short acting compositions are administered daily whereas long acting pharmaceutical compositions are administered every 2, 3 to 4 days, every week, or once every two weeks. Depending on half-life and clearance rate of the particular formulation, the pharmaceutical compositions of the invention are administered once, twice, three, four, five, six, seven, eight, nine, ten or more times per day.

Routes of administration of the delivery systems of the invention are primarily topical, although it is desired to administer some embodiments to cells that reside in deep skin layers. Topical administration is accomplished via a topically applied cream, gel, rinse, etc. containing a delivery system of the invention. Compositions of delivery system-containing compounds suitable for topical application include, but are not limited to, physiologically acceptable ointments, creams, rinses, and gels.

In some embodiments, the transdermal delivery composition is incorporated into a device that facilitates application. The embodied compositions generally have a vessel joined to an applicator, wherein a transdermal delivery composition of the invention is incorporated in the vessel. Some devices, for example, facilitate delivery by encouraging vaporization of the mixture. These apparatus have a transdermal delivery composition of the invention incorporated in a vessel that is joined to an applicator such as a sprayer (*e.g.*, a pump-driven sprayer). These embodiments can also comprise a propellant for driving the incorporated transdermal delivery composition out of the vessel. Other apparatus can be designed to allow for a more focused application. A device that facilitates a focused application of a transdermal delivery composition of the invention can have a roll-on or swab-like applicator joined to the vessel that houses the transdermal delivery composition. Several devices that facilitate the administration of a delivery system of the invention have a wide range of cosmetic or therapeutic applications. An exemplary transdermal delivery device is described in the section that follows.

### Transdermal Delivery Dispenser

In some embodiments, the transdermal delivery composition is provided in a single dose application containing a pre-measured amount of a delivered agent. For example, septum sealed vials with or without an applicator (*e.g.*, a swab) containing a pre-measured amount of transdermal delivery composition (*e.g.*, 0.5ml) containing a pre-measured amount of a delivered agent (*e.g.*, 400mg of ibuprofen, 0.6mg marine collagen, or 1g of aspirin) are embodiments of the invention. These embodiments have significant utility because pre-determined doses of certain delivered agents facilitate appropriate treatment regimens and the individually sealed doses of the transdermal delivery composition with delivered agent maintain sterility of the composition between applications.

Figures 1A and 1B show an exemplary embodiment of a dispenser 100. As can be seen in Figure 1A, in which the dispenser 100 is shown in an exploded state, the dispenser 100 comprises a removable cartridge 102 and a body portion 104. A latch member 106 on the body portion 104, is shown in an unsecured state, permitting the insertion and removal of the removable cartridge 102. The latch member 106 is slidable between the unsecured position as shown and a secured position 108, shown in shadow, in which the insertion and/or removal of a removable cartridge 102 is inhibited. Although a slidable latch member 106 is shown in this embodiment, it will be understood that any method of securing the removable cartridge 102 to the body portion 104 can be used. For example, a pin attached to the body portion 104 could engage an aperture on the removable cartridge 102. Alternately, if the body portion is formed from a sufficiently resilient material, the body portion can be designed such that a snug fit is formed without any need for additional securing methods. Transparent portion 103 permits the user to view the amount of fluid remaining in removable cartridge 102. Similarly, transparent portion 105 allows the user to see the amount of fluid to be dosed.

Figure 1B shows the dispenser 100 in an assembled state, where the removable cartridge 102 has been inserted into the body portion 104. The latch member 106 has been moved to the secured position 108 shown in shadow in Figure 1A.

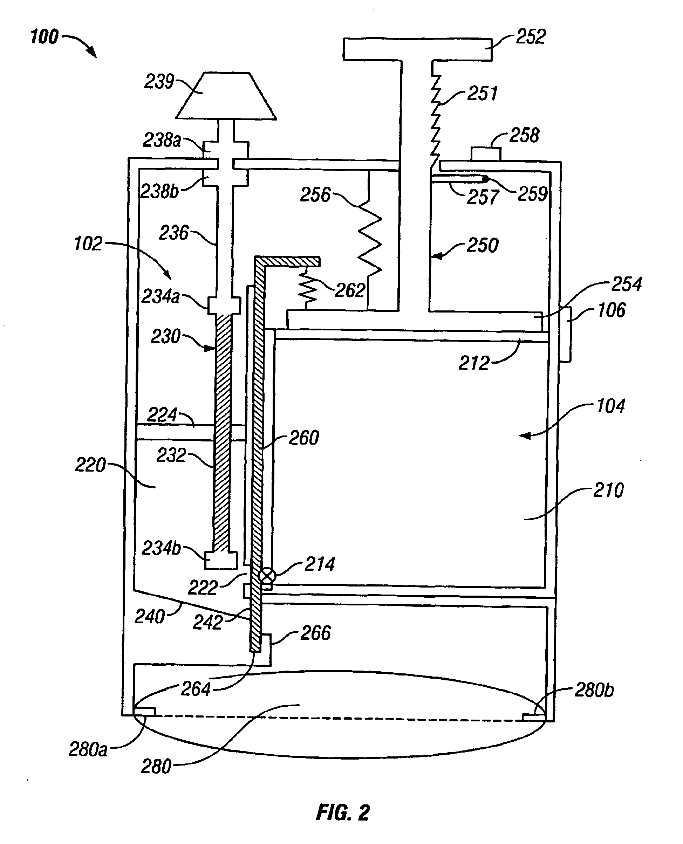

Figure 2 schematically depicts a cross-section of the dispenser 100 of Figure 1, in an assembled state. It can be seen that the removable cartridge 104 comprises a fluid reservoir 210, which is configured to hold the therapeutic drug delivery fluid. The removable cartridge 104 further comprises a movable upper wall 212, which forms the upper wall of the fluid reservoir 210. The movable upper wall is displaceable in at least the downward direction. The removable cartridge 102 also includes a one-way valve 214, such as a check valve, located at the bottom of the removable cartridge, which is in fluid communication with the fluid reservoir 210. As will be described in greater detail below, displacement of the movable upper wall 212 in the downward direction will cause fluid to flow from the fluid reservoir 210 through the valve 214.

Still with respect to Figure 2, the body portion 102 of the dispenser 100 includes a dosing chamber 220. When the dispenser 100 is in an assembled space, the dosing chamber 220 is in fluid communication with the valve 214 of the removable cartridge via an aperture 222 in the dosing chamber aligned with the valve 214. The upper wall of the dosing chamber 220 is formed by the lower surface of a movable member 224, alternately referred to as a dosing member. In this embodiment, movable member 224 comprises a threaded aperture through which a threaded portion 232 portion of shaft 230 extends. Stop members 234a and 234b are located at the upper and lower ends, respectively, of the shaft 230. A non-threaded portion 236 of shaft 230 extends through an aperture in the top of the body portion 104, and wide sections 238a and 238b of shaft 230 constrain vertical translation of the shaft 230 with respect to the body portion 102. A knob 239 at the top of the shaft 220 facilitates rotation by a user.

The size of the dosing chamber 220 can be adjusted by rotating the knob 239, causing rotation of the shaft 230. As the dosing chamber 220 and the movable member 224 have a non-circular shape, the movable member 224 cannot rotate along with the shaft 230. The rotational movement of the shaft therefore results in vertical translation of the movable member 224, changing the volume of the dosing chamber 220. When the movable member reaches one of stop members 234a,b, the rotational movement of the shaft 230 will be inhibited. The movable member 224 may comprise a ring of partially deformable material (not shown), such as a rubberized material, around the edges of the movable member which come in contact with the walls of the dosing chamber, in order to facilitate a tight seal between the edges of the movable member and the walls of the dosing chamber, so as to prevent undesired leakage along the sides of the movable member.

The lower end of the dosing chamber 220 comprises a sloped surface 240, and an aperture 242 in the wall of the dosing chamber. This aperture 242 preferably extends to the bottom of the dosing chamber at least one point along the bottom surface of the dosing chamber, such that all fluid in the dosing chamber 220 can flow out of the aperture 242.

The body portion 104 further comprises a plunger 250 having an upper end 252 and a lower end 254 configured to engage the movable upper wall of the removable cartridge 102. The plunger 250 extends through an aperture in the top of the body portion 104. The plunger 250 is preferably biased to return to a position in which the upper surface of the removable cartridge is not engaged. This may be done, for example, via a spring 256 connecting the body portion 104 and the plunger 250. As will be discussed in greater detail below, it may be desirable to permit the user to control the timing of the return to the initial position. This may be accomplished via the inclusion of teeth 251 along the shaft of the plunger, and a locking member 257 which is attached to the interior of the body portion 102 and biased to remain in a position against the plunger 250, as shown. The teeth 251 therefore permit the downward movement of the plunger, but inhibit the return of the plunger upward to its original position. Locking member 257 is operably connected to a release button 258 on the exterior of the dispenser 100. Engaging the release button rotates locking member 257 about pivot point 259, and permits the return of the plunger to its original position. When the release button is disengaged, the bias of locking member 257 returns it to the position shown in Figure 2.

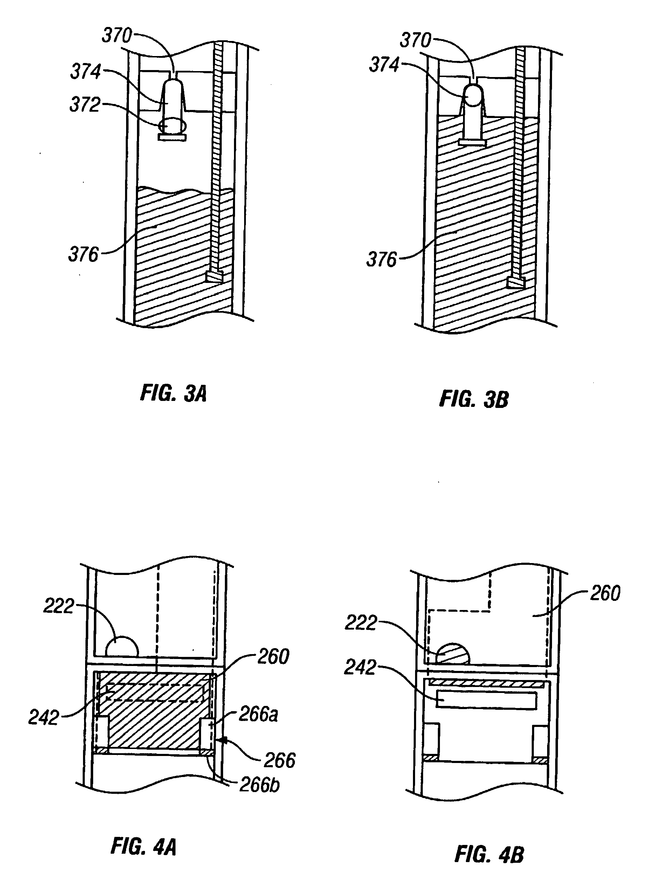

The body portion 104 further comprises a slidable member 260 which is movable between a first position in which the slidable member 260 inhibits fluid flow out of the dosing chamber 220 through the aperture 242, and a second position in which the slidable member 250 inhibits fluid flow from the removable cartridge 102 to the dosing chamber 220 via aperture 222. The slidable member 260 is in the first position when the plunger is in a depressed position, and the second position when the plunger retracts to an undepresed position. This may be accomplished, for example, via spring 262, which connects the plunger 250 to the slidable member 262. When the plunger is depressed, the spring 262 holds the slidable member 260 within a slot 264, located below the sloped surface 240 which forms the bottom of the chamber. As the pressure increases, the sliding member is prevented from flexing away from the dosing chamber by tabs 266. Thus, when the sliding member 260 is in the first position, shown in Figure 2, the dosing chamber can be filled and fluid will not leak out. Fluid is permitted to flow into the dosing chamber due to the shape of sliding member 260, discussed in greater detail with respect to Figure 4. When the plunger is moved to an undepressed position, the slidable member will be pushed upward to the second position, where the flow of fluid through the aperture 242 is permitted.

At the bottom of the dispenser 100 is an applicator. In the present embodiment, the applicator consists of an ellipsoidal applicator 280 mounted on pins 280a and 280b which extend at least partially into the applicator 280 along the axis of the applicator. Applicator 280 thus provides a roll-on applicator, such that once the therapeutic fluid is released from the dosing chamber after the release button 258 is pressed, the fluid will flow downwards onto applicator 280. The applicator can then be placed in contact with the skin of the user, and the dispenser moved to cause the applicator to roll across the skin of the patient, applying the desired dose of the therapeutic fluid to the patient. It will be understood that alternate non-invasive applicators can be used in place of the roll-on applicator. These alternate non-invasive applicators may include, but are not limited to, an absorbent applicator tip, such as a sponge, or an applicator surface having perforations through which the therapeutic fluid can flow.

In the above embodiment, the dosing chamber will be partially filled with fluid when the plunger is depressed, but the air in the dosing chamber will not be permitted to escape, and will therefore be compressed in the dosing chamber. In order to protect the dispenser 100 from damage due to excessive pressure created in the dosing chamber, the valve 214 may be designed to close when a certain pressure has been reached. Taking into account this pressure, and the volume of the trapped air at that pressure, accurate dosing can be obtained by accounting for the volume of the trapped air in the dosing chamber.

In an alternative embodiment, the movable member 224 may comprise a mechanism for allowing air to exit the dosing chamber without permitting fluid. An exemplary system for doing so is shown in Figures 3A and 3B. In Figure 3A, it can be seen that the movable member 324 includes an aperture 370. A sphere 372, which is buoyant relative to the fluid 376which will be used, is suspended within a track 374, which permits movement of the sphere 372 upward to engage the aperture 370, forming a seal, but inhibits movement of the sphere 372 downward below a level necessary to allows air to flow over the sphere 372 and out through the aperture 370.

Figure 3B shows the dosing chamber full of fluid 376. The buoyant sphere 372 is lifted as the fluid level rises within the dosing chamber. Because the sphere 372 is kept level with the fluid, almost all of the air is allowed to escape, but the fluid cannot escape through the aperture once the sphere engages the aperture. The sphere 372 may advantageously be formed of a partially deformable material, to facilitate the forming of a seal between the sphere and the movable member 324. Bouancy of the sphere 372 may be achieved through selection of an appropriate material, or through the use of a hollow sphere, in order to increase buoyancy.

It will be understood that alternate methods of permitting air to escape while preventing fluid flow through the movable member 220 may be utilized, including the use of specialized valves which permit the flow of air while inhibiting the flow of fluid through the valve.

The operation of the sliding member 370 is now described with respect to Figures 4A and 4B. Figure 4A depicts a portion of the cross section of the dispenser 100 of Figure 2, taken along line 4 of Figure 2. In particular, it can be seen that Figure 4A depicts an embodiment in which the slidable member 260, shown partially in shadow where it is locate behind other features, is in a first position in which the slidable member does not inhibit the flow of fluid from the fluid reservoir 210 through the aperture 222 into the dosing chamber 220 (for simplicity, the valve 214 is not depicted, but would be in line with aperture 222). This is due to the design of the slidable member 260 such that it is substantially L-shaped. It can be seen that when the slidable member 260 is in this first position, flow of the fluid out of the dosing chamber 230 through the aperture 242 is prevented by the lower portion of slidable member 260. Slidable member 260 is prevented from flexing away from aperture 242 by the upper portions 266a of tab members 266. In addition, the lower portions 266b of tab members 266 prevent additional downward movement of slidable member 260. Thus, tab members 266 form a slot 264 (see Figure 2) which constrains the slidable member such that it forms a sufficiently rigid barrier to constrain fluid flow out of the dosing chamber.

In Figure 4B, the slidable member 260 has been moved to a second position in which the slidable member 260 obstructs the flow of fluid through aperture 222 into dosing chamber 220, but permits the flow of fluid from dosing chamber 220 through aperture 242, and downward to applicator 280 (not shown). Because the slidable member 260 is operably connected via spring 262 to plunger 250, the slidable member 260 is in the first position when the plunger is depressed and fluid is being dispensed into the dosing chamber, and in the second position when the plunger returns to its original position after the release button is depressed. As can be seen, the lower, thicker portion of the slidable member 260 desirably has sufficient height that at intermediate positions of the slidable member, both of the apertures 222, 242 are completely occluded. Thus, no additional fluid beyond what is already in the dosing chamber will be dispensed.

For example, in another embodiment, the dispenser may not include a fluid reservoir contained within a removable cartridge, but may instead be a disposable dispenser without a replaceable cartridge. In another embodiment, the volume of the dosing chamber need not be adjustable by the user. Such an embodiment may be advantageous in situations where precise dosing is required, or where regular fixed doses are required.

In another embodiments, the user actuatable knob which controls the size of the dosing chamber need not be fixed directly to the rotatable shaft, but may instead be operably connected to the rotatable shaft via a gear or a series of gears, so as to facilitate either rapid adjustment of the dosing volume or very precise adjustment of the dosing volume, depending on the relative properties of the gears. In alternate embodiments, one or more of the operably connected features need not be mechanically connected, as described and depicted above. For instance, electrical connections between features and electrical actuators, such as servo motors, stepper motors, or hydraulics, can be used to replace the mechanical interconnections described above. For example, the knob 239 could be replaced by two buttons, electrically connected to a motor, one of which causes the motor to drive the rotatable shaft in one direction, and the other of which causes the motor to drive the rotatable shaft in the other direction. Similarly, the plunger could be replaced by a plunger which is electronically actuatable at the push of a button. A pressure sensor within the dosing chamber could be used to release the plunger once a sufficient pressure has been reached.

Example 1 below describes a clinical study that was performed to evaluate the efficacy of a transdermal delivery composition that comprised capsaicin.

### EXAMPLE 1

In this example, evidence is provided that a transdermal delivery composition of the invention can administer a therapeutically effective amount of a low molecular weight delivered agent (*e.g*., 0.225% oleoresin capsicum). A clinical study was performed to evaluate the effectiveness of a transdermal delivery composition of the invention comprising 0.225% capsaicin ("EPRS") as compared to a commercially available cream comprising Boswellin, 10% methyl salicylate, and 0.25% capsaicin. (Nature's Herbs). The two pain relief preparations were tested on six subjects who suffer from degenerative arthritis, debilitating back pain, and/ or bursitis. For the first five days of the study, the subjects applied the commercially available cream three times a day. On day six, application of the commercially available cream was stopped and subjects applied the EPRS transdermal delivery composition. The EPRS pain relief solution was also applied for five days, three times a day. Daily analysis of the efficacy of the particular pain relief formulations was taken by the subjects and observations such as the time of administration, odor, and therapeutic benefit were recorded after each administration.

The five day use of the commercially available cream was found to provide only minimal therapeutic benefit. The cream was reported to irritate the skin, have a noxious smell, and provide little decrease in pain or increase in flexibility or range of motion. In contrast, the five day use of EPRS was reported to provide significant pain relief, relative to the relief obtained from the oral consumption of NSAIDs. Further, EPRS was reported to increase flexibility and range of motion within five to twenty minutes after application. Additionally, EPRS did not present a significant odor nor did it cause skin irritation. The results of this study demonstrate that a delivery system comprising a low molecular weight compound, capsaicin, can effectively administer the delivered agent to cells of the body where it provides therapeutic benefit. The next example describes a clinical study that was performed to evaluate the efficacy of several different formulations of transdermal delivery composition that comprised low and high molecular weight collagens.

### EXAMPLE 2

In this example, evidence is provided that a transdermal delivery composition of the invention can administer a therapeutically effective amount of a low and high molecular weight delivered agent (*e.g*., a low and high molecular weight collagens). A clinical study was performed to evaluate the effectiveness of several transdermal delivery compositions comprising various penetration enhancers, aqueous adjuvants, and collagen delivered agents. The various transdermal delivery compositions that were evaluated are provided in **TABLE 18**. Of the formulations that were originally screened, three were extensively evaluated by ten subjects (three men and seven women) in a single blind study. The formulations analyzed in the single blind study are indicated in **TABLE 18** by a dagger. That is, the three different formulations ("P1", "P2", and "F4") were evaluated.

The P1 formulation comprised approximately 0.73% to 1.46% Solu-Coll, a soluble collagen having a molecular weight of 300,000 daltons. The P2 formulation comprised approximately 1.43% to 2.86% Plantsol, a plant collagen obtained from yeast having a molecular weight of 500,000 daltons. The F4 formulation comprised approximately 11.0% of HydroColl EN-55, a hydrolyzed collagen having a molecular weight of 2,000 daltons. The evaluation of the P1, P2, and F4 formulations was as follows. Left, right, and center mug-shot photographs were taken with a Pentax camera having a zoom 60X lens and Kodak-Gold 100 film before beginning the study. Shortly after, each subject was given a bottle having a formulation of transdermal delivery composition and was instructed to apply the solution to the right side of the face and neck, leaving the left side untreated, twice daily for 15 days. The F4 formulation was tested first and the application was carried out after showering or washing and before application of any other product to the treated area of the face. After the 15 day period, three mug-shot photographs were again taken, the subjects recorded their observations on the effectiveness of the formulation in a questionnaire, and a 7 day period without application of a collagen product provided. The questionnaire requested the subject to assign a score (*e.g.*, a numerical value that represents effectiveness) on characteristics of the transdermal delivery composition formulation. Characteristics that were evaluated included tackiness, odor, marketability, and overall effectiveness of the formulation, as well as, whether the formulation tightened the skin, decreased lines, conditioned or softened the skin, and had any negative side-effects. The scale for the scoring was 1-10, with 1 being the worst rating and 10 being the best rating.

Following the test of F4, the evaluation detailed above was conducted on the P1 formulation. Again, photographs were taken before and after the second 15 day protocol, a questionnaire evaluating the efficacy of the particular formulation was completed, and a 7 day period without application of a collagen product was provided. Further, after the test of P1, the same evaluation was conducted on the P2 formulation, photographs were taken before and after the trial, and a questionnaire evaluating the efficacy of the particular formulation was completed.

The data from the three evaluation questionnaires were pooled, analyzed using a "t-table" and standard deviation calculations were made. See **TABLE 19**. An overall rating for each particular formulation was assigned. A perfect score by this system was a 7.875 overall rating. P1 was found to have a 4.25 overall rating (approximately 54% effective), P2 was found to have a 4.625 overall rating (approximately 59% effective), and F4 was found to have a 5.625 overall rating (approximately 71% effective).

The before and after treatment photographs also revealed that the three tested transdermal delivery compositions provided therapeutic benefit. A decrease in wrinkles was observed and an increase in skin tightness and firmness can be seen. That is, P1, P2, and F4 all provided therapeutic and/or cosmetic benefit in that they restored skin tone in the subjects tested. The results presented above also demonstrate that transdermal delivery compositions of the invention can be used to administer high molecular weight delivered agents.

**TABLE 18**

| ECO | *Aloe* | IPA | Plantsol | EN-55 | Solu-coll | DMPX | YYO | Score | ID |
|---|---|---|---|---|---|---|---|---|---|
| | | | | | | | | | |
| 29.7% * | 50.0%* | 5.0%* | 0* | 8.3%* | 0* | 0* | 0* | 2 | F-1 |
| 10.4% | 79.0% | 5.3% | 0 | 8.7% | 0 | 0 | 0 | 3 | F-2 |
| 5.2% | 63.0% | 5.3% | 0 | 17.4% | 0 | 0 | 0 | 3 | F-3 |
| 5.0% | 70.0% | 5.0% | 0 | 11.0% | 0 | 0 | 0 | 3+ | F-4 t |
| 4.5% | 18.2% | 4.6% | 0 | 0 | 0.7% to 1.5% | 0 | 0 | 3+ | P-1 † |
| 8.3% | 8.3% | 8.3% | 0.7% to 1.4% | 4.6% | 0.3% to 0.7% | 0 | 0 | 2 | Y-500 |
| 0.7% | 22.2% | 11.1% | 1.3% to 2.7% | 0 | 0 | 0 | 0 | 3+ | P-501 |
| 0.4% | 35.7% | 3.6% | 1.1% to 2.1% | 0 | 0 | 0 | 0 | 2 | P-502 |
| 0.9% | 8.7% | 0 | 0 | 0 | 2.3% to 4.6% | 0 | 0 | 1 | SC-1 |
| 1.8% | 18.5% | 0 | 0 | 44.8% | 0 | 0 | 0 | 3+ | SC-2 |
| 1.8% | 17.9% | 7.1% | 0 | 43.2% | 0 | 0 | 0 | 3 | SC-3 |
| 0.9% | 9.4% | 4.7% | 0 | 34.3% | 0.3% to 0.6% | 0 | 0 | 1 | PSC EN |
| 1.8% | 31.3% | 6.3% | 1.3% to 2.5% | 0 | 0 | 0 | 0 | 3+ | P-1A |
| 0.8% | 19.2% | 3.8% | 1.5% to 3.1% | 0 | 0 | 7.7% | 0.3% | 5 | P-1C |
| 0.7% | 17.9% | 7.1% | 1.4% to 2.9% | 0 | 0 | 1.1% | 0.3% | 5 | P-2 † |
| 0.7% | 22.2% | 11.1% | 1.3% to 2.7% | 0 | 0 | 0 | 0 | 3+ | P-501 |

| | | | | | | | | | |
|---|---|---|---|---|---|---|---|---|---|
| *Abbreviations:* ECO - ethoxylated castor oil (BASF) *Aloe - Aloe Vera* (*Aloe* Labs; (800)-258-5380) IPA - Absolute isopropyl alcohol (Orange County Chemical, Santa Ana, California) Plantsol - Yeast extract collagen (Brooks Industries Inc., Code No. 06485) EN-55 - hydrolyzed bovine collagen (Brooks Industries Inc., Code No. 01000) SoluColl - soluble collagen (Brooks Industries Inc., Code No. 01029) DMPX - dimethyl polysiloxane (5 centistokes) (Sigma) YYO - Y-ling-Y-lang oil (Young Living Essential Oils, Lehl, Utah) ID - Identification number * The percentages reflect volume to volume. † Sample used in the 45 day clinical trial. | | | | | | | | | |

**TABLE 19**

| COLLAGEN T-TABLE | | | | |
|---|---|---|---|---|
| Formulations | P1 | P2 | F4 | standard deviation |
| Tackiness | 5 | 3 | 10 | 2.94 |
| Skin tightness | 7 | 5 | 8 | 1.25 |
| Odor | 2 | 8 | 8 | 2.83 |
| Decrease lines | 2 | 2 | 1 | 0.47 |
| Soften skin | 8 | 7 | 4 | 1.7 |
| Total skin restoration | 5 | 5 | 6 | 0.47 |
| Market Buying Power | 5 | 7 | 8 | 1.25 |
| Side effects | 0 | 0 | 0 | 0 |
| Total Score (Average) | 4.25 | 4.63 | 5.63 | 1.36 |

Several *in vitro* techniques are now widely used to assess the percutaneous absorption of delivered agents. (*See e.g.*, Bronaugh and Collier in In vitro Percutaneous absorption studies: Principle, Fundamentals, and Applications, eds. Bronaugh and Maibach, Boca Raton, F1, CRC Press, pp237-241 (1991) and Nelson et al., J. Invest. Dermatol. 874-879 (1991)). Absorption rates, and skin metabolism can be studied in viable skin without the interference from systemic metabolic processes. The next example describes several approaches that can be used to evaluate the ability of a particular formulation of transdermal delivery composition to deliver a particular delivered agent.

### EXAMPLE 3

Skin barrier function can be analyzed by examining the diffusion of fluorescent and colored proteins and dextrans of various molecular weights ("markers") across the skin of nude mice or swine. Swine skin is preferred for many studies because it is inexpensive, can be maintained at -20°C, and responds similarly to human skin. Prior to use, frozen swine skin is thawed, hair is removed, and subcutaneous adipose tissue is dissected away. Preferably, a thickness of skin that resembles the thickness of human skin is obtained so as to prepare a membrane that accurately reflects the thickness of the barrier layer. A dermatome can be pushed across the surface of the skin so as to remove any residual dermis and prepare a skin preparation that accurately reflects human skin. Elevation of temperature can also be used to loosen the bond between the dermis and the epidermis of hairless skin. Accordingly, the excised skin is placed on a hot plate or in heated water for 2 minutes at a temperature of approximately 50°C - 60°C and the dermis is removed by blunt dissection. Chemical approaches (*e.g.*, 2M salt solutions) have also been used to separate the dermis from the epidermis of young rodents.

Many different buffers or receptor fluids can be used to study the transdermal delivery of delivered agents across excised skin prepared as described above. Preferably, the buffer is isotonic, for example a normal saline solution or an isotonic buffered solution. More physiological buffers, which contain reagents that can be metabolized by the skin, can also be used. (See *e.g*., Collier et al., Toxicol. Appl. Pharmacol. 99:522-533 (1989)).

Several different markers with molecular weight from 1,000 daltons to 2,000,000 daltons are commercially available and can be used to analyze the transdermal delivery compositions of the invention. For example, different colored protein markers having a wide range of molecular weights (6,500 to 205,000 daltons) and FITC conjugated protein markers (*e.g*., FITC conjugated markers from 6,500 to 205,000 daltons) are available from Sigma (C3437, M0163, G7279, A2065, A2190, C1311, T9416, L8151, and A2315). Further, high molecular weight FITC conjugated dextrans (*e.g*., 250,000, 500,000, and 2,000,000 daltons) are obtainable from Sigma. (FD250S, FD500S, and FD2000S).

Accordingly, in one approach, swine skin preparations, obtained as described above, are treated with a delivery system lacking a delivered agent and control swine skin preparations are treated with water. Subsequently, the skin is contacted with a 1mM solution of a marker with a known molecular weight suspended in Ringer's solution (pH 7.4) at 37°C. After one hour, the skin is frozen and sliced at a thickness of 5µm. The sections are counter stained with 5µg/ml propidium and, if the marker is FITC conjugated, the sections are analyzed by fluoresence microscopy. If the marker is a colored marker, diffusion of the marker can be determined by light microscope. The marker will be retained in the upper layers of the stratum corneum in the skin(delete "untreated mice") but the skin treated with the delivery system will be found to have the dye distributed throughout the stratum corneum and any dermal layer that remains.

Additionally, modifications of the experiments described above can be performed by using a delivery system comprising various molecular weight markers. Accordingly, skin preparations are treated with the delivery system comprising one or more markers and control skin preparations are treated with water. After one hour, the skin is frozen and sliced at a thickness of 5µm. The sections can be counter stained with 5µg/ml propidium iodide and can be analyzed by fluoresence microscopy (*e.g.*, when a fluorescent marker is used) or alternatively, the sections are analyzed under a light microscope. The marker will be retained in the upper layers of the stratum corneum in the skin(delete "untreated mice") but the skin treated with the delivery system will be found to have the dye distributed throughout the stratum corneum and any dermal layer that remains.

In another method, the transdermal water loss (TEWL) of penetration enhancer-treated skin preparations can be compared to that of untreated skin preparations. Accordingly, skin preparations are obtained, as described above, and are treated with a delivery system of the invention lacking a delivered agent (*e.g*., a penetration enhancer). Control skin preparations are untreated. To assess TEWL, an evaporimeter is used to analyze the skin preparation. The Courage and Khazaka Tewameter TM210, an open chamber system with two humidity and temperature sensors, can be used to measure the water evaporation gradient at the surface of the skin. The parameters for calibrating the instrument and use of the instrument is described in Barel and Clarys Skin Pharmacol. 8: 186-195 (1995) and the manufacturer's instructions. In the controls, TEWL will be low. In contrast, TEWL in penetration enhancer-treated skin preparations will be significantly greater.

Further, skin barrier function can be analyzed by examining the percutaneous absorption of labeled markers (*e.g*., radiolabeled, fluorescently labeled, or colored) across skin preparations in a diffusion chamber. Delivery systems of the invention having various molecular weight markers, for example, the proteins and dextrans described above, are administered to swine skin preparations. Swine skin preparations are mounted in side-by-side diffusion chambers and are allowed to stabilize at 37°C with various formulations of penetration enhancer. Donor and receiver fluid volumes are 1.5ml. After 1 hour of incubation, a labeled marker is added to the epidermal donor fluid to yield a final concentration that reflects an amount that would be applied to the skin in an embodiment of the invention. Five hundred microliters of receiver fluid is removed at various time points, an equal volume of penetration enhancer is added to the system. The aliquot of receiver fluid removed is then analyzed for the presence of the labeled marker (*e.g*., fluorescent detection, spectroscopy, or scintillation counting). Control swine skin preparations are equilibrated in Ringer's solution (pH 7.4) at 37°C; the same concentration of labeled marker as used in the experimental group is applied to the donor fluid after one hour of equilibration; and 500µl of receiver fluid is analyzed for the presence of the marker. In the experimental group, the steady-state flux of labeled marker in the skin will be significantly greater than that of the control group. By using these approaches, several transdermal delivery compositions can be evaluated for their ability to transport low and high molecular weight delivered agents across the skin. The next example describes several different formulations of transdermal delivery composition that were made to comprise various delivered agents, demonstrating the wide-range of utility of aspects of the invention.

### EXAMPLE 4

In this example, several different formulations of transdermal delivery composition containing various delivered agents are provided. The formulations described include: compositions for removing age spots and restoring skin brightness, compositions for advanced pain relief, muscle relaxers, hormone replacement products, wound healing formulations, products for reducing fine lines and wrinkles, stretch mark reducing products, growth factor products, and anti-psoriasis products.

### SKIN BRIGHTENING OR AGE SPOT REDUCING PRODUCT:

| | |
|---|---|
| Melaslow (10%) | 30 ml |
| Ethoxylated Macadamia nut oil (16 ethoxylations/molecule) | 160 ml |
| Ethanol | 80 ml |
| Water | 40 ml |
| Marine collagen (1%) | 40 ml |
| Etioline (5%) | 30 ml |

This formulation was found to rapidly reduce the appearance of age spots in a subject that applied daily amounts of the product for thirty days.

### STRETCH MARK REDUCING PRODUCTS:

### FORMULATION #1

| | |
|---|---|
| Eucalyptus oil | 400 ml |
| Ethanol | 180 ml |
| Ethoxylated macadamia nut oil (16 ethoxylations/molecule) | 180 ml |
| Distilled water | 40 ml |
| various perfumes were added including | |
| lemon oil or | 30 drops |
| lavender or | 30 drops |
| sweet orange or | 1 ml |
| tangerine | 30 drops |

### FORMULATION #2

| | |
|---|---|
| Eucalyptus oil | 500 ml |
| Ethanol | 225 ml |
| Ethoxylated macadamia nut oil (16 ethoxylations/molecule) | 225 ml |
| Distilled water | 50 ml |

### FORMULATION #3

| | |
|---|---|
| Eucalyptus oil (*Kayuuputih* oil) | 400 ml |
| Ethanol | 220 ml |
| Ethoxylated macadamia nut oil (16 ethoxylations/molecule) | 180 ml |
| Distilled water | 40 ml |
| Y-Ling-Y-Lang | 22 drops |
| Coconut oil | 3 ml |

These formulations were found to rapidly reduce the appearance of stretch marks in a subject that applied daily amounts of the products for thirty days.

### TESTOSTERONE SUPPLEMENTATION PRODUCTS:

### FORMULATION #1

| | |
|---|---|
| Ethanol | 30 ml |
| Ethoxylated macadamia nut oil (16 ethoxylations/molecule) | 30 ml |
| Water | 20 ml |
| Testosterone | 10 ml (200 mg/ml) |
| Coconut oil | 10 drops |

### FORMULATION #2

| | |
|---|---|
| Ethanol | 40 ml |
| Ethoxylated macadamia nut oil (16 ethoxylations/molecule) | 40 ml |
| Water | 5 ml |
| Testosterone | 5 ml (200 mg/ml) |
| Coconut oil | 10 drops |
| Y-Ling-Y-Lang oil | 10 drops |

### FORMULATION #3

| | |
|---|---|
| Testosterone | 10 ml (200 mg/ml) |
| Ethanol | 40 ml |
| Ethoxylated macadamia nut oil (16 ethoxylations/molecule) | 40 ml |
| Coconut oil | 10 drops |
| Y-Ling-Y-Lang oil | 10 drops |
| Water | 3 ml |

### FORMULATION #4

| | |
|---|---|
| Testosterone | 1,000 mg in 5 ml |
| Ethanol | 50 ml |
| Ethoxylated macadamia nut oil (16 ethoxylations/molecule) | 40 ml |
| Water | 5 ml |
| Y-Ling-Y-Lang oil | 15 drops |
| Rain water | 15 drops |

These formulations were found to rapidly increase the amount of testosterone in the blood of a subject that applied approximately 0.5ml of the product daily.

### PAIN RELIEF PRODUCTS:

### FORMULATION #1

| | |
|---|---|
| Ethyl alcohol | 10.4 g |
| White willow bark extract | 10.4 g |
| Glucosamine HCL | 10g |
| MSM | 10g |
| Chrondroitan sulfate sodium | 10g |
| Marine collagen (1%) | 100 ml |
| *Aloe Vera* (whole leaf) concentrate | 100 ml |
| Ethoxylated macadamia nut oil (16 ethoxylations/molecule) | 300 ml |
| Y-Ling-Y-Lang oil | 28 drops |
| Coconut oil | 3 ml |
| Ibuprofen | 16 g |

### FORMULATION #2

| | |
|---|---|
| Ibuprofen | 3 g |
| Methocarbanol | 3 g |
| Chlorzoxazone | 5 g |
| Ethanol | 75 ml |
| Macadamia nut oil (16 ethoxylations/molecule) | 75 ml |
| *Aloe Vera* (whole leaf) concentrate | 5 ml |
| Y-Ling-Y-Lang oil | 10 drops |

Compounds brought into solution with slight heat.

### FORMULATION #3

| | |
|---|---|
| Acetyl salicylic acid | 22 g |
| Ibuprofen | 8.5 g |
| Ethanol (undenatured) | 500 ml |
| Ethoxylated macadamia nut oil (16 ethoxylations/molecule) | 400 ml |
| Distilled water | 100 ml |
| Peppermint oil | 20 drops |

### FORMULATION #4

| | |
|---|---|
| Acetyl salicylic acid | 44 g |
| Undenatured ethanol | 800 ml |
| Ethoxylated macadamia nut oil (16 ethoxylations/molecule) | 200 ml |
| Distilled water | 40 drops |
| Y-ling Y-lang oil | 40 drops |
| Peppermint oil | 40 drops |

### FORMULATION #5

| | |
|---|---|
| Acetyl salicylic acid | 44 g |
| Undenatured ethanol | 900 ml |
| Ethoxylated macadamia nut oil (16 ethoxylations/molecule) | 1000 ml |
| Distilled water | 100 ml |
| Y-ling y-lang oil | 40 drops |
| Peppermint oil | 40 drops |

### FORMULATION #6

| | |
|---|---|
| Liquid aspirin | 44 g |
| Undenatured ethanol | 800 ml |
| Ethoxylated macadamia nut oil (16 ethoxylations/molecule) | 200 ml |
| Distilled water | 40 drops |
| Y-ling y-lang oil | 20 drops |
| Peppermint oil | 40 drops |

These formulations were found to reduce pain in several subjects within 5-20 minutes after application. Depending on the formulation, the period of pain reduction lasted from 45 minutes (*e.g*., acetyl salicylic acid preparations) to several hours (*e.g.*, ibuprofen containing preparations).

### SKIN CARE/ANTI-PSORIASIS/ANTI-ECZEMA/ WOUND HEALING PRODUCTS:

### FORMULATION #1

| | |
|---|---|
| Dmae bitartrate | 22.5 g |
| Alpha lipoic acid | 5 g |
| Ethyl alcohol | 25 ml |
| Marine collagen (1%) | 25 ml |
| *Aloe Vera* | 25 ml |
| Macadamia nut oil (16 ethoxylations/molecule) | |

The Dmae bitartrate and alpha lipoic acid was brought into solution and filtered prior to mixture with the ethoxylated macadamia nut oil.

### FORMULATION #2

| | |
|---|---|
| Ichtyocollagene (1%) | 500 ml |
| Distilled water | 248 ml |
| LKEKK (**SEQ. ID. No. 1**) | 1 vial (about 1 ml ∼10 µg) |
| Ethoxylated macadamia nut oil (16 ethoxylations/molecule) | 150 ml |
| Ethanol | 25 ml |
| Phenochem (*i.e.*, a mixture of Methyl Paraben, Ethyl Paraben, Propyl Paraben, Butyl Paraben, and Isobutyl Paraben) | 39 ml |

### FORMULATION #3

| | |
|---|---|
| Distilled water | 100 ml |
| LKEKK (**SEQ. ID. No. 1**) | 5 bottles (∼50µg) |
| Ethoxylated macadamia nut oil (16 ethoxylations/molecule) | 40 ml |
| Ethanol | 5 ml |

These formulations were found to improve the healing of a wound (a laceration) and were found to reduce psoriasis and eczema in an afflicted subject.

### FORMULATION #4

| | |
|---|---|
| Distilled Water with Sodium Bi Carbonate (pH 8.2-8.6) | 18 ml |
| Hepsyl | 5 g |
| Ethoxylated macadamia nut oil (16 ethoxylations/molecule) | 20 ml |
| Ethyl Alcohol Anhydrous | 20 ml |

This formulation reduces psoriasis and eczema in an afflicted subject.

### PRODUCTS THAT REDUCE THE APPEARANCE OF FINE LINES AND WRINKLES

### FORMULATION #1

| | |
|---|---|
| Ichtyocollagene (1%) | 2,990 ml |
| Distilled water | 1,483 ml |
| Ethoxylated Macadamia nut oil (16 ethoxylations/molecule) | 922 ml |
| Ethanol | 150 ml |
| Matrixyl (8%) | 236 ml |
| Phenochem | 236 ml |
| Ethoxydiglycol | 33 ml |

### FORMULATION #2

| | |
|---|---|
| Ichtyocollagene (6%) | 250 ml |
| Distilled water | 124 ml |
| Ethoxylated macadamia nut oil (16 ethoxylations/molecule) | 78 ml |
| Phenochem | 20 ml |
| Bio-ten (Atrium Biotechnologies, Inc., Quebec, Canada) | 1 ml |
| Ethanol | 10 ml |

### FORMULATION #3

| | |
|---|---|
| Ichtyocollagene (1%) | 500 ml |
| Distilled water | 250 ml |
| Ethoxylated macadamia nut oil (16 ethoxylations/molecule) | 125 ml |
| Ethanol | 2 ml |
| Bio-ten | 3 ml |
| Phenochem | 40 ml |

### FORMULATION #4

| | |
|---|---|
| Ichtyocollagene (1%) | 2,990 ml |
| Distilled water | 1,483 ml |
| Ethoxylated macadamia nut oil | 922 ml |
| (16 ethoxylations/molecule) | |
| Ethyl alcohol | 150 ml |
| Matrixyl | 236 ml |
| Phenochem | 236 ml |

### FORMULATION #5

| | |
|---|---|
| Ichtyocollagene (1%) | 1,994 ml |
| Distilled water | 999 ml |
| Ethoxylated macadamia nut oil | 675 ml |
| (16 ethoxylations/molecule) | |
| Ethanol | 100 ml |
| Bioserum | 24 ml |
| (Atrium Biotechnologies, Inc., Quebec, Canada) | |
| Phenochem | 157 ml |

### FORMULATION #6

| | |
|---|---|
| Ichtyocollagene (1%) | 500 ml |
| Distilled water | 250 ml |
| Ethoxylated macadamia nut oil | 168.75 ml |
| (16 ethoxylations/molecule) | |
| Ethanol | 25 ml |
| Bioserum | 10 ml |
| Phenochem | 43.75 ml |

### FORMULATION #7

| | |
|---|---|
| Ichtyocollagene (1%) | 1,000 ml |
| Ethoxylated macadamia nut oil | 338 ml |
| (16 ethoxylations/molecule) | |
| Distilled water | 500 ml |
| Ethanol | 50 ml |
| Matrixyl | 76 ml |
| Phenochem | 76 ml |

### FORMULATION #8

| | |
|---|---|
| Ichtyocollagene (1%) | 22.55 ml |
| Distilled Water | 11.7 ml |
| Ethoxylated macadamia nut oil | 7 ml |
| (16 ethoxylations/molecule) | |
| Phenochem | 0.5 ml |
| Ethanol | 1.5 ml |
| Bio Serum | 1 ml |
| | |
| TOTAL | 44.25 ml |

### FORMULATION #9

| | |
|---|---|
| Ichtyocollagene (1%) | 15.03 ml |
| Distilled Water | 7.8 ml |
| Ethoxylated macadamia nut oil | 4.67 ml |
| (16 ethoxylations/molecule) | |
| Phenochem | 0.333 ml |
| Ethanol | 1 ml |
| Bio Serum | 0.67 ml |
| | |
| TOTAL | 29.5 ml |

### FORMULATION #10

| | |
|---|---|
| Ichtyocollagene (1%)(Marine Collagen, Sderma) | 150 ml |
| Distilled Water | 400 ml |
| Ethoxylated macadamia nut oil | 120 ml |
| (16 ethoxylations/molecule) | |
| Ethyl Alcohol (anhydrous) | 10 ml |
| Yling Ylang | 16 drops |
| Crodaderm B (Croda, Inc.) | 5.0 ml |
| Phenochem | 2.0 ml |
| Sepigel | 15 g |

Formulation #10 is mixed in the order listed, and heated to 80°F.

These formulations were found to reduce the appearance of fine lines and wrinkles in subjects that applied the formulations daily for thirty days. It should be noted that Bioserum, which is obtainable from Atrium Biosciences, Ontario Canada, may contain one or more of the following: placental protein, amniotic fluid, calf skin extract, and serum protein. Also, phenochem may contain one or more of the following: Methyl Paraben, Ethyl Paraben, Propyl Paraben, Butyl Paraben, and Isobutyl Paraben, and sodium methylparaban imidizolidinyl urea. Additional components that may be included in some formulations of products that reduce the appearance of fine lines and wrinkles include: igepal cephene distilled, synasol, ethoxylated glycerides, trisodium EDTA, potassium sorbate, citric acid, ascorbic acid, and distilled water. For example, one formulation contains: Collagen (Marine), Distilled Water, Igepal Cephene Distilled, Methyl Paraben, Ethyl Paraben, Propyl Paraben, Butyl Paraben, Isobutyl Paraben, Synasol, Serum Protein, Purified Water, Amniotic Fluid. Placental Protein. Calfskin Extract, Hydrolyzed Collagen Sodium Methylparaben Imidazolidinyl Urea. Ethoxylated Glycerides, Trisodium EDTA, Potassium Sorbate, Citric Acid, and Ascorbic Acid.

### SPOT FAT REDUCERS

### FORMULATION #1

| | |
|---|---|
| Epigallocatechin Gallate (ECGC) | 40 g |
| Ethyl Alcohol | 100 ml |
| Distilled Water | 100 ml |
| Ethoxylated macadamia nut oil (16 ethoxylations/molecule) | 60 ml |
| Lipase | 1 ml |

### FORMULATION #2

| | |
|---|---|
| Epigallocatechin Gallate (ECGC)(DSM, Netherlands) | 40 g |
| Ethyl Alcohol | 100 ml |
| Distilled Water | 100 ml |
| Ethoxylated macadamia nut oil (16 ethoxylations/molecule) | 60 ml |
| Lipase Caffeine | 1 ml 2.0 g |

These formulations were found to reduce fat when applied to the body in individual. Polyphenols other than ECGC, such as analogs of green tea extract, are suitable in the above formulations and can be substituted for or used in combination with ECGC in the above forumations. The following example describes experiments that employed two different skin cell model systems to evaluate the ability of a transdermal delivery composition containing collagen to transport collagen to skin cells.

### EXAMPLE 5

In this example, it is shown that a transdermal delivery composition of the invention comprising marine type 1 collagen or native collagen efficiently transported the delivered agent to skin cells. Two different *in vitro* skin cell model systems were used, human cadaver skin and a cellulose acetate skin cell model system. Based on the physiology of the skin, three possible pathways exist for passive transport of molecules through the skin to the vascular network: (1) intercellular diffusion through the lipid lamellae; (2) transcellular diffusion through both the keratinocytes and lipid lamellae; and (3) diffusion through appendages (hair follicles and sweat ducts). The cellulose acetate skin model evaluates the ability of the delivered agent to transport using the first two pathways and the human cadaver skin evaluates the ability to use all three pathways.

In brief, the transdermal delivery composition comprising collagen was applied to the cellulose acetate and the human cadaver skin in a diffusion chamber and the results were recorded after 10 minutes, 30 minutes and one hour. The diffused material was analyzed by a spectrophotometer (Hitachi U2000 multiscan spectrophotometer). A portion of the diffused material was also separated on a gel by electrophoresis and the collagen was stained using a collagen-specific dye. A portion of the diffused material was also immunoprecipitated using polyclonal antibodies specific for collagens types 1-7 and the immunoprecipitates were analyzed by immunodiffusion.

The table below provides the collagen concentration in the various samples of transdermal delivery compositions tested. The protein concentration was determined using a micro-protein assay (Bio-Rad).

**TABLE 20**

| Sample number | Protein Concentrations | |
|---|---|---|
| | Native type 1 Collagen | Marine type 1 collagen |
| Sample 1 | 0.40 mg/ml | 1.14 mg /ml |
| Sample 2 | 0.44 mg/ml | 1.09 mg /ml |
| Sample 3 | 0.42 mg/ml | 1.14 mg /ml |
| Average | 0.42 | 1.12 |
| Standard error | 0.011 | 0.017 |
| Variance | 0.0004 | 0.0008 |
| Standard deviation | 0.02 | 0.03 |

### Penetration analysis

The transdermal delivery composition containing either marine collagen or native collagen was applied to the human cadaver skin and the cellulose acetate skin model systems. The penetration studies were performed in a diffusion chamber and the results were recorded at 10 minutes, 30 minutes and an hour later. Sections of skin or cellulose acetate were stained with a collagen specific dye and a light microscope was used to visualize the transported collagen. **TABLE 21** provides the results of these experiments. Note, that the native collagen appeared to penetrate the skin in less time than the marine collagen. This may be due to the differing concentrations of collagen used in the transdermal delivery compositions (*i.e.*, the concentration of the native collagen was 0.40 mg/ml and the concentration of the marine collagen was 1.14 mg/ml). Nevertheless, by one hour, almost all of both types of collagen had penetrated the skin in the model systems employed.

**TABLE 21**

| Product Hydroderm | Percent Penetration as per time interval | | | |
|---|---|---|---|---|
| Marine Collagen Vial A | 10 minutes | 20 minutes | 30 minutes | 60 minutes |
| Sample A1 | 40% | 60% | 75% | 95% |
| Sample A2 | 40% | 60% | 75% | 95% |
| Sample A3 | 40% | 60% | 75% | 95% |

| Marine Collagen Vial B | | | | |
|---|---|---|---|---|
| Sample B1 | 40% | 60% | 75% | 95% |
| Sample B1 | 40% | 60% | 75% | 95% |
| Sample B1 | 40% | 60% | 75% | 95% |

| Marine collagen Vial C | | | | |
|---|---|---|---|---|
| Sample C1 | 40% | 60% | 75% | 95% |
| Sample C1 | 40% | 60% | 75% | 95% |
| Sample C1 | 40% | 60% | 75% | 95% |

| Native Collagen | | | | |
|---|---|---|---|---|
| Sample 1 | 80% | 95% | | |
| Sample 2 | 80% | 95% | | |
| Sample 3 | 80% | 95% | | |

When similar concentrations of native collagen and marine collagen were used in a transdermal delivery composition, the native collagen and the marine collagen penetrated the upper three layers of the epidermis in approximately one hour. The marine collagen and the native collagen were localized in the upper three layers of the human cadaver epidermis using a collagen specific dye. A similar distribution of the collagen was confirmed by the cellulose acetate skin model. *See* **TABLES 22** and **23.**

**TABLE 22**

| PENETRATION IN THE LAYERS OF THE HUMAN SKIN EPIDERMIS | | | | | |
|---|---|---|---|---|---|
| | Penetration of Epidermis layers of the Skin (Human Skin diffusion chamber study) | | | | |
| Marine collagen Vial A | *Stratum Corneum* | *Stratum lucidum* | *Stratum Granulosum* | *Stratum Spinosum* | *Stratum Basale* |
| Sample A1 | √ | √ | √ | - | - |
| Sample A2 | √ | √ | √ | - | - |
| Sample A3 | √ | √ | √ | - | - |

| Marine collagen Vial B | | | | | |
|---|---|---|---|---|---|
| Sample B1 | √ | √ | √ | - | - |
| Sample B1 | √ | √ | √ | - | - |
| Sample B1 | √ | √ | √ | - | - |

| Marine collagen Vial C | | | | | |
|---|---|---|---|---|---|
| Sample C1 | √ | √ | √ | - | - |
| Sample C1 | √ | √ | √ | - | - |
| Sample C1 | √ | √ | √ | - | - |

| Native collagen | | | | | |
|---|---|---|---|---|---|
| Sample 1 | √ | √ | √ | - | - |
| Sample 2 | √ | √ | √ | - | - |
| Sample 3 | √ | √ | √ | - | - |

Note: (√) indicates the presence of the product in the above layers of the epidermis as determined by collagen specific staining observed by light microscopy after one hour of product application. (-) indicates absence of products in these layers of the epidermis.

**TABLE 23**

| | Penetration Hydroderm in Epidermis layers of the Skin (Cellulose Acetate model skin diffusion chamber study) | | | | |
|---|---|---|---|---|---|
| Marine collagen Vial A | *Stratum Corneum* | *Stratum lucidum* | *Stratum Granulosum* | *Stratum Spinosum* | *Stratum Basale* |
| Sample A1 | √ | √ | √ | - | - |
| Sample A2 | √ | √ | √ | - | - |
| Sample A3 | √ | √ | √ | - | - |

| Marine collagen Vial B | | | | | |
|---|---|---|---|---|---|
| Sample B1 | √ | √ | √ | - | - |
| Sample B1 | √ | √ | √ | - | - |
| Sample B1 | √ | √ | √ | - | - |

| Marine collagen Vial C | | | | | |
|---|---|---|---|---|---|
| Sample C1 | √ | √ | √ | - | - |
| Sample C1 | √ | √ | √ | - | - |
| Sample C1 | √ | √ | √ | - | - |

| Native Collagen | | | | | |
|---|---|---|---|---|---|
| Sample 1 | √ | √ | √ | - | - |
| Sample 2 | √ | √ | √ | - | - |
| Sample 3 | √ | √ | √ | - | - |

Note: (√) indicates the presence of the product in the above layers of the epidermis as determined by collagen specific staining observed by light microscopy after one hour of product application. (-) indicates absence of products in these layers of the epidermis.

### Spectrophotometric Analysis

Spectrophotometric analysis of the diffused material revealed that the transdermal delivery composition enabled significant transport of both types of collagens. *See* **TABLE 24**.

**TABLE 24**

| Sample number | Spectral Absorbance at wavelength 280nm | |
|---|---|---|
| | Native type 1 collagen | Marine type 1 collagen |
| Sample 1 | 2.35 | 2.832 |
| Sample 2 | 2.766 | 2.772 |
| Sample 3 | 2.751 | 2.683 |
| Average | 2.622 | 2.762 |
| Standard error | 0.136 | 0.043 |
| Variance | 0.0557 | 0.0056 |
| Standard deviation | 0.24 | 0.07 |

### Electrophoresis Analysis

A portion of the diffused material was then separated by electrophoresis and visualized by staining with a collagen-specific dye. The penetrated marine collagen remained intact during and after the analysis because the labeled marine collagen detected in the diffused material was observed to have the same molecular weight as marine collagen that had not undergone the analysis (control sample). The results showed that the marine collagen prior to the penetration study and after the penetration study maintained its molecular structure around 500 kilodaltons (KD). The native collagen also maintained a molecular weight around 500KD before and after penetration of the epidermis, demonstrating that the native collagen that was delivered by the transdermal delivery composition, like the marine collagen, remained intact into the epidermis.

### Immunoprecipitation Analysis

When the transdermal delivery composition containing marine collagen was immunoprecipitated using polyclonal antibodies specific for collagens types 1-7 before and after the penetration study, more evidence that the marine collagen remained in tact after the transdermal delivery was obtained. Immuno-diffusion studies verified that the marine collagen prior to penetration of the skin and post penetration of skin consisted mainly of type I collagen. This further confirmed that the collagen remained intact post penetration.

The penetration study described above provided strong evidence that the transdermal delivery compositions described herein are effective at transporting high molecular weight molecules to skin cells. It was found, for example, that marine collagen type 1 (∼500 kD) effectively penetrated the upper 3 layers of the epidermis and remained intact within an hour. These findings were supported by histology, spectrophotometric analysis, electrophoretic separation analysis, immunoprecipitation analysis, and immuno-diffusion analysis. The following example describes a clinical study that was performed, which verified that the transdermal delivery compositions described herein effectively reduce wrinkles and improve skin tone in humans in need thereof.

### EXAMPLE 6

A clinical study was performed to evaluate the ability of a transdermal delivery composition containing collagen, prepared as described herein, to reduce wrinkles and fine lines and otherwise restore skin tone to subjects in need thereof. The medial half of the facial region including the neck and the upper chest areas were assigned as the regions under investigation. During a subject's routine application of the product, three times a day, digital pictures were taken at days 0, 3, 7, 14 and 21 of the regions under investigation of the face including the symmetrical region of the face where the product was not applied. Micrometer measurements of the wrinkles were then made from the digital pictures and also from the facial areas under investigation.

Subjects invited to participate in the study had facial wrinkles and were 25 years or older. Non-facial wrinkle individuals were also invited and served as the control group. The source of subjects for the study was randomly selected from the ethnically diverse population group ages ranging from 25 years to 88 years old.

**Table 25**

| *Description of the subjects participating in the study* | | | | |
|---|---|---|---|---|
| Identification Number | Gender | Ethnicity | Age | General Description |
| F101601 | Female | Hispanic American | 88 | Distinct facial wrinkles |
| F101602 | Female | Hispanic American | 67 | Distinct facial wrinkles |
| F101603 | Female | Hispanic American | 25 | Distinct facial wrinkles around the eyes |
| F101604 | Female | Caucasian | 28 | Distinct facial wrinkles around the eye region |
| M101605 | Male | Asian | 40 | Distinct facial wrinkles around the eye region |

Subjects that signed the study consent form received 30 mls of a transdermal delivery composition comprising marine collagen. Micrometer measurements of the wrinkles were performed using a 10X magnification objective eye piece. The measurements were recorded and tabulated together with the digital photographs before and after application of the product. The wrinkle measurements were determined within the 3-week duration of the study. The tabulated results provided in **TABLE 26,** which indicates the general observations by subjects utilizing the product, and **TABLE 27,** which shows the wrinkle measurements. **TABLE 28** shows the average percent of wrinkle reduction data generated after 21 days of application of the transdermal delivery composition comprising collagen.

**TABLE 26**

| **Identification Number** | **Days of product application on one half of the face including the upper chest and neck regions** | | | |
|---|---|---|---|---|
| | **Day 3** | **Day 7** | **Day 14** | **Day 21** |
| F101601 | Skin felt soft, and clear, when compared to the other half without product application, slight burning sensation for 3-5 minutes upon product application. | The right half of the face cleared up and felt smooth, the slight burning sensation was still present for 3-5 minutes. | The right half of the face cleared up and felt smooth, the slight burning sensation no longer present. | The right half of the face cleared up and felt smooth, the slight burning sensation no longer present. |
| F101602 | Skin felt soft, and clear, when compared to the other half without product application, slight burning sensation for 3-5 minutes upon product application. | The right half of the face cleared up and felt smooth, the slight burning sensation ' was still present for 3-5 minutes. | The right half of the face cleared up and felt smooth, the slight burning sensation no longer present. | The right half of the face cleared up and felt smooth, the slight burning sensation no longer present. |
| F101603 | Skin felt soft, and clear, when compared to the other half without product application, slight burning sensation for 3-5 minutes upon product application. | The right half of the face cleared up and felt smooth, the slight burning sensation was still present for 3-5 minutes. | The right half of the face cleared up and felt smooth, the slight burning sensation no longer present. | The right half of the face cleared up and felt smooth, the slight burning sensation no longer present.. |
| F101604 | Skin felt soft, and clear, when compared to the other half without product application, slight burning sensation for 3-5 minutes upon product application | The skin felt smooth and very soft in the facial region where product was applied. | Developed rashes in the neck region, stopped using product. | The rashes cleared up, and the skin had normal appearance as the other half in which the product was not applied. |
| M101605 | Skin felt soft, and clear, when compared to the other half without product application, slight burning sensation for 3-5 minutes upon product application. | The right half of the face cleared up and felt smooth, the slight burning sensation was still present for 3-5 minutes. | The right half of the face cleared up and felt smooth, the slight burning sensation still present for 3-5 minutes. | The right half of the face cleared up and felt smooth, the slight burning sensation still present for 3-5 minutes. |

**TABLE 27**

| **Subject's Identification Number** | **Average wrinkle measurements with product application on one half of the face including the upper chest and neck areas in µm** | | | | | | |
|---|---|---|---|---|---|---|---|
| | **Regions of the face** | **Day 0** | **Day 3** | **Day 5** | **Day 7** | **Day 14** | **Day 21** |
| F101601 | Around eyes | 6 µm | 6 µm | 6 µm | 5 µm | 4.5 µm | 4.5 µm |
| | Temporal cheek | 7 µm | 7 µm | 7 µm | 7 µm | 6 µm | 5.5 µm |
| | Chin | 7.5 µm | 7.5 µm | 7.5 µm | 7.5 µm | 7.0 µm | 6.5 µm |
| | Around mouth | 6.5 µm | 6.5 µm | 6.5 µm | 6.5 µm | 6.0 µm | 5.5 µm |
| F101602 | Around eyes | 3.5 µm | 3.5 µm | 3.5 µm | 3.5 µm | 3.5 µm | 3.2 µm |
| | Temporal cheek | 4.1 µm | 4.1 µm | 4.1 µm | 4.1 µm | 3.9 µm | 3.5 µm |
| | Chin | 2.5 µm | 2.5 µm | 2.5 µm | 2.5 µm | 2.0 µm | 2.0 µm |
| | Around mouth | 2.0 µm | 2.0 µm | 2.0 µm | 2.0 µm | 2.0 µm | 2.0 µm |
| F101603 | Around eyes | 1.5 µm | 1.5 µm | 1.5 µm | 1.5 µm | 1.5 µm | 1.2 µm |
| | Temporal cheek | 1.0 µm | 1.0 µm | 1.0 µm | 1.0 µm | 1.0 µm | 1.0 µm |
| | Chin | 0.9 µm | 0.9 µm | 0.9 µm | 0.9 µm | 0.9 µm | 0.85 µm |
| | Around mouth | 0.5 µm | 0.5 µm | 0.5 µm | 0.5 µm | 0.5 µm | 0.45 µm |
| F101604 | Around eyes | 0.2 µm | 0.2 µm | 0.2 µm | 0.2 µm | 0.2 µm | ** |
| | Temporal cheek | 1.5 µm | 1.5 µm | 1.5 µm | 1.5 µm | 1.5 µm | ** |
| | Chin | 1.0 µm | 1.0 µm | 1.0 µm | 1.0 µm | 1.0 µm | ** |
| | Around mouth | 0.5 µm | 0.5 µm | 0.5 µm | 0.5 µm | 0.5 µm | ** |
| M101605 | Around eyes | 1.5 µm | 1.5 µm | 1.5 µm | 1.5 µm | 1.5 µm | 1.0 µm |
| | Temporal cheek | 0.5 µm | 0.5 µm | 0.5 µm | 0.5 µm | 0.5 µm | 0.3 µm |
| | Chin | 1.0 µm | 1.0 µm | 1.0 µm | 1.0 µm | 1.0 µm | 0.9 µm |
| | Around mouth | 1.5 µm | 1.5 µm | 1.5 µm | 1.5 µm | 1.5 µm | 1.2 µm |

| | | | | | | | |
|---|---|---|---|---|---|---|---|
| Note ** Indicates the subject stopped using the product. | | | | | | | |

**TABLE 28**

| **Subject's Identification Number** | **The percent reduction of wrinkle measurement on the regions of the face at day 21 of Hydroderm product application** | | | |
|---|---|---|---|---|
| | **Around eyes** | **Temporal cheek** | **Chin** | **Around mouth** |
| F101601 | 25% | 21.4% | 13.3% | 15.4% |
| F101602 | 8.6% | 14.6% | 20.0% | 0.0% |
| F101603 | 20.0% | 0.0% | 5.6% | 10.0% |
| F101604 | 0.0% | 0.0% | 0.0% | 0.0% |
| M101605 | 33.3% | 40.0% | 10% | 20.0% |
| | | | | |
| Average % | 17.42% | 15.20% | 9.78% | 9.08% |
| Overall effectiveness | On the entire facial region where the product was applied. | | | 10.29% |

The data generated from this study indicates that the overall effectiveness of transdermal delivery composition comprising marine collagen as a wrinkle reducer is 10.29% when applied twice daily for 21 days. As indicated by Table 28, the percent reduction of the wrinkles varies with the various areas of the face where it is applied, with 17.4% reduction around the eye regions and 15.20% at the temporal cheeks at the high end and around 9% at the chin and mouth regions. The next example sets forth experiments that demonstrate that transdermal delivery compositions containing ethoxylated oils of less than 20 ethoxylations/molecule transfer a delivered agent to the skin more effectively than transdermal delivery compositions containing ethoxylated oils of 20 or more ethoxylations/molecule.

### EXAMPLE 7

Several transdermal delivery composition formulations containing collagen (1.2mg/ml) and an ethoxylated oil having different amounts of ethoxylations/molecule are prepared. Formulations containing ethoxylated oil of either 12, 16, 18, 20, 24, and 30 ethoxylations/molecule, water, and marine collagen (1.2mg/ml) are made. Approximately 0.5ml of each of these formulations are applied to human cadaver skin in a diffusion chamber and the penetration of collagen is monitored over time (*e.g.*, 10 minutes, 30 minutes, 45 minutes and one hour). Sections of the skin are taken, stained with a collagen specific dye, and the stained sections are analyzed under a light microscope.

A greater amount of collagen-specific staining will be seen in stained skin sections collected at the various time points with formulations containing less than 20 ethoxylations/molecule than with formulations containing 20 or more ethoxylations/molecule. Formulations containing less than 20 ethoxylations/molecule will also penetrate the skin faster than formulations containing 20 or more ethoxylations/mo lecule.

In a second set of experiments, the collagen that has penetrated the skin at the various time points above is collected from the diffusion chamber and analyzed in a spectrophotometer. As above, a greater amount of collagen will be detected in samples collected at the various time points with formulations containing less than 20 ethoxylations/molecule than formulations containing 20 or more ethoxylations/molecule. Formulations containing less than 20 ethoxylations/molecule will also be observed to penetrate the skin faster than formulations containing 20 or more ethoxylations/molecule. The next example sets forth experiments that demonstrate that transdermal delivery compositions containing ethoxylated fatty acids having 10-19 ethoxylations/molecule transfer a delivered agent to the skin as effectively as transdermal delivery compositions containing ethoxylated oils having 10-19 ethoxylations/molecule.

### EXAMPLE 8

A transdermal delivery composition containing collagen (1.2mg/ml) and an ethoxylated fatty moiety having 16 ethoxylations/molecule, water and marine collagen is made. Several transdermal delivery compositions containing ethoxylated fatty moieties and having 16 ethoxylations/molecule, water, and marine collagen are made. Approximately 0.5ml of each of these formulations are applied to human cadaver skin in a diffusion chamber and the penetration of collagen is monitored over time (e.g., 10 minutes, 30 minutes, 45 minutes and one hour). Sections of the skin are taken, stained with a collagen specific dye, and the stained sections are analyzed under a light microscope.

The same amount of collagen-specific staining will be seen in stained skin sections collected at the various time points with formulations containing ethoxylated fatty moieties as compared to formulations containing ethoxylated oils. Formulations containing ethoxylated fatty moieties will also penetrate the skin at approximately the same rate as compared to formulations containing ethoxylated oils.

In a second set of experiments, the collagen that has penetrated the skin at the various time points above is collected from the diffusion chamber and analyzed in a spectrophotometer. As above, approximately the same amount of collagen will be detected in samples collected at the various time points with formulations containing ethoxylated fatty moieties as compared to formulations containing ethoxylated oils. Formulations containing ethoxylated fatty moieties will also be observed to penetrate the skin at approximately the same rate as formulations containing ethoxylated oils.

### EXAMPLE 9

A transdermal delivery composition containing collagen (1.2mg/ml) and an ethoxylated oil having 16 ethoxylations/molecule, water and marine collagen is made. A portion of the composition is transferred to a cartridge adapted for the exemplary transdermal delivery device described herein. The transdermal delivery device is preset to load approximately 0.5ml of the formulation. Approximately 0.5ml of the formulation is applied to human cadaver skin, either manually, or using the transdermal delivery device, in a diffusion chamber and the penetration of collagen is monitored over time (e.g., 10 minutes, 30 minutes, 45 minutes and one hour). Sections of the skin are taken, stained with a collagen specific dye, and the stained sections are analyzed under a light microscope. Several samples are prepared and treated at the same time.

The amount of collagen-specific staining seen in stained skin sections collected is substantially more consistent in the samples in which the formulation is administered via the transdermal delivery device than in the samples in which the formulation is delivered manually.

In a second set of experiments, the collagen that has penetrated the skin at the various time points above is collected from the diffusion chamber and analyzed in a spectrophotometer. As above, the amount of collagen detected in samples collected from the various samples in which the formulation is delivered via the transdermal delivery device shows considerably less variation than the amounts of collagen calculated from samples in which the formulation was applied manually.

## Claims

1. A transdermal delivery composition comprising a liposphere, wherein said liposphere comprises an ethoxylated fatty moiety or lipid moiety and a delivered agent, wherein the amount of ethoxylation of said fatty moiety or lipid moiety is 10, 11, 12, 13, 14, 15, 16, 17, 18, or 19 ethoxylations per molecule, and wherein said fatty moiety or lipid moiety comprises 10 carbon residues.

2. The transdermal delivery composition of Claim 1 further comprising water.

3. The transdermal delivery composition of Claim 1 further comprising alcohol.

4. The transdermal delivery composition of Claim 1, wherein said fatty moiety or lipid moiety comprises at least 10 carbon residues but less than or equal to 30 carbon residues.

5. The transdermal delivery composition of Claim 1, wherein the amount of ethoxylation is the same as the number of carbons residues in the fatty moiety or lipid moeity.

6. The transdermal delivery composition of Claim 1, wherein the fatty moiety or lipid moiety chain length is 10, 12, 14, 16, 18, 20, 22 or 24 carbon residues in length.

7. The transdermal delivery composition of Claim 1, wherein the fatty moiety is a fatty acid.

8. The transdermal delivery composition of Claim 7, wherein the fatty acid is selected from the group consisting of C16:1, delta 1; C16:1, delta 2; C16:1, delta 3; C16:1, delta 4; C16:1, delta 5; C16:1, delta 6; C16:1, delta 7; C16:1, delta 8; C16:1, delta 9 (palmitoleic acid); C16:1, delta 10; C16:1, delta 11; C16:1, delta 12; C16:1, delta 13; and C16:1, delta 14.

9. The transdermal delivery composition of Claim 1, wherein said fatty moiety is a fatty amine.

10. The transdermal delivery composition of Claim 1, wherein said lipid moiety is a sphingolipid, a glycolipid or a glycosphingolipid.

11. The transdermal delivery composition of Claim 1, wherein said liposphere comprises a homogeneous mixture of an ethoxylated fatty moiety or a heterogeneous mixture of ethoxylated fatty acids.

12. The transdermal delivery composition of Claim 1, wherein the delivered agent is a polypeptide hormone.

13. The transdermal delivery composition of Claim 1, wherein said delivered agent is collagen.

14. The transdermal delivery composition of Claim 1, wherein said delivered agent is an anesthetic.

15. The transdermal delivery composition of Claim 1, wherein said delivered agent is an analgesic.

16. The transdermal delivery composition of Claim 1, wherein said delivered agent is a non-steroidal anti inflammatory drug (NSAID) selected from the group consisting of ibuprofen (2-(isobutylphenyl)-propionic acid); methotrexate (N-[4-(2, 4 diamino 6 - pteridinyl - methyl] methylamino] benzoyl)-L-glutamic acid); aspirin (acetylsalicylic acid); salicylic acid; diphenhydramine (2-(diphenylmethoxy)-NN-dimethylethylamine hydrochloride); naproxen (2-naphthaleneacetic acid, 6-methoxy-9-methyl-, sodium salt, (-)); phenylbutazone (4-butyl-1,2-diphenyl-3,5-pyrazolidinedione); sulindac-(2)-5-fuoro-2-methyl-1-[[p-(methylsulfinyl)phenyl]methylene-]-1H-indene-3-acetic acid; diflunisal (2',4', -difluoro-4-hydroxy-3-biphenylcarboxylic acid; piroxicam (4-hydroxy-2-methyl-N-2-pyridinyl-2H-1, 2-benzothiazine-2-carboxamide 1, 1-dioxide, an oxicam; indomethacin (1-(4-chlorobenzoyl)-5-methoxy-2-methyl-H-indole-3-acetic acid); meclofenamate sodium (N-(2, 6-dichloro-m-tolyl) anthranilic acid, sodium salt, monohydrate); ketoprofen (2-(3-benzoylphenyl)-propionic acid; tolmetin sodium (sodium 1-methyl-5-(4-methylbenzoyl-1H-pyrrole-2-acetate dihydrate); diclofenac sodium (2-[(2,6-dichlorophenyl)amino] benzeneatic acid, monosodium salt); hydroxychloroquine sulphate (2-{[4-[(7-chloro-4-quinolyl)amino]pentyl] ethylaminolethanol sulfate (1:1); penicillamine (3-mercapto-D-valine); flurbiprofen ([1,1-biphenyl]-4-acetic acid, 2-fluoro-alphamethyl-, (+-); cetodolac (1-8-diethyl-13,4,9, tetrahydropyrano-[3-4-13]indole-1-acetic acid; mefenamic acid (N-(2,3-xylyl)anthranilic acid; and diphenhydramine hydrochloride (2-diphenyl methoxy-N, N-di-methylethamine hydrochloride).

17. The transdermal delivery composition of Claim 1, wherein the delivered agent is an anti-inflammatory compound selected from the group consisting of hydrocortisone, prednisolone, triamcinolone, cortisone and piroxicam or an anti-infective compound selected from the group consisting of amoxicillin, clavulanate potassium, itraconazole, acyclovir, fluconazole, terbinafine hydrochloride, erythromycin ethylsuccinate, acetyl sulfisoxazole, penicillin V, cephalexin, erythromycin, azithromycin, tetracycline, ciproflaxin, gentamycin, sulfathiazole, nitrofurantoin, norfloxacin, flumequine, and ibafloxacin, metronidazole, nystatin, lamivudine, indinavir sulfate, and stavudine.

18. The transdermal delivery composition of Claim 1, wherein the delivered agent is metformin hydrochloride or acarbose.

19. The transdermal delivery composition of Claim 1, wherein the delivered agent is an immunogen such as an immunogen comprising a polynucleotide.

20. The transdermal delivery composition of Claim 1 wherein the delivered agent is an immune response modifier.

21. The transdermal delivery composition of Claim 1, wherein the delivered agent is an enzyme inhibitor selected from the group consisting of zileuton, captopril, and lisinopril; or the agent is selected from the group consisting of ergotamine, melatonin, sumatriptan, zolmitriptan, and rizatriptan; or is selected from the group consisting of zolpidem, zolpidem tartrate, triazolam, and hycosine butylbromide; or is selected from the group consisting of iohexol, technetium, TC99M, sestamibi, iomeprol, gadodiamide, ioversol, and iopromide or is selected from the group consisting of alsactide, americium, betazole, histamine, mannitol, metyrapone, petagastrin, phentolamine, radioactive B₁₂, gadodiamide, gadopentetic acid, gadoteridol, perflubron, cyclosporine, sildenafil citrate, paclitaxel, ritonavir, and saquinavir.

22. The transdermal delivery composition of any of claims 1-21 for use as a medicament.

23. The transdermal delivery composition of any of claims 1-21 for use for the delivery of a delivered agent.

24. The transdermal delivery composition of Claim 1, wherein the fatty moiety is an unsaturated fatty acid.

25. The transdermal delivery composition of Claim 1, wherein the fatty moiety is a polyunsaturated fatty acid.

26. The transdermal delivery composition of Claim 12, wherein the polypeptide hormone is selected from the group consisting of oxytocin, vasopressin, melanocyte-stimulating hormone, corticortropin, lipotropin, thyrotropin, growth hormone, prolactin, luteinizing hormone, human chorionic gonadotropin, follicle stimulating hormone, corticotropin-releasing factor, gonadotropin-releasing factor, prolactin-releasing factor, prolactin-inhibiting factor, growth-hormone releasing factor, somatostatin, thyrotropin-releasing factor, calcitonin, calcitonin gene-related peptide, parathyroid hormone, glucagon-like peptide 1, glucose-dependent insulinotropic polypeptide, gastrin, secretin, cholecystokinin, motilin, vasoactive intestinal peptide, substance P, pancreatic polypeptide, peptide tyrosine tyrosine, neuropeptide tyrosine, amphiregulin, insulin, glucagon, placental lactogen, relaxin, angiotensin II, atrial natriuretic peptide, and melatonin.

27. The transdermal delivery composition of Claim 1, wherein the delivered agent is a non-peptide hormone.

28. The transdermal delivery composition of Claim 1, wherein the hormone is selected from the group consisting of thyroxine, triiodothyronine, estradiol, estrone, progesterone, testosterone, cortisol, corticosterone, aldosterone, epinephrine, norepinepherine, and calctriol.

29. The transdermal delivery composition of Claim 14, wherein said anesthetic is selected from the group consisting of articaine, procaine, tetracaine, chloroprocaine and benzocaine, novocain, mepivicaine, bupivicaine, benzocaine, and lidocaine.

30. The transdermal delivery composition of Claim 15, wherein said analgesic is selected from the group consisting of tramadol hydrochloride, fentanyl, metamizole, morphine sulphate, ketorolac tromethamine, hydrocodone, oxycodone, morprhine, loxoprofen, Capsaicin, and Boswellin.

31. The transdermal delivery composition of Claim 20, wherein said immune response modifier selected from the group consisting of purine derivatives, adenine derivatives, cyclosporine, and CpGs.

## Patentansprüche

1. Zusammensetzung für die transdermale Abgabe, umfassend eine Liposphäre, wobei die Liposphäre einen ethoxylierten Fettanteil oder Lipidanteil und ein abzugebendes Mittel umfasst, wobei der Ethoxylierungsgrad des Fettanteils oder Lipidanteils 10, 11, 12, 13, 14, 15, 16, 17, 18 oder 19 Ethoxylierungen pro Molekül beträgt und wobei der Fettanteil oder Lipidanteil 10 Kohlenstoffreste umfasst.

2. Zusammensetzung für die transdermale Abgabe nach Anspruch 1, die weiterhin Wasser umfasst.

3. Zusammensetzung für die transdermale Abgabe nach Anspruch 1, die weiterhin Alkohol umfasst.

4. Zusammensetzung für die transdermale Abgabe nach Anspruch 1, wobei der Fettanteil oder Lipidanteil mindestens 10 Kohlenstoffreste, aber weniger als und höchstens 30 Kohlenstoffreste umfasst.

5. Zusammensetzung für die transdermale Abgabe nach Anspruch 1, wobei der Ethoxylierungsgrad gleich der Anzahl der Kohlenstoffreste des Fettanteils oder Lipidanteils ist.

6. Zusammensetzung für die transdermale Abgabe nach Anspruch 1, wobei die Kettenlänge des Fettanteils oder Lipidanteils 10, 12, 14, 16, 18, 20, 22 oder 24 Kohlenstoffreste beträgt.

7. Zusammensetzung für die transdermale Abgabe nach Anspruch 1, wobei der Fettanteil eine Fettsäure ist.

8. Zusammensetzung für die transdermale Abgabe nach Anspruch 7, wobei die Fettsäure ausgewählt ist aus der Gruppe, bestehend aus C16:1, delta 1; C16:1, delta 2; C16:1, delta 3; C16:1, delta 4; C16:1, delta 5; C16:1, delta 6; C16:1, delta 7; C16:1, delta 8; C16:1, delta 9 (Palmitoleinsäure); C16:1, delta 10; C16:1, delta 11; C16:1, delta 12; C16:1, delta 13; und C16:1, delta 14.

9. Zusammensetzung für die transdermale Abgabe nach Anspruch 1, wobei der Fettanteil ein Fettamin ist.

10. Zusammensetzung für die transdermale Abgabe nach Anspruch 1, wobei der Lipidanteil ein Sphingolipid, ein Glycolipid oder ein Glycosphingolipid ist.

11. Zusammensetzung für die transdermale Abgabe nach Anspruch 1, wobei die Liposphäre eine homogene Mischung eines ethoxylierten Fettanteils oder eine heterogene Mischung aus ethoxylierten Fettsäuren umfasst.

12. Zusammensetzung für die transdermale Abgabe nach Anspruch 1, wobei das abzugebende Mittel ein Polypeptidhormon ist.

13. Zusammensetzung für die transdermale Abgabe nach Anspruch 1, wobei das abzugebende Mittel Kollagen ist.

14. Zusammensetzung für die transdermale Abgabe nach Anspruch 1, wobei das abzugebende Mittel ein Anästhetikum ist.

15. Zusammensetzung für die transdermale Abgabe nach Anspruch 1, wobei das abzugebende Mittel ein Analgetikum ist.

16. Zusammensetzung für die transdermale Abgabe nach Anspruch 1, wobei das abzugebende Mittel ein nicht steroidales Antiphlogistikum (NSAID) ist, ausgewählt aus der Gruppe, bestehend aus Ibuprofen (2-(Isobutylphenyl)propionsäure); Methotrexat (N-[4-(2,4-Diamino-6-pteridinylmethyl]-methylamino]-benzoyl)-L-glutaminsäure); Aspirin (Acetylsalicylsäure); Salicylsäure; Diphenhydramin (2-(Diphenylmethoxy)-NN-dimethylethylamin-Hydrochlorid); Naproxen (6-Methoxy-9-methyl-Natriumsalz der 2-Naphthalinessigsäure, (-)); Phenylbutazon (4-Butyl-1,2-diphenyl-3,5-pyrazolidindion); Sulindac (2)-5-fluoro-2-methyl-1-[[p-(methylsulfinyl)-phenyl]-methylen-]-1H-inden-3-essigsäure; Diflunisal (2',4'-Difluor-4-hydroxy-3-biphenylcarbonsäure); Piroxicam (4-Hydroxy-2-methyl-N-2-pyridinyl-2H-1,2-benzothiazin-2-carboxamid-1,1-dioxid), ein Oxicam; Indomethacin (1-(4-Chlorbenzoyl)-5-methoxy-2-methyl-H-indol-3-essigsäure); Meclofenamat-Natrium (Natriumsalz der N-(2,6-Dichlor-m-tolyl)-anthranilsäure-Monohydrat); Ketoprofen (2-(3-Benzoylphenyl)-propionsäure); Tolmetin-Natrium (Natrium-1-methyl-5-(4-methylbenzoyl)-1H-pyrrol-2-acetat-Dihydrat); Diclofenac-Natrium (Mononatriumsalz der 2-[(2,6-Dichlorphenyl)-amino]-phenyl]essigsäure); Hydroxychlorochin-Sulfat (2-{[4-[(7-Chlor-4-chinolyl)-amino]-pentyl]-ethylamino}ethanolsulfat (1:1)); Penicillamin (3-Mercapto-D-valin); Flurbiprofen ([1,1-Biphenyl]-4-essigsäure-2-fluor-alpha-methyl (+/-)); Cetodolac (1,8-Diethyl-13,4,9-tetrahydropyrano-[3,4,13]-indol-1-essigsäure); Mefenaminsäure (N-(2,3-Xylyl)anthranilsäure); und Diphenhydramin-Hydrochlorid (2-Diphenylmethoxy-N,N-di-methylethamin-Hydrochlorid).

17. Zusammensetzung für die transdermale Abgabe nach Anspruch 1, wobei das abzugebende Mittel ein Antiphlogistikum ist, ausgewählt aus der Gruppe, bestehend aus Hydrocortison, Prednisolon, Triamcinolon, Cortison und Piroxicam, oder ein Antiinfektivum, ausgewählt aus der Gruppe, bestehend aus Amoxicillin, Clavulanat-Kalium, Itraconazol, Acyclovir, Fluconazol, Terbinafin-Hydrochlorid, Erythromycin-Ethylsuccinat, Acetylsulfisoxazol, Penicillin V, Cephalexin, Erythromycin, Azithromycin, Tetracyclin, Ciproflaxin, Gentamycin, Sulfathiazol, Nitrofurantoin, Norfloxacin, Flumechin und Ibafloxacin, Metronidazol, Nystatin, Lamivudin, Indinavirsulfat und Stavudin.

18. Zusammensetzung für die transdermale Abgabe nach Anspruch 1, wobei das abzugebende Mittel ein Metformin-Hydrochlorid oder Acarbose ist.

19. Zusammensetzung für die transdermale Abgabe nach Anspruch 1, wobei das abzugebende Mittel ein Immunogen, wie ein ein Polynukleotid enthaltendes Immunogen, ist.

20. Zusammensetzung für die transdermale Abgabe nach Anspruch 1, wobei das abzugebende Mittel ein Immunmodulator ist.

21. Zusammensetzung für die transdermale Abgabe nach Anspruch 1, wobei das abzugebende Mittel ein Enzymehemmer ist, ausgewählt aus der Gruppe, bestehend aus Zileuton, Captopril und Lisinopril; oder das Mittel ausgewählt ist aus der Gruppe, bestehend aus Ergotamin, Melatonin, Sumatriptan, Zolmitriptan und Rizatriptan; oder ausgewählt ist aus der Gruppe, bestehend aus Zolpidem, Zolpidemtartrat, Triazolam und Hycosinbutylbromid; oder ausgewählt ist aus der Gruppe, bestehend aus Iohexol, Technetium, TC99M, Sestamibi, Iomeprol, Gadodiamid, Ioversol und Iopromid oder ausgewählt ist aus der Gruppe, bestehend aus Alsactid, Americium, Betazol, Histamin, Mannitol, Metyrapon, Petagastrin, Phentolamin, radioaktivem B₁₂, Gadodiamid, Gadopentetsäure, Gadoteridol, Perflubron, Cyclosporin, Sildenafilcitrat, Paclitaxel, Ritonavir und Saquinavir.

22. Zusammensetzung für die transdermale Abgabe nach einem der Ansprüche 1-21 zur Verwendung als Arzneimittel.

23. Zusammensetzung für die transdermale Abgabe nach einem der Ansprüche 1-21 zur Verwendung für die Abgabe eines abzugebenden Mittels.

24. Zusammensetzung für die transdermale Abgabe nach Anspruch 1, wobei der Fettanteil eine ungesättigte Fettsäure ist.

25. Zusammensetzung für die transdermale Abgabe nach Anspruch 1, wobei der Fettanteil eine mehrfach ungesättigte Fettsäure ist.

26. Zusammensetzung für die transdermale Abgabe nach Anspruch 12, wobei das Polypeptidhormon ausgewählt ist aus der Gruppe, bestehend aus Oxytocin, Vasopressin, melanocytstimulierendes Hormon, Corticortropin, Lipotropin, Thyrotropin, Wachstumshormon, Prolactin, luteinisierendes Hormon, humanes Choriongonadotropin, follikelstimulierendes Hormon, Corticotropin-freisetzendes Hormon, Gonadotropin-freisetzendes Hormon, Prolaktin-freisetzender Faktor, Prolaktin-hemmender Faktor, Wachstumshormon-freisetzender Faktor, Somatostatin, Thyrotropin-freisetzendes Hormon, Calcitonin, Calcitoningen-verwandtes Peptid, Parathyroidhormon, glucagonähnliches Peptid 1, glucoseabhängiges insulinotropes Polypeptid, Gastrin, Secretin, Cholecystokinin, Motilin, vasoaktives intestinales Peptid, Substanz P, pankreatisches Polypeptid, Peptid Tyrosin Tyrosin, Neuropeptid Tyrosin, Amphiregulin, Insulin, Glucagon, Placentalactogen, Relaxin, Angiotensin II, atriales natriuretisches Peptid und Melatonin.

27. Zusammensetzung für die transdermale Abgabe nach Anspruch 1, wobei das abzugebende Mittel ein Nichtpeptidhormon ist.

28. Zusammensetzung für die transdermale Abgabe nach Anspruch 1, wobei das Polypeptidhormon ausgewählt ist aus der Gruppe, bestehend aus Thyroxin, Triiodthyronin, Estradiol, Estron, Progesteron, Testosteron, Cortisol, Corticosteron, Aldosteron, Epinephrin, Norepinepherin und Calctriol.

29. Zusammensetzung für die transdermale Abgabe nach Anspruch 14, wobei das Anästhetikum ausgewählt ist aus der Gruppe, bestehend aus Articain, Procain, Tetracain, Chlorprocain und Benzocain, Novocain, Mepivicain, Bupivicain, Benzocain und Lidocain.

30. Zusammensetzung für die transdermale Abgabe nach Anspruch 15, wobei das Analgetikum ausgewählt ist aus der Gruppe, bestehend aus Tramadol-Hydrochlorid, Fentanyl, Metamizol, Morphinsulfat, Ketorolactromethamin, Hydrocodon, Oxycodon, Morphin, Loxoprofen, Capsaicin und Boswellin.

31. Zusammensetzung für die transdermale Abgabe nach Anspruch 20, wobei das Immunmodulator ausgewählt ist aus der Gruppe, bestehend aus Purinderivaten, Adeninderivaten, Cyclosporin und CpGs.

## Revendications

1. Composition à administration transdermique comprenant une liposphère, dans laquelle ladite liposphère comprend une fraction grasse ou fraction lipidique éthoxylée et un agent administré, dans laquelle le nombre d'éthoxylations de ladite fraction grasse ou fraction lipidique est de 0, 11, 12, 13, 14, 15, 16, 17, 18 ou 19 éthoxylations par molécule, et dans laquelle ladite fraction grasse ou fraction lipidique comprend 10 résidus de carbone.

2. La composition à administration transdermique de la revendication 1, comprenant également de l'eau.

3. La composition à administration transdermique de la revendication 1, comprenant également de l'alcool.

4. La composition à administration transdermique de la revendication 1, dans laquelle ladite fraction grasse ou fraction lipidique comprend au moins 10 résidus de carbone mais sans excéder 30 résidus de carbone.

5. La composition à administration transdermique de la revendication 1, dans laquelle le nombre d'éthoxylations est identique au nombre de résidus de carbone de la fraction grasse ou de la fraction lipidique.

6. La composition à administration transdermique de la revendication 1, dans laquelle la longueur de chaîne de la fraction grasse ou de la fraction lipidique est de 10, 12, 14, 16, 18, 20, 22 ou 24 résidus de carbone.

7. La composition à administration transdermique de la revendication 1, dans laquelle la fraction grasse est un acide gras.

8. La composition à administration transdermique de la revendication 7, dans laquelle l'acide gras est choisi dans le groupe consistant en C16:1, delta 1 ; C16:1, delta 2 ; C16:1, delta 3 ; C16:1, delta 4 ; C16:1, delta 5 ; C16:1, delta 6 ; C16:1, delta 7 ; C16:1, delta 8 ; C16:1, delta 9 (acide palmitoléique) ; C16:1, delta 10 ; C16:1, delta 11 ; C16:1, delta 12 ; C16:1, delta 13 ; et C16:1, delta 14.

9. La composition à administration transdermique de la revendication 1, dans laquelle ladite fraction grasse est une amine grasse.

10. La composition à administration transdermique de la revendication 1, dans laquelle ladite fraction lipidique est un sphingolipide, un glycolipide ou un glycosphingolipide.

11. La composition à administration transdermique de la revendication 1, dans laquelle ladite liposphère comprend un mélange homogène d'une fraction grasse éthoxylée ou un mélange hétérogène d'acides gras éthoxylés.

12. La composition à administration transdermique de la revendication 1, dans laquelle l'agent administré est une hormone polypeptidique.

13. La composition à administration transdermique de la revendication 1, dans laquelle ledit agent administré est du collagène.

14. La composition à administration transdermique de la revendication 1, dans laquelle ledit agent administré est un anesthésique.

15. La composition à administration transdermique de la revendication 1, dans laquelle ledit agent administré est un analgésique.

16. La composition à administration transdermique de la revendication 1, dans laquelle ledit agent administré est un médicament anti-inflammatoire non stéroïdien (AINS) choisi dans le groupe consistant en ibuprofène (acide 2-(isobutylphényl)propionique) ; méthotrexate (acide N-[4-(2,4-diamino-6-ptéridinyl-méthyl]méthylamino]benzoyl)-L-glutamique) ; aspirine (acide acétylsalicylique) ; acide salicylique ; diphénhydramine (chlorhydrate de 2-(diphénylméthoxy)-NN-diméthyléthylamine) ; naproxène (acide 2-naphthalèneacétique, 6-méthoxy-9-méthyl-, sel sodique, (-)) ; phénylbutazone (4-butyl-1,2-diphényl-3,5-pyrazolidinedione) ; sulindac (acide (2)-5-fluoro-2-méthyl-1-[[p-(méthylsulfinyl)phényl]méthylène-]-1H-indène-3-acétique) ; diflunisal (acide 2',4'-difluoro-4-hydroxy-3-biphénylcarboxylique ; piroxicam (4-hydroxy-2-méthyl-N-2-pyridinyl-2H-1,2-benzothiazine-2-carboxamide 1,1-dioxyde, une oxicam ; indométacine (acide 1-(4-chlorobenzoyl)-5-méthoxy-2-méthyl-H-indole-3-acétique) ; méclofénamate sodique (acide N-(2,6-dichloro-m-tolyl)anthranilique, sel sodique, monohydrate) ; kétoprofène (acide 2-(3-benzoylphényl)-propionique ; tolmétine sodique (dihydrate de sodium 1-méthyl-5-(4-méthylbenzoyl-1H-pyrrole-2-acétate) ; diclofénac sodique (acide 2-[(2,6-dichlorophényl)amino]benzèneacétique, sel monosodique) ; sulfate d'hydroxychloroquine (sulfate de 2-{[4-[(7-chloro-4-quinolyl)amino]pentyl]éthylamino}éthanol (1:1) ; pénicillamine (3-mercapto-D-valine) ; flurbiprofène (acide [1,1-biphényl]-4-acétique, 2-fluoro-alphaméthyl-, (+-) ; cétodolac (acide 1-8-diéthyl-13,4,9, tétrahydropyrano-[3-4-13]indole-1-acétique ; acide méfénamique (acide N-(2,3-xylyl)anthranilique ; et chlorhydrate de diphénhydramine (chlorhydrate de 2-diphényl méthoxy-N, N-di-méthyléthamine).

17. La composition à administration transdermique de la revendication 1, dans laquelle l'agent administré est un composé anti-inflammatoire choisi dans le groupe consistant en hydrocortisone, prednisolone, triamcinolone, cortisone et piroxicam ou un composé anti-infectieux choisi dans le groupe consistant en amoxicilline, clavulanate potassique, itraconazole, acyclovir, fluconazole, chlorhydrate de terbinafine, éthylsuccinate d'érythromycine, acétyl sulfisoxazole, pénicilline V, céphalexine, érythromycine, azithromycine, tétracycline, ciproflaxine, gentamicine, sulfathiazole, nitrofurantoïne, norfloxacine, fluméquine, et ibafloxacine, métronidazole, nystatine, lamivudine, sulfate d'indinavir et stavudine.

18. La composition à administration transdermique de la revendication 1, dans laquelle l'agent administré est du chlorhydrate de métformine ou de l'acarbose.

19. La composition à administration transdermique de la revendication 1, dans laquelle l'agent administré est un immunogène tel qu'un immunogène comprenant un polynucléotide.

20. La composition à administration transdermique de la revendication 1, dans laquelle l'agent administré est un modificateur de la réponse immunitaire.

21. La composition à administration transdermique de la revendication 1, dans laquelle l'agent administré est un inhibiteur d'enzyme choisi dans le groupe consistant en zileuton, captopril et lisinopril ; ou l'agent est choisi dans le groupe consistant en ergotamine, mélatonine, sumatriptan, zolmitriptan et rizatriptan ; ou est choisi dans le groupe consistant en zolpidem, tartrate de zolpidem, triazolam et butylbromure d'hyoscine ; ou est choisi dans le groupe consistant en iohexol, technétium, TC99M, sestamibi, ioméprol, gadodiamide, ioversol et iopromide ou est choisi dans le groupe consistant en alsactide, américium, bétazole, histamine, mannitol, métyrapone, pentagastrine, phentolamine, B₁₂ radioactive, gadodiamide, acide gadopentétique, gadotéridol, perflubron, cyclosporine, citrate de sildénafil, paclitaxel, ritonavir et saquinavir.

22. La composition à administration transdermique de l'une quelconque des revendications 1 à 21 pour utilisation comme médicament.

23. La composition à administration transdermique de l'une quelconque des revendications 1 à 21 pour utilisation pour l'administration d'un agent administré.

24. La composition à administration transdermique de la revendication 1, dans laquelle la fraction grasse est un acide gras insaturé.

25. La composition à administration transdermique de la revendication 1, dans laquelle la fraction grasse est un acide gras polyinsaturé.

26. La composition à administration transdermique de la revendication 12, dans laquelle l'hormone polypeptidique est choisie dans le groupe consistant en oxytocine, vasopressine, hormone mélanostimulante, corticortropine, lipotropine, thyrotropine, hormone de croissance, prolactine, hormone lutéinisante, chorio-gonadotrophine humaine, hormone folliculo-stimulante, facteur de libération de la corticotropine, facteur de libération de la gonadotropine, facteur de libération de la prolactine, facteur inhibiteur de prolactine, facteur de libération de l'hormone de croissance, somatostatine, facteur de libération de la thyréostimuline, calcitonine, peptide apparenté au gène de la calcitonine, parathormone, glucagon-like peptide 1, polypeptide insulinotrope dépendant du glucose, gastrine, sécrétine, cholécystokinine, motiline, peptide intestinal vasoactif, substance P, polypeptide pancréatique, peptide tyrosine tyrosine, neuropeptide tyrosine, amphiréguline, insuline, glucagon, hormone lactogène placentaire, relaxine, angiotensine II, peptide natriurétique auriculaire et mélatonine.

27. La composition à administration transdermique de la revendication 1, dans laquelle l'agent administré est une hormone non peptidique.

28. La composition à administration transdermique de la revendication 1, dans laquelle l'hormone polypeptidique est choisie dans le groupe consistant en thyroxine, triiodothyronine, oestradiol, oestrone, progestérone, testostérone, cortisol, corticostérone, aldostérone, épinéphrine, norépinéphrine et calcitriol.

29. La composition à administration transdermique de la revendication 14, dans laquelle ledit anesthésique est choisi dans le groupe consistant en articaïne, procaïne, tétracaïne, chloroprocaïne et benzocaïne, novocaïne, mépivicaïne, bupivicaïne, benzocaïne et lidocaïne.

30. La composition à administration transdermique de la revendication 15, dans laquelle ledit analgésique est choisi dans le groupe consistant en chlorhydrate de tramadol, fentanyl, métamizole, sulfate de morphine, kétorolac trométhamine, hydrocodone, oxycodone, morphine, loxoprofène, capsaïcine et Boswellin.

31. La composition à administration transdermique de la revendication 20, dans laquelle ledit modificateur de la réponse immunitaire est choisi dans le groupe consistant en dérivés de purines, dérivés d'adénine, cyclosporine et CpG.
